(19) Europäisches Patentamt
European Patent Office
Office européen des brevets

(11) **EP 2 479 264 B1**

(12) **EUROPEAN PATENT SPECIFICATION**

(45) Date of publication and mention
of the grant of the patent:
**18.06.2014 Bulletin 2014/25**

(51) Int Cl.:
*C12N 9/24* *(2006.01)*          *C12N 9/34* *(2006.01)*
*C07K 14/37* *(2006.01)*         *C12N 15/56* *(2006.01)*
*C12N 15/31* *(2006.01)*         *C12R 1/885* *(2006.01)*
*C12N 15/62* *(2006.01)*

(21) Application number: **12164776.2**

(22) Date of filing: **08.04.2008**

(54) **Glucoamylase variants with altered properties**

Glucoamylase-Varianten mit veränderten Eigenschaften

Variants de glucoamylase présentant des propriétés modifiées

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.10.2007 PCT/US2007/021683**

(43) Date of publication of application:
**25.07.2012 Bulletin 2012/30**

(62) Document number(s) of the earlier application(s) in accordance with Art. 76 EPC:
**08742678.9 / 2 195 423**

(73) Proprietor: **Danisco US Inc.**
**Palo Alto, CA 94304 (US)**

(72) Inventors:
 • **Aehle, Wolfgang**
   **Palo Alto, CA 94304 (US)**
 • **Bott, Richard R.**
   **Palo Alto, CA 94304 (US)**
 • **Nikolaev, Igor**
   **Palo Alto, CA 94304 (US)**
 • **Scheffers, Martijn**
   **Palo Alto, CA 94304 (US)**
 • **Van Solingen, Piet**
   **Palo Alto, CA 94304 (US)**
 • **Vroemen, Casper**
   **Palo Alto, CA 94304 (US)**

(74) Representative: **Casley, Christopher Stuart et al**
**Mewburn Ellis LLP**
**33 Gutter Lane**
**London**
**EC2V 8AS (GB)**

(56) References cited:
**WO-A-00/04136**          **WO-A-02/46429**
**WO-A-2006/060062**

 • **DATABASE EMBL [Online] 2 February 2001 (2001-02-02), "Talaromyces emersonii ga gene for glucoamylase, exons 1-5", XP002495030, retrieved from EBI accession no. EMBL: AJ304803 Database accession no. AJ304803 -& NIELSEN BJARNE R ET AL: "Cloning, heterologous expression, and enzymatic characterization of a thermostable glucoamylase from Talaromyces emersonii", PROTEIN EXPRESSION AND PURIFICATION, ACADEMIC PRESS, SAN DIEGO, CA, US, vol. 26, no. 1, October 2002 (2002-10), pages 1-8, XP002419229, ISSN: 1046-5928**
 • **FAGERSTRÖM R ET AL: "CHARACTERIZATION, SUBSITE MAPPING AND PARTIAL AMINO ACID SEQUENCE OF GLUCOAMYLASE FROM THE FILAMENTOUS FUNGUS TRICHODERMA REESEI", BIOTECHNOLOGY AND APPLIED BIOCHEMISTRY, ACADEMIC PRESS, US, vol. 21, no. PART 2, April 1995 (1995-04), pages 223-231, XP009063876, ISSN: 0885-4513**
 • **ALESHIN ALEXANDER E ET AL: "Refined crystal structures of glucoamylase from Aspergillus awamori var. X100", JOURNAL OF MOLECULAR BIOLOGY, vol. 238, no. 4, 1994, pages 575-591, XP002473611, ISSN: 0022-2836**
 • **REILLY P J: "Protein engineering of glucoamylase to improve industrial performance a review.", STARCH/STAERKE, vol. 51, no. 8-9, 1999, pages 269-274, XP002473612,**

**Description**

## FIELD OF THE INVENTION

The present invention relates to variants of a parent glucoamylase having altered properties (*e.g.*, improved thermostability and/or specific activity). In particular, the present invention provides compositions comprising the variant glucoamylases, including starch hydrolyzing compositions, animal feed compositions and cleaning compositions. The invention also relates to DNA constructs encoding the variants and methods of producing the glucoamylase variants in host cells.

## BACKGROUND OF THE INVENTION

Glucoamylase enzymes (glucan 1,4-$\alpha$-glucohydrolases, EC 3.2.1.3) are starch hydrolyzing exo-acting carbohydrases, which catalyze the removal of successive glucose units from the non-reducing ends of starch or related oligo and polysaccharide molecules. Glucoamylases can hydrolyze both the linear and branched glucosidic linkages of starch (e.g., amylose and amylopectin).

Glucoamylases are produced by numerous strains of bacteria, fungi, yeast and plants. Particularly interesting, and commercially important, glucoamylases are fungal enzymes that are extracellularly produced, for example from strains of *Aspergillus* (Svensson et al. (1983) Carlsberg Res. Commun. 48:529- 544; Boel et al., (1984) EMBO J. 3:1097 - 1102; Hayashida et al., (1989) Agric. Biol. Chem. 53:923 - 929; USP 5,024,941; USP 4,794,175 and WO 88/09795); *Talaromyces* (USP 4,247,637; USP 6,255,084 and USP 6,620,924); *Rhizopus* (Ashikari et al., (1986) Agric. Biol. Chem. 50:957 -964; Ashikari et al., (1989) App. Microbiol. and Biotech. 32:129 - 133 and USP 4,863,864); *Humicola* (WO 05/052148 and USP 4,618,579) and *Mucor* (Houghton-Larsen et al., (2003) Appl. Microbiol. Biotechnol. 62:210 - 217). Many of the genes that code for these enzymes have been cloned and expressed in yeast, fungal and/or bacterial cells.

Commercially, glucoamylases are very important enzymes and have been used in a wide variety of applications that require the hydrolysis of starch (*e.g.* for producing glucose and other monosaccharides from starch). Glucoamylases are used to produce high fructose corn sweeteners, which comprise over 50% of the sweetener market in the United States. In general, glucoamylases may be, and commonly are, used with alpha amylases in starch hydrolyzing processes to hydrolyze starch to dextrins and then glucose. The glucose may then be converted to fructose by other enzymes (*e.g.* glucose isomerases); crystallized; or used in fermentations to produce numerous end products (*e.g.*, ethanol, citric acid, lactic acid, succinate, ascorbic acid intermediates, glutamic acid, glycerol and 1, 3-propanediol). Ethanol produced by using glucoamylases in the fermentation of starch and/or cellulose containing material may be used as a source of fuel or for alcoholic consumption.

WO 2006/060062 discloses the Trichoderma reesei glucoamylase TrGA and uses in starch conversion, animal feed and alcohol fermentation.

Although glucoamylases have been used successfully in commercial applications for many years, a need still exists for new glucoamylases having altered properties.

## SUMMARY OF THE INVENTION

The present invention provides glucoamylase variants, which are variants of a parent glucoamylase, wherein the parent glucoamylase has an amino acid sequence having at least 97% sequence identity with the amino acid sequence:

SVDDFISTETPIALNNLLCNVGPDGCRAFGTSAGAVIASPSTIDPDYYYMWTRDSALV

FKNLIDRFTETYDAGLQRRIEQYITAQVTLQGLSNPSGSLADGSGLGEPKFELTLKPFT

GNWGRPQRDGPALRAIALIGYSKWLINNNYQSTVSNVIWPIVRNDLNYVAQYWNQT

GFDLWEEVNGSSFFTVANQHRALVEGATLAATLGQSGSAYSSVAPQVLCFLQRFWV

SSGGYVDSNINTNEGRTGKDVNSVLTSIHTFDPNLGCDAGTFQPCSDKALSNLKVVV

DSFRSIYGVNKGIPAGAAVAIGRYAEDVYYNGNPWYLATFAAAEQLYDAIYVWKKT

GSITVTATSLAFFQELVPGVTAGTYSSSSSTFTNIINAVSTYADGFLSEAAKYVPADGS

LAEQFDRNSGTPLSALHLTWSYASFLTATARRAGIVPPSWANSSASTIPSTCSGASVV

GSYSRPTATSFPPSQTPKPGVPSGTPYTPLPCATPTSVAVTFHELVSTQFGQTVKVAG

NAAALGNWSTSAAVALDAVNYADNHPLWIGTVNLEAGDVVEYKYINVGQDGSVT

WESDPNHTYTVPAVACVTQVVKEDTWQS (SEQ ID NO: 2),

and the variants have only one, two, three or four substitutions as compared to the parent glucoamylase, wherein the substitutions comprise the substitution L417V, L417A, L417D, L417E, L417F, L417G, L417I, L417K, L417Q, L417R, L417S, L417T, L417W or L417Y in SEQ ID NO: 2 or an equivalent position in the parent glucoamylase as determined by sequence alignment.

In some embodiments, the glucoamylase variants are variants of a parent glucoamylase which has at least 98% sequence identity with the sequence of SEQ ID NO: 2.

In some embodiments, the glucoamylase variants have only two, three or four substitutions as compared to the parent glucoamylase, wherein the further substitutions are selected from substitutions at a position corresponding to position 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182,204, 205, 214, 216, 219, 228, 229, 230, 231, 236, 239, 240, 241, 242, 244, 263. 264, 265, 268, 269, 276, 284, 291, 300, 301, 303, 310, 311, 313, 316, 338, 342, 344, 346, 349, 359, 361, 364, 379, 382, 390, 391, 393, 394, 408, 410, 415, 418, 430, 431, 433, 436, 442, 443, 444, 448 and 451 of SEQ ID NO: 2 or an equivalent position in the parent glucoamylase as determined by sequence alignment.

In some embodiments, the glucoamylase variants have increased specific activity compared to the glucoamylase comprising the sequence of SEQ ID NO: 2. In some embodiments, the glucoamylase variant of claim 1, wherein the variants have increased thermostability compared to the glucoamylase comprising the sequence of SEQ ID NO: 2. In some embodiments, the glucoamylase variants have increased specific activity and increased thermostability compared to the glucoamylase comprising SEQ ID NO: 2.

In some embodiments, the glucoamylase variants are variants of a parent glucoamylase obtained from a Trichoderma spp.

The present invention also provides polynucleotides encoding glucoamylase variants as defined in claim 1.

Also provided are host cells comprising polynucleotides of the invention.

The present invention also provides enzyme compositions comprising glucoamylase variants as defined in claim 1.

In some embodiments, the enzyme compositions are suitable for use in a starch conversion process, an animal feed composition, or a starch containing substrate fermentation process. In some embodiments, the enzyme compositions further comprise an alpha amylase.

The present invention also provides methods of producing variant glucoamylases in a host cell comprising: transforming host cells with DNA constructs comprising polynucleotides of the invention and culturing the host cells under conditions suitable for the expression and production of glucoamylase variants and producing the variants.

In some embodiments, the methods further comprise recovering the glucoamylase variants from the culture.

**BRIEF DESCRIPTION OF THE DRAWINGS**

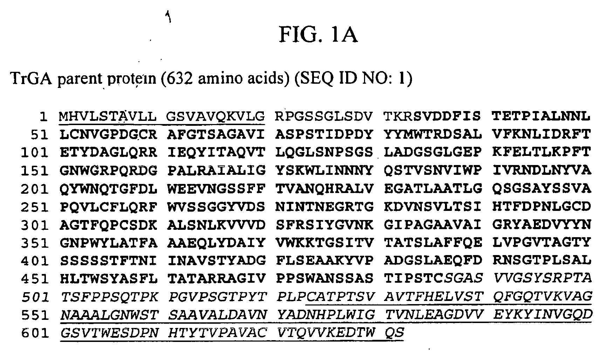

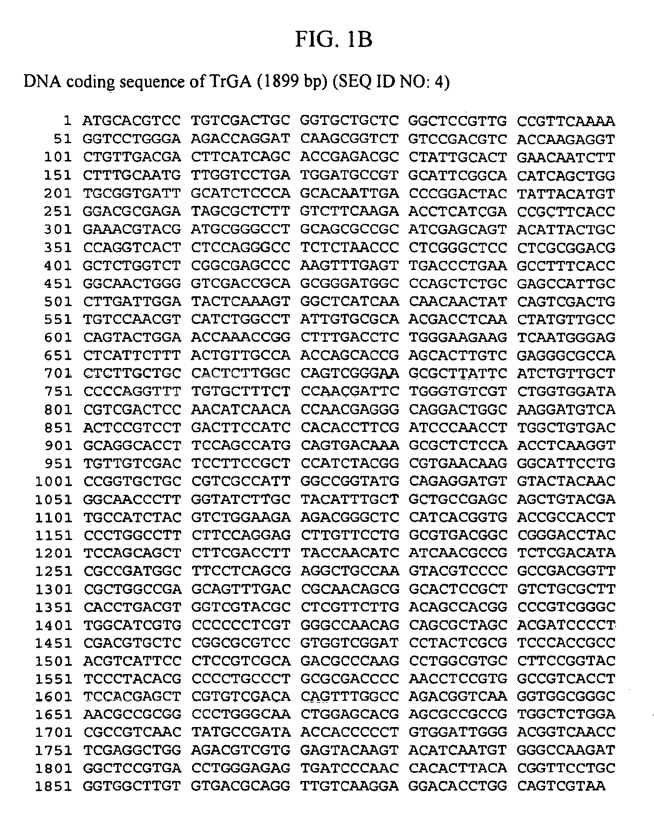

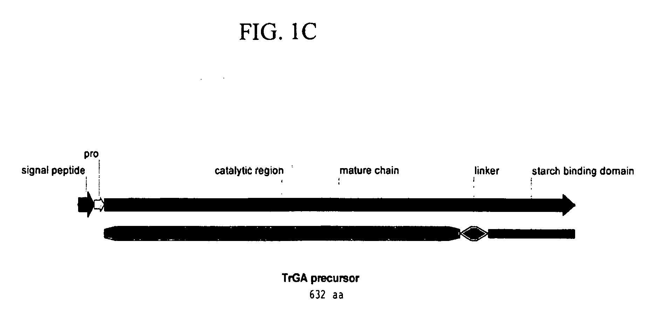

FIG. 1A illustrates a *Trichoderma reesei* glucoamylase (TrGA) having 632 amino acids (SEQ ID NO: 1). The signal peptide is underlined, the catalytic region (SEQ ID NO:3) starting with amino acid residues SVDDFI (SEQ ID NO:12) and having 453 amino acid residues is in bold; the linker region is in italics and the starch binding domain (SBD) is both italics and underlined. The mature protein which includes the catalytic domain (SEQ ID NO:3), linker region (SEQ ID NO:10) and starch binding domain (SEQ ID NO:11) is represented by SEQ ID NO:2. Fig. 1B illustrates the cDNA (SEQ ID NO:4) which codes for the TrGA. Fig. 1C illustrates the precursor and mature protein TrGA domains.

FIG. 2 illustrates the destination plasmid pDONR-TrGA which includes the cDNA (SEQ ID NO:4) of the TrGA.

FIG. 3 illustrates the plasmid pTTT-Dest.

FIG. 4 illustrates the final expression vector pTTT-TrGA.

FIGS. 5A - 5B illustrate an alignment comparison of the catalytic domains of parent glucoamylases including glucoamylase derived from *Aspergillus awamori* (AaGA) (SEQ ID NO:5); *Aspergillus niger* (AnGA) (SEQ ID NO:6); *Aspergillus oryzae* (AoGA) (SEQ ID NO:7); *Trichoderma reesei* (TrGA) (SEQ ID NO:3); *Humicola grisea* (HgGA) (SEQ ID NO:8); and *Hypocrea vinosa* (HvGA) (SEQ ID NO:9). Identical amino acids are indicated by an asterisk (*). FIG. 5C illustrates the *Talaromyces* glucoamylase (TeGA) mature protein sequence (SEQ ID NO:308).

FIG. 6 is a comparison of the three dimensional structures of *Trichoderma* glucoamylase (black) (SEQ ID NO:2) and *Aspergillus awamori* glucoamylase (grey) viewed from the side. The side is measured in reference to the active site and the active site entrance is at the "top" of the molecule.

FIG. 7 is a comparison of the three dimensional structures of *Trichoderma glucoamylase* (black) and *Aspergillus awamori* glucoamylase (grey) viewed from the top. The active site entrance is at the "top" of the molecule.

## DETAILED DESCRIPTION OF THE INVENTION

### I. Definitions

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

As used herein, the term "glucoamylase (EC 3.2.1.3)" refers to an enzyme that catalyzes the release of D-glucose from the non-reducing ends of starch and related oligo-and polysaccharides.

As used herein, the term "parent" or "parent sequence" refers to a sequence that has sequence and/or structural identity with TrGA (e.g., SEQ ID NOs: 1, 2 and/or 3) and is a native or naturally occurring sequence in a host cell.

As used herein, the term "TrGA" refers to a *Trichoderma reesei* glucoamylase sequence having the mature protein sequence illustrated in SEQ ID NO:2, which includes the catalytic domain having the sequence illustrated in SEQ ID NO:3. The isolation, cloning and expression of the TrGA are described in WO 2006/060062 and U.S. Pat. Pub. No. 2006/0094080 published May 4, 2006. The TrGA is also considered a parent glucoamylase sequence. In some embodiments, the parent sequence refers to a TrGA that is the starting point for protein engineering. The numbering of the glucoamylase amino acids herein is based on the sequence of the TrGA glucoamylase sequence (SEQ ID NO: 2 and SEQ ID NO: 3).

The phrase "mature form of a protein or polypeptide" refers to the final functional form of the protein or polypeptide. To exemplify, a mature form of the TrGA includes the catalytic domain, linker region and starch binding domain having the amino acid sequence of SEQ ID NO:2.

The term "*Trichoderma* glucoamylase homologues" refers to parent glucoamylases having at least 80% amino acid sequence identity to the TrGA sequence (SEQ ID NO:1, SEQ ID NO:2 or SEQ ID NO:3) and which glucoamylases retain the functional characteristics of a glucoamylase.

As used herein, a "homologous sequence" means a nucleic acid or polypeptide sequence having at least 100%, at least 99%, at least 98%, at least 97%, at least 96%, at least 95%, at least 94%, at least 93%, at least 92%, at least 91%, at least 90%, at least 88%, at least 85%, at least 80%, at least 75%, at least 70%, at least 65%, at least 60%, at least 55%, at least 50%, or at least 45% sequence identity to a nucleic acid sequence or polypeptide sequence when optimally aligned for comparison, wherein the function of the candidate nucleic acid sequence or polypeptide sequence is essentially the same as the nucleic acid sequence or polypeptide sequence said candidate homologous sequence is being compared with. In some embodiments, homologous sequences have between 85% and 100% sequence identity, while in other embodiments there is between 90% and 100% sequence identity, and in other embodiments, there is 95% and 100% sequence identity. In some embodiments, the candidate homologous sequence or parent is compared with the TrGA nucleic acid sequence or mature protein sequence. The sequence identity can be measured over the entire length of the parent or homologous sequence.

As used herein, the terms "glucoamylase variant", "variant" and "TrGA variant" are used in reference to glucoamylases that are similar to a parent glucoamylase sequence (e.g., the TrGA or *Trichoderma* glucoamylase homologues) but have at least one substitution, deletion or insertion in their amino acid sequence that makes them different in sequence from a parent glucoamylase. In some cases they have been manipulated/engineered to include at least one substitution, deletion or insertion in their amino acid sequence that makes them different in sequence from a parent glucoamylase.

As used herein the term "catalytic domain" refers to a structural region of a polypeptide, which contains the active site for substrate hydrolysis.

The term "linker" refers to a short amino acid sequence generally having between 3 and 40 amino acid residues that covalently bind an amino acid sequence comprising a starch binding domain with an amino acid sequence comprising a catalytic domain.

The term "starch binding domain" refers to an amino acid sequence that binds preferentially to a starch substrate.

As used herein, the terms "mutant sequence" and "mutant gene" are used interchangeably and refer to a polynucleotide sequence that has an alteration in at least one codon occurring in a host cell's parent sequence. The expression product of the mutant sequence is a variant protein with an altered amino acid sequence relative to the parent. The expression product may have an altered functional capacity (*e.g.*, enhanced enzymatic activity).

The term "property" or grammatical equivalents thereof in the context of a polypeptide, as used herein, refers to any characteristic or attribute of a polypeptide that can be selected or detected. These properties include, but are not limited to oxidative stability, substrate specificity, catalytic activity, thermal stability, pH activity profile, resistance to proteolytic degradation, $K_M$, $K_{CAT}$, $K_{CAT}/K_M$ ratio, protein folding, ability to bind a substrate and ability to be secreted.

The term "property" or grammatical equivalent thereof in the context of a nucleic acid, as used herein, refers to any characteristic or attribute of a nucleic acid that can be selected or detected. These properties include, but are not limited to, a property affecting gene transcription (*e.g.*, promoter strength or promoter recognition), a property affecting RNA processing (*e.g.*, RNA splicing and RNA stability), a property affecting translation (*e.g.*, regulation, binding of mRNA to ribosomal proteins).

The terms "thermally stable" and "thermostable" refer to glucoamylase variants of the present invention that retain a specified amount of enzymatic activity after exposure to identified temperatures over a given period of time under conditions prevailing during the hydrolysis of starch substrates, for example while exposed to altered temperatures.

The term "enhanced stability" in the context of a property such as thermostability refers to a higher retained starch hydrolytic activity over time as compared to another reference (e.g., parent) glucoamylase.

The term "diminished stability" in the context of a property such as thermostability refers to a lower retained starch hydrolytic activity over time as compared to another reference glucoamylase.

The term "specific activity" The specific activity is defined as the activity per mg of glucoamylase protein. In some embodiments, the activity for glucoamylase is determined by the ethanol assay described herein and expressed as the amount of glucose which is produced from the starch substrate. In some embodiments, the protein concentration can be determined using the Caliper assay described herein.

The terms "active" and "biologically active" refer to a biological activity associated with a particular protein. It follows that the biological activity of a given protein refers to any biological activity typically attributed to that protein by those skilled in the art. For example, an enzymatic activity associated with a glucoamylase is hydrolytic and, thus an active glucoamylase has hydrolytic activity.

The terms "polynucleotide" and "nucleic acid", used interchangeably herein, refer to a polymeric form of nucleotides of any length, either ribonucleotides or deoxyribonucleotides. These terms include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases.

As used herein, the terms "DNA construct," "transforming DNA" and "expression vector" are used interchangeably to refer to DNA used to introduce sequences into a host cell or organism. The DNA may be generated *in vitro* by PCR or any other suitable technique(s) known to those in the art. The DNA construct, transforming DNA or recombinant expression cassette can be incorporated into a plasmid, chromosome, mitochondrial DNA, plastid DNA, virus, or nucleic acid fragment. Typically, the recombinant expression cassette portion of an expression vector, DNA construct or transforming DNA includes, among other sequences, a nucleic acid sequence to be transcribed and a promoter. In embodiments, expression vectors have the ability to incorporate and express heterologous DNA fragments in a host cell.

As used herein, the term "vector" refers to a polynucleotide construct designed to introduce nucleic acids into one or more cell types. Vectors include cloning vectors, expression vectors, shuttle vectors, plasmids, cassettes and the like.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction.

As used herein, the terms "transformed" and "stably transformed" refer to a cell that has a non-native (heterologous) polynucleotide sequence integrated into its genome or as an episomal plasmid that is maintained for at least two generations.

As used herein, the terms "selectable marker" and "selective marker" refer to a nucleic acid (*e.g.*, a gene) capable of expression in host cells which allows for ease of selection of those hosts containing the vector. Typically, selectable markers are genes that confer antimicrobial resistance or a metabolic advantage on the host cell to allow cells containing the exogenous DNA to be distinguished from cells that have not received any exogenous sequence during the transformation.

As used herein, the term "promoter" refers to a nucleic acid sequence that functions to direct transcription of a downstream gene. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

A nucleic acid is "operably linked" when it is placed into a functional relationship with another nucleic acid sequence. For example, DNA encoding a secretory leader (*i.e.*, a signal peptide), is operably linked to DNA for a polypeptide if it is expressed as a preprotein that participates in the secretion of the polypeptide. Generally, "operably linked" means that the DNA sequences being linked are contiguous, and, in the case of a secretory leader, contiguous and in reading phase.

As used herein the term "gene" refers to a polynucleotide (*e.g.*, a DNA segment), that encodes a polypeptide and includes regions preceding and following the coding regions as well as intervening sequences (introns) between individual coding segments (exons).

As used herein, "homologous genes" refers to a pair of genes from different, but usually related species, which correspond to each other and which are identical or very similar to each other. The term encompasses genes that are separated by speciation (*i.e.*, the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene (*i.e.*, a homologous gene) by speciation. Typically, orthologs retain the same function during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "homology" refers to sequence similarity or identity, with identity being preferred. This homology is determined using standard techniques known in the art (*See e.g.,* Smith and Waterman, (1981)Adv. Appl. Math., 2:482; Needleman and Wunsch, (1988) J. Mol. Biol., 48:443; Pearson and Lipman, (1988) Proc. Natl. Acad. Sci. USA 85:2444; programs such as GAP, BESTFIT, FASTA, and TFASTA in the Wisconsin Genetics Software Package (Genetics Computer Group, Madison, WI); and Devereux et al., (1984) Nucl. Acid Res., 12:387-395).

The "percent (%) nucleic acid sequence identity" or "percent (%) amino acid sequence identity" is defined as the percentage of nucleotide residues or amino acid residues in a candidate sequence that are identical with the nucleotide residues or amino acid residues of the starting sequence (*i.e.*, TrGA). The sequence identity can be measured over the entire length of the starting sequence (*i.e.*, TrGA SEQ ID NO:2 or 3).

Homologous sequences are determined by known methods of sequence alignment. A commonly used alignment method is BLAST described by Altschul *et al.,* (Altschul et al., (1990) J. Mol. Biol., 215:403-410; and Karlin et al., (1993) Proc. Natl. Acad. Sci. USA 90:5873-5787). A particularly useful BLAST program is the WU-BLAST-2 program (*See*, Altschul et al., (1996) Meth. Enzymol., 266:460-480). WU-BLAST-2 uses several search parameters, most of which are set to the default values. The adjustable parameters are set with the following values: overlap span = 1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Other methods find use in aligning sequences. One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, (1987) J. Mol. Evol., 35:351-360). The method is similar to that described by Higgins and Sharp (Higgins and Sharp, (1989) CABIOS 5:151-153). Useful PILEUP parameters including a default gap weight of 3.00, a default gap length weight of 0.10, and weighted end gaps.

The term "optimal alignment" refers to the alignment giving the highest percent identity score. An "equivalent position" refers to an optimal alignment between two sequences. For example using Figs. 5D and 5E, position 491 in TrGA (SEQ ID NO: 2) is C491; the equivalent position for *Aspergillus niger* is position C509; and the equivalent position for *Aspergillus awamori* is position Q538. See Figure 8 for an exemplary alignment of the three-dimensional sequence.

As used herein, the term "hybridization" refers to the process by which a strand of nucleic acid joins with a complementary strand through base pairing, as known in the art.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while an intermediate or low stringency hybridization can be used to identify or detect polynucleotide sequence homologs.

Moderate and high stringency hybridization conditions are well known in the art. An example of high stringency conditions includes hybridization at about 42°C in 50% formamide, 5X SSC, 5X Denhardt's solution, 0.5% SDS and 100 μg/ml denatured carrier DNA followed by washing two times in 2X SSC and 0.5% SDS at room temperature and two additional times in 0.1 X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. as necessary to accommodate factors such as probe length and the like.

As used herein, "recombinant" includes reference to a cell or vector, that has been modified by the introduction of a heterologous or homologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention.

In some embodiments of the present disclosure, mutated DNA sequences are generated with site saturation mutagenesis in at least one codon. In other embodiments, site saturation mutagenesis is performed for two or more codons. In a further embodiment, mutant DNA sequences have more than 50%, more than 55%, more than 60%, more than 65%, more than 70%, more than 75%, more than 80%, more than 85%, more than 90%, more than 95%, more than 98% or more than 99% homology with the parent sequence. In alternative embodiments, mutant DNA is generated *in vivo* using any known mutagenic procedure such as, for example, radiation, nitrosoguanidine and the like. The desired DNA sequence is then isolated and used in the methods provided herein.

As used herein, "heterologous protein" refers to a protein or polypeptide that does not naturally occur in the host cell.

As used herein, "homologous protein" refers to a protein or polypeptide native or naturally occurring in a cell and includes native proteins that are over-expressed in the cell whether by recombinant DNA technology or naturally.

An enzyme is "over-expressed" in a host cell if the enzyme is expressed in the cell at a higher level than the level at which it is expressed in a corresponding wild-type cell.

The terms "protein" and "polypeptide" are used interchangeably herein. In the present disclosure and claims, the conventional one-letter and three-letter codes for amino acid residues are used. The 3-letter code for amino acids as defined in conformity with the IUPAC-IUB Joint Commission on Biochemical Nomenclature (JCBN). It is also understood that a polypeptide may be coded for by more than one nucleotide sequence due to the degeneracy of the genetic code.

Variants of the present disclosure are described by the following nomenclature: [original amino acid residue/position/substituted amino acid residue]. For example the substitution of leucine for arginine at position 76 is represented as R76L. When more than one amino acid is substituted at a given position, the substitution is represented as 1) Q172C, Q172D or Q172R; 2) Q172C, D, or R or c) Q172C/D/R. When a position suitable for substitution is identified herein without a specific amino acid suggested, it is to be understood that any amino acid residue may be substituted for the amino acid residue present in the position. Where a variant glucoamylase contains a deletion in comparison with other glucoamylases the deletion is indicated with "*". For example, a deletion at position R76 is represented as R76*. A deletion of two or more consecutive amino acids is indicated for example as (76 - 78)*.

A "prosequence" is an amino acid sequence between the signal sequence and mature protein that is necessary for the secretion of the protein. Cleavage of the pro sequence will result in a mature active protein.

The term "signal sequence" or "signal peptide" refers to any sequence of nucleotides and/or amino acids which may participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein. The signal sequence may be endogenous or exogenous. The signal sequence may be that normally associated with the protein (*e.g.*, glucoamylase), or may be from a gene encoding another secreted protein.

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polypeptides that are involved in post-translational activity (*e.g.*, polypeptides cleaved therefrom to leave the mature form of a protein or peptide).

"Host strain" or "host cell" refers to a suitable host for an expression vector comprising DNA according to the present invention.

The terms "derived from" and "obtained from" refer to not only a glucoamylase produced or producible by a strain of the organism in question, but also a glucoamylase encoded by a DNA sequence isolated from such strain and produced in a host organism containing such DNA sequence. Additionally, the term refers to a glucoamylase which is encoded by a DNA sequence of synthetic and/or cDNA origin and which has the identifying characteristics of the glucoamylase in question.

A "derivative" within the scope of this definition generally retains the characteristic hydrolyzing activity observed in the wild-type, native or parent form to the extent that the derivative is useful for similar purposes as the wild-type, native or parent form. Functional derivatives of glucoamylases encompass naturally occurring, synthetically or recombinantly produced peptides or peptide fragments which have the general characteristics of the glucoamylases of the present invention.

The term "isolated" or "purified" refers to a material that is removed from its original environment (*e.g.*, the natural environment if it is naturally occurring). In some embodiments, an isolated protein is more than 10% pure, preferably more than 20% pure, and even more preferably more than 30% pure, as determined by SDS-PAGE. Further aspects of the present disclosure encompass the protein in a highly purified form (*i.e.*, more than 40% pure, more than 60% pure, more than 80% pure, more than 90% pure, more than 95% pure, more than 97% pure, and even more than 99% pure), as determined by SDS-PAGE.

As used herein, the term, "combinatorial mutagenesis" refers to methods in which libraries of variants of a starting sequence are generated. In these libraries, the variants contain one or several mutations chosen from a predefined set of mutations. In addition, the methods provide means to introduce random mutations which were not members of the predefined set of mutations. In some embodiments, the methods include those set forth in USP 6,582,914. In alternative embodiments, combinatorial mutagenesis methods encompass commercially available kits (*e.g.*, QUIKCHANGE® Multisite, Stratagene, San Diego, CA).

As used herein, the term "library of mutants" refers to a population of cells which are identical in most of their genome but include different homologues of one or more genes. Such libraries can be used, for example, to identify genes or operons with improved traits.

As used herein the term "dry solids content (DS or ds)" refers to the total solids of a slurry in % on a dry weight basis.

As used herein, the term "initial hit" refers to a variant that was identified by screening a combinatorial consensus mutagenesis library. In embodiments, initial hits have improved performance characteristics, as compared to the starting gene.

As used herein, the term "improved hit" refers to a variant that was identified by screening an enhanced combinatorial consensus mutagenesis library.

As used herein, the term "target property" refers to the property of the starting gene that is to be altered. It is not intended that the present invention be limited to any particular target property. However, in some embodiments, the target property is the stability of a gene product (*e.g.*, resistance to denaturation, proteolysis or other degradative factors), while in other embodiments, the level of production in a production host is altered. Indeed, it is contemplated that any property of a starting gene will find use in the present invention. Other definitions of terms may appear throughout the specification.

Before the exemplary embodiments are described in more detail, it is to be understood that this invention is not limited to particular embodiments described herein, as such may, vary. It is also to be understood that the terminology used herein is for the purpose of describing particular embodiments only, and is not intended to be limiting.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

As used herein and in the appended claims, the singular forms "a", "an", and "the" include plural referents unless the context clearly dictates otherwise. Thus, for example, reference to "a gene" includes a plurality of such candidate agents and reference to "the cell" includes reference to one or more cells and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention.

**Detailed Embodiments**

An objective of the present invention was to alter properties, such as thermal stability and/or specific activity of parent glucoamylases and in particular *Trichoderma reesei* glucoamylase (TrGA), to obtain glucoamylase variants having the altered properties which would be useful in various applications such as starch conversion or alcohol fermentation processes.

**Parent glucoamylases:**

In some embodiments, the present invention provides a glucoamylase variant of a parent glucoamylase as defined in the claims. The parent glucoamylase comprises a sequence that has sequence and/or structural identity with TrGA (SEQ ID NOs:2). In some embodiments, the parent glucoamylase is a homologue. In some embodiments the parent glucoamylase has at least 97% sequence identity, at least 98% sequence identity and also at least 99% sequence identity with the TrGA amino acid sequence of SEQ ID NO: 2.

In some embodiments, the parent glucoamylase of the present disclosure comprises a catalytic domain having an amino acid sequence having at least 50% amino acid sequence identity, at least 60% amino acid sequence identity, at least 70% amino acid sequence identity, at least 80% amino acid sequence, at least 85% amino acid sequence identity, at least 90% amino acid sequence identity, at least 93% amino acid sequence identity, at least 95% amino acid identity, at least 97% amino acid sequence identity and at least 99% amino acid sequence identity with one or more of the amino acid sequences illustrated in SEQ ID NO:1, 2, 3, 5, 6, 7, or 8. In other embodiments, the parent glucoamylase will have at least 80% sequence identity, at least 85% sequence identity, at least 90% sequence identity, at least 95% sequence identity, at least 97% sequence identity and also at least 98% sequence identity with the catalytic domain of the TrGA amino acid sequence of SEQ ID NO:3.

The parent glucoamylase of the present disclosure can be encoded by a DNA sequence which hybridizes under medium, high or stringent conditions with a DNA encoding a glucoamylase having one of the amino acid sequences of SEQ ID NO: 1, 2 or 3. In some embodiments, the parent glucoamylase with at least 50% sequence identity, at least 60% amino acid sequence identity, at least 70% amino acid sequence identity, at least 80% amino acid sequence identity, at least 90% amino acid sequence identity, at least 95% amino acid identity and at least 97% amino acid sequence identity also has structural identity with SEQ ID NOs:2 and/or 3. While the parent glucoamylase is a native or naturally occurring sequence in a host cell, in some embodiments, the parent glucoamylase is a naturally occurring variant. In some embodiments, the parent glucoamylase is a variant that has been engineered and/or a hybrid glucoamylase.

Predicted structures and known sequences of glucoamylases are conserved among fungal species (Coutinho et al., (1994) Protein Eng., 7:393 - 400 and Coutinho et al., (1994), Protein Eng., 7: 749-760). In some embodiments, the parent glucoamylase is a filamentous fungal glucoamylase. In some embodiments, the parent glucoamylase is obtained from a *Trichoderma* strain (*e.g., T. reesei, T. longibrachiatum, T. strictipilis, T. asperellum, T. konilangbra* and *T. hazianum*), an *Aspergillus* strain (*e.g. A. niger, A. nidulans, A. kawachi, A. awamori* and *A. orryae*), a *Talaromyces* strain (*e.g. T. emersonii, T. thermophilus,* and *T. duponti*), a *Hypocrea* strain (*e.g. H. gelatinosa, H. orientalis, H. vinosa,* and *H. citrina*), a *Fusarium* strain (*e.g., F. oxysporum, F. roseum,* and *F. venenatum*), a *Neurospora* strain (*e.g., N. crassa*) and a *Humicola* strain (*e.g., H. grisea, H. insolens* and *H. lanuginosa*), a *Penicillium* strain (e.g. *P. notatum or P. chrysogenum*), or a *Saccharomycopsis* strain (e.g. *S. fibuligera*).

In some embodiments, the parent glucoamyrase of the present disclosure is a bacterial glucoamylase. For example, the polypeptide may be obtained from a gram positive bacterial strain such as *Bacillus* (*e.g., B. alkalophilus, B. amyloliquefaciens, B. lentus, B. licheniformis, B. stearothermophilus, B. subtilis* and *B. thuringiensis*) or a *Streptomyces* strain (*e.g., S. lividans*).

In some other embodiments, the parent glucoamylase will comprise an amino acid sequence having at least 90% sequence identity, at least 93% sequence identity, at least 95% sequence identity, at least 96% sequence identity, at least 97% sequence identity, at least 98% sequence identity and also at least 99% sequence identity with the catalytic domain of the *Aspergillus* parent glucoamylase of SEQ ID NO:5 or SEQ ID NO:6.

In other embodiments, the parent glucoamylase will comprise an amino acid sequence having at least 90% sequence identity, at least 95% sequence identity, at least 97% sequence identity and also at least 99% sequence identity with the catalytic domain of the *Humicola grisea* (HgGA) parent glucoamylase of SEQ ID NO:8.

In some embodiments, the parent glucoamylase has at least 50% sequence identity, at least 60% sequence identity, at least 70% sequence identity, at least 80% sequence identity, at least 85% sequence identity, at least 88% sequence identity, at least 90% sequence identity, at least 93% sequence identity, at least 95% sequence identity, at least 96% sequence identity, at least 97% sequence identity, at least 98% sequence identity and also at least 99% sequence identity with the TrGA amino acid sequence of SEQ ID NO: 2 or 3 and has structural identity to the glucoamylase of SEQ ID NO:2 or 3.

In further embodiments, a *Trichoderma* glucoamylase homologue will be obtained from a *Trichoderma* or *Hypocrea* strain. Some *Trichoderma* glucoamylase homologues are described in US Pat. Pub. No. 2006/0094080 and reference is made specifically to amino acid sequences set forth in SEQ ID NOs: 17 - 22 and 43 - 47 of said reference.

In some embodiments, the parent glucoamylase is TrGA comprising the amino acid sequence of SEQ ID NO:2 or a *Trichoderma* glucoamylase homologue having at least 80%, at least 85%, at least 88%, at least 90%, at least 93%, at least 95%, at least 96%, at least 97%, at least 98%, at least 99% sequence identity to the TrGA sequence.

A parent glucoamylase of the present disclosure can be isolated and/or identified using standard recombinant DNA techniques. Any standard techniques can be used that are known to the skilled artisan. For example, probes and/or primers specific for conserved areas of the glucoamylase can be used to identify homologues in bacterial or fungal cells (the catalytic domain, the active site, etc.). Alternatively degenerate PCR can be used to identify homologues in bacterial or fungal cells. In some cases, known sequences, such as in a database, can be analyzed for sequence and/or structural homology to one of the known glucoamylases, including SEQ ID NOs: 1, 2 or 3. Functional assays can also be used to identify glucoamylase activity in a bacterial or fungal cell. Proteins having glucoamylase activity can be isolated and reverse sequenced to isolate the corresponding DNA sequence. Such methods are known to the skilled artisan.

**Glucoamylase Structural Homology:**

The central dogma of molecular biology is that the sequence of DNA encoding a gene for a particular enzyme, determines the amino acid sequence of the protein, this sequence in turn determines the three-dimensional folding of the enzyme. This folding brings together disparate residues that create a catalytic center and substrate binding surface and this results in the high specificity and activity of the enzymes in question.

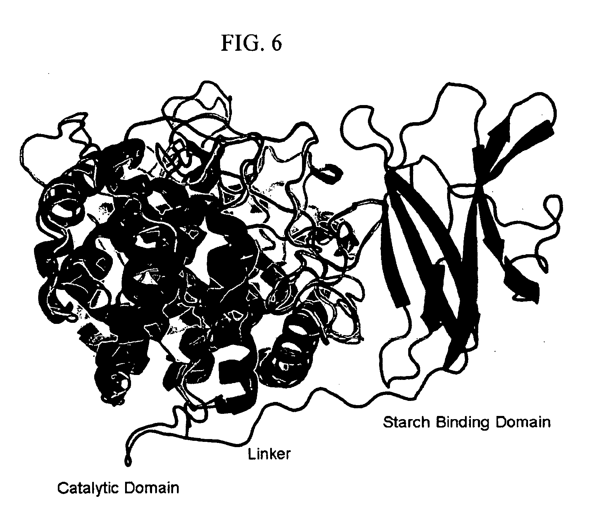

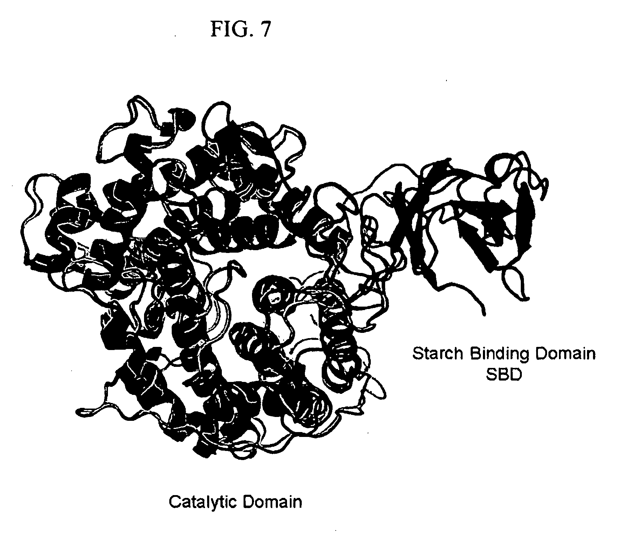

Glucoamylases consist of as many as three distinct structural domains, a catalytic domain of approximately 450 residues which is structurally conserved, generally followed by a linker region consisting of between 30 and 80 residues which are connected to a starch binding domain of approximately 100 residues. The structure of the *Trichoderma reesei* glucoamylase with all three regions intact was determined to 1.8 Angstrom resolution herein (see Table 9 and Examples 8 and 9). Using the coordinates (see Table 9) the catalytic structure was aligned with the coordinates of the catalytic domain from *Aspergillus awamorii* strain X100 that was determined previously (Aleshin, et al (1994) J Mol Biol 238: 575-591). The *Aspergillus awamori* crystal structure only included the catalytic domain. As seen in Figures 6 and 7 the structure of the catalytic domains overlap very closely and it is possible to identify equivalent residues based on this structural superposition. The inventors believe that all glucoamylases share the basic structure depicted in Figures 6 and 7. The conservation of structure in the glucoamylase molecule correlates with the conservation of activity and a conserved mechanism of action for all glucoamylases. Given this high homology, site specific variants of the *Trichoderma* glucoamylase resulting in altered function would also have similar structural and therefore functional consequences in other glucoamylases.

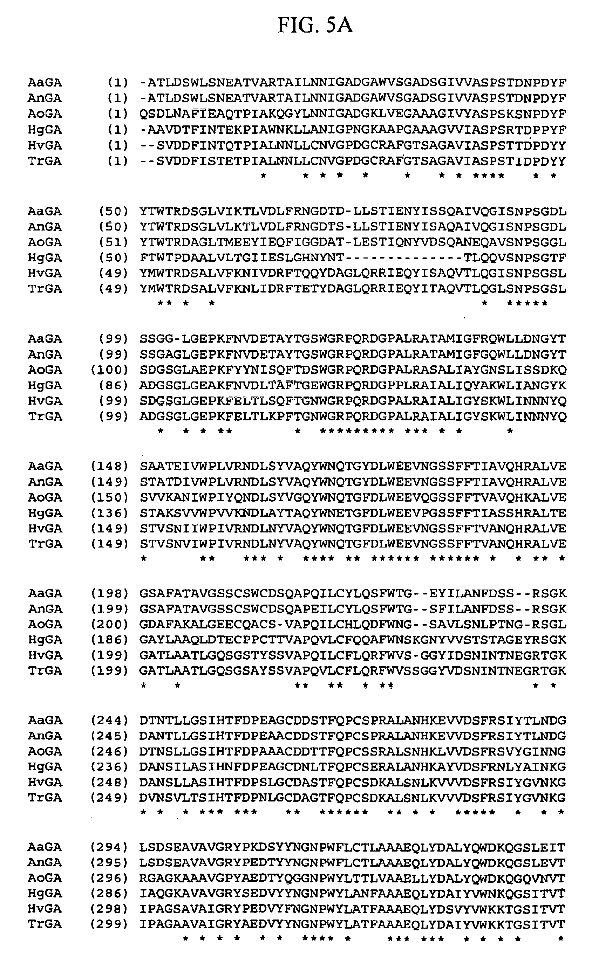

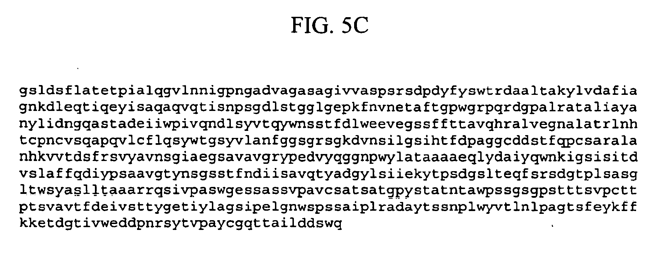

The present disclosure, however, is not limited to the variants of the parent *Trichoderma* glucoamylase disclosed in Figure 1, but extends to variants of parent glucoamylases comprising amino acid residues at positions which are "equivalent" to the particular identified residues in *Trichoderma reesei* glucoamylase (SEQ ID NO:2). In some embodiments, of the present disclosure, the parent glucoamylase is a *Talaromyces* GA and the substitutions are made at the equivalent amino acid residue positions in *Talaromyces* glucoamylase (see e.g. SEQ ID NO:308, Figure 5C) as those described herein. In other embodiments, the parent glucoamylase comprises SEQ ID NO:5; SEQ ID NO:6; SEQ ID NO:7; SEQ ID NO:3; SEQ ID NO:8; and SEQ ID NO:9 (See Figures 5A- 5B). In further embodiments, the parent glucoamylase is a *Penicillium* glucoamylase, such as *Penicillium chrysogenum.*

Structural identity determines whether the amino acid residues are equivalent. Structural identity is a one-to-one topological equivalent when the two structures (three dimensional and amino acid structures) are aligned. A residue (amino acid) position of a glucoamylase is equivalent to a residue of *T. reesei* glucoamylase if it is either homologous (i.e., corresponding in position in either primary or tertiary structure) or analogous to a specific residue or portion of that residue in *T. reesei* glucoamylase (having the same or similar functional capacity to combine, react, or interact chemically).

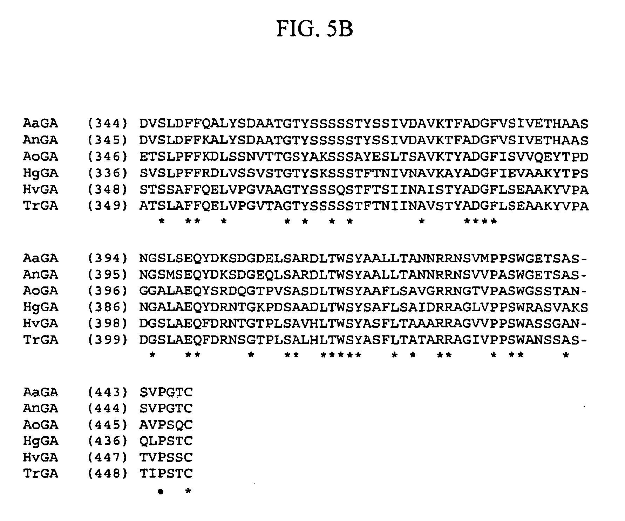

In order to establish identity to the primary structure, the amino acid sequence of a glucoamylase can be directly compared to *Trichoderma reesei* glucoamylase primary sequence and particularly to a set of residues known to be invariant in glucoamylases for which sequence is known. For example, Figures 5A and B herein shows the conserved residues between glucoamylases. After aligning the conserved residues, allowing for necessary insertions and deletions in order to maintain alignment (i.e. avoiding the elimination of conserved residues through arbitrary deletion and insertion), the residues equivalent to particular amino acids in the primary sequence of *Trichoderma reesei* glucoamylase are defined. Alignment of conserved residues can conserve 100% of such residues. However, alignment of greater than 75% or as little as 50% of conserved residues is also adequate to define equivalent residues, particularly when alignment based on structural identity is included.

For example, in Fig. 5, glucoamylases from six organisms are aligned to provide the maximum amount of homology between amino acid sequences. A comparison of these sequences shows that there are a number of conserved residues contained in each sequence as designated by an asterisk. These conserved residues, thus, may be used to define the corresponding equivalent amino acid residues of *Trichoderma reesei* glucoamylase in other glucoamylases such as glucoamylase from *Aspergillus niger.*

Structural identity involves the identification of equivalent residues between the two structures. "Equivalent residues" can be defined by determining homology at the level of tertiary structure (structural identity) for an enzyme whose tertiary structure has been determined by x-ray crystallography. Equivalent residues are defined as those for which the atomic coordinates of two or more of the main chain atoms of a particular amino acid residue of the *Trichoderma reesei* glucoamylase (N on N, CA on CA, C on C and O on O) are within 0.13nm and preferably 0.1 nm after alignment. Alignment is achieved after the best model has been oriented and positioned to give the maximum overlap of atomic coordinates of non-hydrogen protein atoms of the glucoamylase in question to the *Trichoderma reesei* glucoamylase. The best model is the crystallographic model giving the lowest R factor for experimental

$$R\ factor = \frac{\Sigma_h\,|\,Fo(h)\,|\,\text{-}\,|\,Fc(h)\,|}{\Sigma_h\,|\,Fo(h)\,|}$$

diffraction data at the highest resolution available.

Equivalent residues which are functionally analogous to a specific residue of *Trichoderma reesei* glucoamylase are defined as those amino acids of the enzyme which may adopt a conformation such that they either alter, modify or contribute to protein structure, substrate binding or catalysis in a manner defined and attributed to a specific residue of the *Trichoderma reesei* glucoamylase. Further, they are those residues of the enzyme (for which a tertiary structure has been obtained by x-ray crystallography) which occupy an analogous position to the extent that, although the main chain atoms of the given residue may not satisfy the criteria of equivalence on the basis of occupying a homologous position, the atomic coordinates of at least two of the side chain atoms of the residue lie with 0.13nm of the corresponding side chain atoms of *Trichoderma reesei* glucoamylase. The coordinates of the three dimensional structure of *Trichoderma reesei* glucoamylase are set forth in Table 9 and can be used as outlined above to determine equivalent residues on the level of tertiary structure.

**Variants:**

The variants according to the present disclosure include at least one substitution, deletion or insertion in the amino acid sequence of a parent glucoamylase that makes the variant different in sequence from the parent glucoamylase. In some embodiments, the variants of the present disclosure will have at least 20%, at least 40%, at least 50%, at least 60%, at least 70%, at least 80%, at least 85%, at least 90%, at least 95%, at least 97% and also at least 100% of the glucoamylase activity of TrGA (SEQ ID NO:2). In some embodiments, the variants will comprise a substitution, deletion or insertion in at least one amino acid position of the parent TrGA (SEQ ID NO:2), or in an equivalent position in the sequence of another parent glucoamylase having at least 50%, at least 60%, at least 70%, at least 80%, at least 90% sequence identity to the TrGA sequence, including but not limited to; at least 93% sequence identity, at least 95%, at least 97%, and at least 99% sequence identity. In some embodiments, the parent glucoamylase will have structural identity to the TrGA sequence.

In other embodiments, the variant according to the present disclosure will comprise a substitution, deletion or insertion in at least one amino acid position of a fragment of the parent TrGA, wherein the fragment comprises the catalytic domain of the TrGA sequence (SEQ ID NO:3) or in an equivalent position in a fragment comprising the catalytic domain of a parent glucoamylase having at least 50%, at least 60%, at least 70%, at least 80% sequence identity to the fragment of the TrGA sequence, at least 90%, at least 95%, at least 97%, and at least 99% sequence identity. In some embodiments, the fragment will comprise at least 400, 425, 450, and/or 500 amino acid residues. In some embodiments, when the parent glucoamylase includes a catalytic domain, linker region and starch binding domain, the fragment may include part of the linker region. In some embodiments, the variant will comprise a substitution, deletion or insertion in the amino acid sequence of a fragment of the TrGA sequence (SEQ ID NO: 2 or SEQ ID NO: 3). In some embodiments, the variant will have structural identity with the TrGA sequence (SEQ ID NOs: 2 or 3).

Structural identity with reference to an amino acid substitution, means that the substitution occurs at the equivalent amino acid position in the homologous glucoamylase or parent glucoamylase. The term equivalent position means a position that is common to two parent sequences which is based on an alignment of the amino acid sequence of the parent glucoamylase in question as well as alignment of the three-dimensional structure of the parent glucoamylase in question with the TrGA reference glucoamylase amino acid sequence and three-dimensional sequence. For example, with reference to Fig. 5, position 24 in TrGA (SEQ ID NO: 3) is D24 and the equivalent position for *Aspergillus niger* (SEQ ID NO. 6) is position D25 and the equivalent position for *Aspergillus oryzae* (SEQ ID NO: 7) is position D26. See Figures 6 and 7 for an exemplary alignment of the three-dimensional sequence.

In some embodiments, the glucoamylase variant will have more than one substitution (*e.g.* two, three or four substitutions) as compared to a corresponding parent glucoamylase.

In some embodiments, a glucoamylase variant comprises a substitution, deletion or insertion in at least one amino acid position in a position corresponding to the regions of non-conserved amino acids as illustrated in Figures 5A and 5B (*e.g.* amino acid positions corresponding to those positions which are not designated by "*" in Figures 5A and 5B). In some embodiments, the variant comprises a substitution in at least one amino acid position in a position corresponding to the regions of non-conserved amino acids as illustrated in Figures 5A and 5B.

While the variants can be in any position in the mature protein sequence (SEQ ID NOs: 2 or 3), in some embodiments, a glucoamylase variant comprises one or more substitutions in the following positions in the amino acid sequence set forth in SEQ ID NOs: 2 or 3: 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182, 204, 205, 214, 216, 219, 228, 229, 230, 231, 236, 239, 240, 241, 242, 244, 263, 264, 265, 268, 269, 276, 284, 291, 300, 301, 303, 310, 311, 313, 316, 338, 342, 344, 346, 349, 359, 361, 364, 375, 379, 382, 390, 391, 393, 394, 408, 410, 415, 417, 418, 430, 431, 433, 436, 442, 443, 444, 448 and 451, or in an equivalent position in a parent glucoamylase. In some embodiments, the parent glucoamylase will have at least 50%, at least 60%, at least 70%, at least 80%, at least 90%, at least 95%, at least 96%, at least 97% at least 98%, and at least 99% sequence identity with SEQ ID NO: 2 or SEQ ID NO: 3. In other embodiments, the parent glucoamylase will be a *Trichoderma* glucoamylase homologue. In some embodiments, the variant will have altered properties. In some embodiments, the parent glucoamylase will have structural identity with the glucoamylase of SEQ ID NOs: 2 or 3.

In some embodiments, the glucoamylase variant comprises one or more substitutions in the following positions in the amino acid sequence set forth in SEQ ID NO: 2 or 3: T10, L14, N15, P23, T42, P45, D46, F59, K60, N61, T67, E68, A72, G73, S97, L98, A99, S102, K108, E110, L113, K114, R122, Q124, R125, I133, K140, N 144, N 145, Y147, S152, N 153, N164, F 175, N 182, A204, T205, S214, V 216, Q219, W228, V229, S230, S231, D236, I239, N240, T241, N242, G244, N263, L264, G265, A268, G269, D276, V284, S291, P300, A301, A303, Y310, A311, D313, Y316, V338, T342, S344, T346, A349, V359, G361, A364, T375, N379, S382, S390, E391, A393, K394, R408, S410, S415, L417, H418, T430, A431, R433, I436, A442, N443, S444, T448 and S451 or an equivalent position in parent glucoamylase (*e.g.* a *Trichoderma* glucoamylase homologue). In some embodiments, the variant will have altered properties as compared to the parent glucoamylase.

In other embodiments, the variant of a glucoamylase parent comprises one or more substitutions in the following positions in the amino acid sequence set forth in SEQ ID NO:2 or 3: 10, 14, 15, 23, 59, 60, 61, 65, 67, 68, 72, 73, 97, 98, 99, 102, 110, 113, 133, 140, 144, 145, 147, 152, 153, 164, 182, 204, 205, 214, 216, 219, 228, 229, 230, 231, 236, 239, 241, 242, 263, 264, 265, 268, 269, 276, 284, 291, 300, 301, 303, 311, 338, 342, 344, 346, 349, 359, 361, 364, 375, 379, 382, 390, 391, 393, 394, 410, 417, 418, 430, 431, 433, 442, 444, 448, and 451 or an equivalent position in a parent glucoamylase. In some embodiments, the parent glucoamylase will have 50%, at least 60%, at least 70%, at least 80%, at least 90%, at least 95%, at least 96%, at least 97% at least 98%, and at least 99% sequence identity with SEQ ID NO: 2 or SEQ ID NO: 3. In other embodiments the parent glucoamylase will be a *Trichoderma* glucoamylase homologue. In some embodiments, the variant will have at least one altered property as compared to the parent glucoamylase. In some embodiments, the at least one altered property is specific activity. In some embodiments, the variant has one or more substitutions corresponding to one of the following positions: 61, 67, 72, 97, 102, 133, 205, 219, 228, 230, 231, 239, 263, 268, 291, 342, 394, 430, 431 and 451 of SEQ ID NO: 2 and/or 3. In some embodiments, the substitution at these positions is chosen from: N61I, T67M, A72Y, S97N, S102M, I133T, T205Q, Q219S, W228H, W228M, S230F, S230G, S230N, S230R, S231L, I239V, I239Y, N263P, A268C, A268G, A268K, S291A, T342V, K394S, T430K, A431Q, and S451K of SEQ ID NO: 2 and/or 3. In some embodiments, the variant has one or more substitutions corresponding to one of the following positions: 72, 133, 219, 228, 230, 231, 239, 263, 268, and 451 of SEQ ID NO: 2 and/or 3. In some embodiments, the substitution at these positions is chosen from: A72Y, I133T, Q219S, W228H, W228M, S230R, S230F, S230G, S231 L, I239V, N263P, A268C, A268G, and S451K of SEQ ID NO: 2 and/or 3. In some embodiments, the variant has at least one altered property and the at least one altered property is an increased specific activity as compared to the parent glucoamylase.

In further embodiments, the variant of a glucoamylase parent comprises at least one of the following substitutions in the following positions in an amino acid sequence set forth in SEQ ID NO:2 or 3: T10D/F/G/K/L/M/P/R/S; L14E/H; N15D/N; P23A/G; F59A/G; K60F/H; N61D/UL/Q/V/W; R65A/C/G/I/K/M/S/V/Y; T67C/I/K/M/T; E68I/M/W; A72E/G/L/M/Q/-R/W/Y; G73C/L/W; S97F/M/N/P/R/S/V/W/Y; L98H/M; A99C/L/M/N/P; S102A/C/IL/M/N/R/V/W/Y; E110Q/S/W; L113E/N; K114C/D/E/L/M/Q/S/T/V; I133K/R/S/T; K140A/E/F/H/K/L/M/N/Q/R/S/V/W/Y; N144C/D/E/I/K; N145A/C/E/I/K/L/M/Q/R/-V/W/Y; Y147A/M/R; S152H/M; N153C/D/K/L/W/Y; N164A/G; N182C/E/K/P/R; A204C/D/G/I/M/Q/T; T205A/D/H/I/K/-M/N/P/Q/S/V/W/Y; S214P/T; V216C/G/H/K/N/Y; Q219D/G/H/N/P/S; W228A/F/G/H/VL/M/Q/S/T/V/Y; V229E/I/M/N/Q; S230C/D/E/F/G/H/I/K/L/N/P/Q/R/T/V/Y; S231C/D/F/L/M/N/Q/R/S/V/Y; D236F/G/L/M/P/S/T/V; I239M/Q/SN/W/Y; T241C/E/H/L/M/P/S/T/V; N242C/F/H/M/T/V/W; N263H/K/P; L264A/C/E/F/L/S; G265E/G/H/I/K/R/T; A268C/D/E/F/G/IK/L/P/R/T/W; G269E; D276S; V284R/T/V/Y/A/E/F/H/K/N/P/W; P300K/R; A301 E/K/L/P/S/W; A303C/D/F/H/I/K/L/N/R/T/V/W/Y; A311N/P/Q/S/Y; V338P/Q/S/Y; T342N/V; S344A/T; T346G/H/M/N/P/Q/Y; A349L/IK/M/N/Q/R/W; G361H/L/R; A364M/W; T375C/D/E/H/V/W/Y; N379A/C/D/G/I/M/P/S; S382A/N/P/V/W; S390A/Y; E391 A/E/I/K/L/M/Q/R/V/W/Y; A393E/G/H/I/K/L/M/N/Q/R/S/TN/W/Y; K394A/H/K/L/M/Q/R/S/T/V/W; S410E/H/N; L417A/D/E/F/G/I/K/Q/R/S/T/V/W/Y; H418E/M; T430A/E/F/G/H/I/K/M/N/Q/R/V; A431C/E/H/IL/M/Q/R/S/W/Y; R433A/C/E/F/G/K/L/M/N/S/V/W/Y; 1436E/F/G/H/K/P/R/S/T/V/Y; S444M/N/P/Q/R/T/V/W; T448F/G/I/P/Q/T/V; and S451 E/H/K/L/Q/T or a substitution in an equivalent position in a parent glucoamylase.

In other embodiments, the variant of a glucoamylase parent comprises one or more substitutions in the following positions in the amino acid sequence set forth in SEQ ID NO:2 or 3: 10, 15, 23, 42, 59, 60, 61, 68, 72, 73, 97, 98, 99, 102, 114, 133, 140, 144, 147, 152, 153, 164, 182, 204, 205, 214, 216, 228, 229, 230, 231, 236, 241, 242, 263, 264, 265, 268, 269, 276, 284, 291, 300, 301, 303, 311, 338, 342, 344, 346, 349, 359, 361, 364, 375, 379, 382, 390, 391, 393, 394, 410, 417, 430, 431, 433, 436, 442, 443, 444, 448, and 451 or an equivalent position in a parent glucoamylase (*e.g., Trichoderma* glucoamylase homologue). In some embodiments, the parent glucoamylase will have at least 50%, at least 60%, at least 70%, at least 80%, at least 90%, at least 95%, at least 96%, at least 97% at least 98%, and at least 99% sequence identity with SEQ ID NO: 2 or SEQ ID NO: 3. In some embodiments, the parent glucoamylase also shows structural identity with the glucoamylase of SEQ ID NOs: 2 and/or 3. In other embodiments the parent glucoamylase will be a *Trichoderma* glucoamylase homologue. In some embodiments, the variant has one or more substitutions corresponding to one of the following positions: 10, 42, 68, 73, 97, 114, 153, 229, 231, 236, 264, 291, 301, 344, 361, 364, 375, 417, and 433 of SEQ ID NO: 2 and/or 3. In some embodiments, the substitution at these positions is chosen from: T10S, T42V, E68C, E68M, G73F, G73W, K114M, K114T, N153A, N153S, N153V, W228V, D236R, G361D, G36E, G361P, G361Y, A364D, A364E, A364F, A364G, A364K, A365L, A365R, R433C, R433G, R433L, R433N, R433S, R433V, and 1436H of SEQ ID NO: 2 and/or 3. In some embodiments, the variant has one or more substitutions corresponding to one of the following positions: 42, 68, 73, 114, 153, 236, 361, and 364 of SEQ ID NOs: 2 and/or 3. In some embodiments, the substitution at these positions is chosen from: T42V, E68M, G73F, G73W, K114T, N153S, N153V, D236R, G361D, A364F, and A364L of SEQ ID NO: 2 and/or 3. In some embodiments, the variant has at least one altered property and the at least one altered property is an increased thermostability as compared to the parent glucoamylase.

In other embodiments, the variant of a glucoamylase parent comprises one or more substitutions in the following positions in the amino acid sequence set forth in SEQ ID NOs:2 or 3: 10, 15, 59, 61, 68, 72, 73, 97, 99, 102, 133, 140, 153, 182, 204, 205, 214, 228, 229, 230, 231, 236, 241, 242, 264, 265, 268, 275, 284, 291, 300, 301, 303, 31 I, 338, 344, 346, 359, 361, 364, 375, 370, 382, 391, 393, 394, 410, 417, 430, 431, 433, 444, 448, and 451 and/or an equivalent position in a parent glucoamylase (e.g., *Trichoderma* glucoamylase homologue). In some embodiments, the parent glucoamylase will have at least 50%, at least 60%, at least 70%, at least 80%, at least 90%, at least 95%, at least 96%, at least 97% at least 98%, and at least 99% sequence identity with SEQ ID NO: 2 or SEQ ID NO: 3. In some embodiments, the parent glucoamylase will also have structural identity with the glucoamylase of SEQ ID NOs: 2 and/or 3. In other embodiments the parent glucoamylase will be a *Trichoderma* glucoamylase homologue. In some embodiments, the variant will have at least one altered property as compared to the parent glucoamylase. In some embodiments, the variant has one or more substitutions corresponding to one of the following positions: 228, 230, 231, 268, 291, 417, 433, and 451 of SEQ ID NOs: 2 and/or 3. In some embodiments, the substitution at these positions is chosen from: W228H, W228M, S230F, S230G, S230R, S231L, A268C, A268G, S291A, L417R, R433Y, and S451K of SEQ ID NOs: 2 and/or 3. In some embodiments, the variant has at least one altered property and the at least one altered property is an increased thermostability or specific activity as compared to the parent glucoamylase. In some embodiments, the variant has both an increased thermostability and an increased specific activity as compared to the parent glucoamylase.

Glucoamylase variants of the present disclosure may also include chimeric or hybrid glucoamylases with, for example a starch binding domain (SBD) from one glucoamylase and a catalytic domain and linker from another. For example, a hybrid glucoamylase can be made by swapping the SBD from AnGA with the SBD from TrGA, making a hybrid with the AnGA SBD and the TrGA catalytic domain and linker. Alternatively, the SBD and linker from AnGA can be swapped for the SBD and linker of TrGA.

In some aspects, the variant glucoamylase exhibits increased thermostability as compared to the parent glucoamylase. In some embodiments, the altered property is increased specific activity compared to the parent glucoamylase. In some embodiments, the altered property is increased thermostability at lower temperatures as compared to the parent glucoamylase. In some embodiments, the altered property is both increased specific activity and increased thermostability as compared to the parent glucoamylase.

A number of parent glucoamylases have been aligned with the amino acid sequence of TrGA. Fig. 5 includes the catalytic domain of the following parent glucoamylases *Aspergillus awamori* (AaGA) (SEQ ID NO:5); *Aspergillus niger* (AnGA) (SEQ ID NO:6); *Aspergillus orzyae* (AoGA) (SEQ ID NO:7), *Humicola grisea* (HgGA) (SEQ ID NO:8) and *Hypocrea vinosa* (HvGA) (SEQ ID NO:9). The % identity of the catalytic domains is represented in Table 1 below.

Table 1

|      | AaGA | AnGA | AoGA | HgGA | HvGA | TrGA |
|------|------|------|------|------|------|------|
| AaGA | 100  | 95   | 58   | 53   | 57   | 56   |
| AnGA |      | 100  | 59   | 53   | 57   | 56   |
| AoGA |      |      | 100  | 55   | 56   | 56   |
| HgGA |      |      |      | 100  | 61   | 63   |
| HvGA |      |      |      |      | 100  | 91   |
| TrGA |      |      |      |      |      | 100  |

In some embodiments, for example, the variant glucoamylase will be derived from a parent glucoamylase which is an *Aspergillus* glucoamylase and the variant will include at least one substitution in a position equivalent to a position set forth in SEQ ID NO:2 or SEQ ID NO:3 and particularly in a position corresponding to: T10, L14, N15, P23, T42, P45, D46, F59, K60, N61, R65, T67, E68, A72, G73, S97, L98, A99, S102, K108, E110, L113, K114, R122, Q124, R125, 1133, K140, N144, N 145, Y147, S152, N 153, N164, F175, N 182, A204, T205, S214, V216, Q219, W228, V229, S230, S231, D236, I239, N240, T241, N242, G244, N263, L264, G265, A268, G269, D276, V284, S291, P300, A301, A303, Y310, A311, D313, Y316, V338, T342, S344, T346, A349, V359, G361, A364, T375, N379, S382, S390, E391, A393, K394, R408, S410, S415, L417, H418, T430, A431, R433, I436, A442, N443, S444, T448 and S451.

## Altered Properties

The present disclosure also provides glucoamylase variants having at least one altered property (*e.g.,* improved property) as compared to a parent glucoamylase and particularly to the TrGA. In some embodiments, at least one altered property (*e.g.* improved property) is chosen from acid stability, thermal stability and specific activity. In some embodiments, the altered property is increased acid stability, increased thermal stability and/or increased specific activity. In some embodiments, the increased thermal stability is at higher temperatures. In some embodiments, the increased pH stability is at high pH. In further embodiments, the increased pH stability is at low pH.

The glucoamylase variants of the present disclosure may also provide higher rates of starch hydrolysis at low substrate concentrations as compared to the parent glucoamylase. The variant may have a higher Vmax or lower Km than a parent glucoamylase when tested under the same conditions. For example the variant glucoamylase may have a higher Vmax at a temperature range of 25°C to 70°C (e.g. at 25°C to 35°C; 30°C - 35°C; 40°C to 50°C; at 50°C to 55°C and at 55°C to 62°C). The Michaelis-Menten constant, Km and Vmax values can be easily determined using standard known procedures.

## Thermal stability (Thermostable variants):

In some aspects, the invention relates to a variant glucoamylase having altered thermal stability as compared to a parent (wild type) as defined in the claims. Altered thermostability can be at increased temperatures or at decreased temperatures. Thermostability is measured as the % residual activity after incubation for 1 hour at 64 degree centigrade in NaAc buffer pH 4.5. Under these conditions TrGA has a residual activity of between about 15% and 44% due to day-to-day variation as compared to the initial activity before incubation. Thus, in some embodiments, variants with increased thermostability of the present disclosure have a residual activity that is between at least 1% and at least 50% more than that of the parent (after incubation for 1 hour at 64 degrees centigrade in NaAc buffer pH 4.5), including 2%, 3%, 4%, 5%, 6%, 7%, 8%, 9%, 10%, 11%, 12%,13%,14%,15%,16%,17%,18%, 19%, 20%, 21 %, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%,48%, 49%, and 50% as compared to the initial activity before incubation. For example, when the parent residual activity is 15%, a variant with increased thermal stability may have a residual activity of between about 16% and about 75%. In some embodiments, the glucoamylase variant will have improved thermostability such as retaining at least 50%, 60%, 70%, 75%, 80%, 85%, 90%, 92%, 95%, 96%, 97%, 98% or 99% enzymatic activity after exposure to altered temperatures over a given time period, for example, at least 60 minutes, 120 minutes, 180 minutes, 240 minutes, 300 minutes, etc. In some embodiments, the variant has increased thermal stability compared to the parent glucoamylase at selected temperatures in the range of 40 to 80°C, also in the range of 50 to 75°C and in the range of 60 to 70°C, and at a pH range of 4.0 to 6.0. In some embodiments, the thermostability is determined as described in the Examples. In some embodiments, the variant has increased thermal stability at lower temperature compared to the parent glucoamylase at selected temperature in the range of 20 to 50°C, including 35 to 45 and 30°C to 40°C.

In some embodiments, variants having an improvement in thermostability include one or more deletions, substitutions or insertions and particularly substitutions in the following positions in the amino acid sequence set forth in SEQ ID NO:2 or SEQ ID NO:3: T10, N15, P23, T42, F59, K60, N61, E68, A72, G73, S97, L98, A99, S102, K114, I133, K140, N144, Y147, S152, N153, N164, N182, A204, T205, S214, V216, W228, V229, S230, S231, D236, T241, N242, N263, L264, G265, A268, G269, D276, V284, S291, P300, A301, A303, A311, V338, T342, S344, T346, A349, V359, G361, A364, T375, N379, S382, S390, E391, A393, K394, S410, L417, T430, A431, R433, I436, A442, N443, S444, T448 and S451 and/or an equivalent position in a parent glucoamylase. In some embodiments, the parent glucoamylase will be a *Trichoderma* glucoamylase homologue and in further embodiments, the parent glucoamylase will have at least 50%, at least 60%, at least 70%, at least 80%, at least 90%, at least 95% and at least 98% sequence identity to SEQ ID NOs:2 or 3. In some embodiments, the parent glucoamylase will also have structural identity to SEQ ID NOs: 2 and/or 3. In some embodiments, the variant having increased thermostability has a substitution in at least one of the positions: 10, 42, 68, 73, 97, 153, 229, 231, 236, 264, 291, 301, 344, 361, 364, 375, and/or 417 of SEQ ID NO: 2 and/or 3. In some embodiments, the variant having thermostability has a substitution in at least one of the positions: 42, 68, 73, 153, 236, 344, 361, 364, and 365 of SEQ ID NO: 2 or SEQ ID NO:3.

**Specific activity:**

As used herein, specific activity is the activity of the glucoamylase per mg of protein. Activity was determined using the ethanol assay. The screening identified variants having a Performance Index (PI) > 1.0 compared to the parent TrGA PI. The PI is calculated from the specific activities (activity/mg enzyme) of the wildtype (WT) and the variant enzymes. It is the quotient "Variant-specific activity/WT-specific activity" and can be a measure of the increase in specific activity of the variant. A PI of 2 should be about 2 fold better than WT In some aspects, the invention relates to a variant glucoamylase having increased specific activity as compared to a parent or wildtype glucoamylase as defined in the claims. Increased specific activity can be defined as an increased performance index of greater than or equal to about 1, including greater than or equal to about 1.1, 1.2, 1.3, 1.4, 1.5, 1.6, 1.7, 1.8, 1.9, and 2. In some embodiments of the present disclosure, the increased specific activity is from about 1.0 to about 5.0, including 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, and 4.9. In some embodiments, the variant of the present disclosure has an at least 1 fold higher specific activity than the parent glucoamylase, including at least 1.1 fold, 1.2 fold, 1.3 fold, 1.4 fold, 1.5 fold, 1.6 fold, 1.7 fold, 1.8 fold, 1.9 fold, 2 fold, 2.2 fold, 2.5 fold, 2.7 fold, 2.9 fold, 3 fold, 4 fold, and 5 fold.

In some embodiments, variants of the present disclosure having an improvement in specific activity include one or more deletions, substitutions or insertions in the following positions in the amino acid sequence set forth in SEQ ID NO:2 and/or 3: T10, L14, N15, P23, F59, K60, N61, T67, E68, A72, G73, S97, L98, A99, S102, E110, L113, I133, K140, N144, N145, Y147, S152, N153, N164, N182, A204, T205, S214, V216, Q219, W228, V229, S230, S231, D236, I239, T241, N242, N263, L264, G265, A268, G269, D276, V284, S291, P300, A301, A311, V338, T342, S344, T346, A349, V359, G361, A364, T375, N379, S382, S390, E391, A393, K394, S410, S415, L417, H418, T430, A431, R433, A442, S444, T448 and/or S451 and/or an equivalent position in a parent glucoamylase. In some embodiments, variants of the present disclosure having improved specific activity include a substitution in the following positions in the amino acid sequence set forth in SEQ ID NO: 2 or 3: 61, 67, 72, 97, 102, 133, 205, 219, 228, 230, 231, 239, 263, 268, 291, 342, 394, 430, 431 and 451 and/or an equivalent position in a parent glucoamylase. In some embodiments, the parent glucoamylase will comprise a sequence having at least 50%, at least 60%, at least 70%, at least 80%, at least 90% or 95% sequence identity to the sequence of SEQ ID NO:2 or 3. In some embodiments, the parent glucoamylase will also have structural identity to SEQ ID NO: 2 and/or 3. In some embodiments, the variant having increased specific activity has a substitution in at least one of the positions: 72, 133, 219, 228, 230, 231, 239, 263, 268, and 451 of SEQ ID NO: 2 and/or 3.

**Combined Thermostability and Specific Activity**

In some aspects, the invention relates to a variant glucoamylase as defined in the claims having both altered thermostability and altered specific activity as compared to a parent (*e.g.,* wildtype). In some embodiments, the altered specific activity is an increased specific activity. In some embodiments, the altered thermostability is an increased thermostability at high temperatures (*e.g.,* at temperatures above 80°C) as compared to the parent glucoamylase.

In some embodiments, variants of the present disclosure with an increased thermostability and increased specific activity include one or more deletions, substitutions or insertions and substitutions in the following positions in the amino acid sequence set forth in SEQ ID NOs:2 or 3: T10, N 15, F59, N61, E68, A72, G73, S97, A99, S102, I133, K140, N153, N182, A204, T205, S214, W228, V229, S230, S231, D236, T241, N242, L264, G265, A268, D276, V284, S291, P300, A301, A303, A311, V338, S344, T346, V359, G361, A364, T375, N379, S382, E391, A393, K394, S410, L417, T430, A431, R433, S444, T448 and/or S451 and/or an equivalent position in a parent glucoamylase. In some embodiments, the parent glucoamylase will be a *Trichoderma* glucoamylase homologue and in further embodiments, the parent glucoamylase will have at least 50%, at least 60%, at least 70%, at least 80%, at least 90%, at least 95% and at least 98% sequence identity to SEQ ID NO:2 or 3. In some embodiments, the parent glucoamylase will also have structural identity to SEQ ID NO: 2 and/or 3. In some embodiments, the variant having increased thermostability and specific activity has a substitution in at least one of the positions: 228, 230, 231, 268, 291, 417, 433, and 451 of SEQ ID NO: 2 and/or 3.

## Polynucleotides:

The present invention also relates to isolated polynucleotides encoding a variant glucoamylase of the invention, as defined in the claims. The polynucleotides may be prepared by established techniques known in the art. The polynucleotides may be prepared synthetically, such as by an automatic DNA synthesizer. The DNA sequence may be of mixed genomic (or cDNA) and synthetic origin prepared by ligating fragments together. The polynucleotides may also be prepared by polymerase chain reaction (PCR) using specific primers. In general, reference is made to Minshull J., et al., (2004), Engineered protein function by selective amino acid diversification, Methods 32(4):416-427. DNA may also be synthesized by a number of commercial companies such as Geneart AG, Regensburg, Germany.

The present disclosure also provides isolated polynucleotides comprising a nucleotide sequence (i) having at least 50% identity to SEQ ID NO:4, including at least 60%, at least 70%, at least 80%, at least 90%, at least 95%, and at least 99%, or (ii) being capable of hybridizing to a probe derived from the nucleotide sequence set forth in SEQ ID NO:4, under conditions of intermediate to high stringency, or (iii) being complementary to a nucleotide sequence having at least 90% sequence identity to the sequence set forth in SEQ ID NO:4. Probes useful according to the present disclosure may include at least 50, 100, 150, 200, 250, 300 or more contiguous nucleotides of SEQ ID NO:4. In some embodiments, the encoded polypeptide also has structural identity to SEQ ID NOs: 2 and/or 3.

The present disclosure further provides isolated polynucleotides that encode variant glucoamylases which comprise an amino acid sequence comprising at least 50%, at least 60%, at least 70%, at least 80% , at least 90%, at least 93%, at least 95%, at least 97%, at least 98%, or at least 99% amino acid sequence identity to SEQ ID NO:2. Additionally, the present invention provides expression vectors comprising any of polynucleotides, as defined in the claims.

The present disclosure also provides fragments (*i.e.,* portions) of the DNA encoding the variant glucoamylases provided herein. These fragments find use in obtaining partial length DNA fragments capable of being used to isolate or identify polynucleotides encoding mature glucoamylase enzymes described herein from filamentous fungal cells (e.g., *Trichoderma, Aspergillus, Fusarium, Penicillium,* and *Humicola*), or a segment thereof having glucoamylase activity. In some embodiments, fragments of the DNA may comprise at least 50, 100, 150, 200, 250 300 or more contiguous nucleotides. In some embodiments, portions of the DNA provided in SEQ ID NO:4 find use in obtaining parent glucoamylase and particularly *Trichoderma* glucoamylase homologues from other species, such as filamentous fungi which encode a glucoamylase.

## DNA constructs and Vectors:

According to one embodiment of the invention, a DNA construct comprising a polynucleotide as described above encoding a variant glucoamylase encompassed by the invention and operably linked to a promoter sequence is assembled to transfer into a host cell.

The DNA construct may be introduced into a host cell using a vector. The vector may be any vector which when introduced into a host cell is stably introduced. In some embodiments, the vector of the present disclosure is integrated into the host cell genome and is replicated. Vectors include cloning vectors, expression vectors, shuttle vectors, plasmids, phage particles, cassettes and the like. In some embodiments, the vector is an expression vector that comprises regulatory sequences operably linked to the glucoamylase coding sequence.

Examples of suitable expression and/or integration vectors are provided in Sambrook *et al.,* (1989) *supra,* and Ausubel (1987) *supra,* and van den Hondel *et al*. (1991) in Bennett and Lasure (Eds.) MORE GENE MANIPULATIONS IN FUNGI, Academic Press pp. 396-428 and U.S. Patent No. 5,874,276. Reference is also made to the Fungal Genetics Stock Center Catalogue of Strains (FGSC, < www.fgsc.net>) for a list of vectors. Particularly useful vectors include vectors obtained from for example Invitrogen and Promega.

Suitable plasmids for use in bacterial cells include pBR322 and pUC19 permitting replication in *E.coli* and pE194 for example permitting replication in *Bacillus.* Other specific vectors suitable for use in *E. coli* host cells include vectors such as pFB6, pBR322, pUC18, pUC100, pDONR™201, pDONR™221, pENTR™, pGEM®3Z and pGEM®4Z.

Specific vectors suitable for use in fungal cells include pRAX, a general purpose expression vector useful in *Aspergillus,* pRAX with a *gla*A promoter, and in *Hypocrea/Trichoderma* includes pTrex3g with a *cbh*1 promoter.

In some embodiments, the promoter shows transcriptional activity in a bacterial or a fungal host cell and may be derived from genes encoding proteins either homologous or heterologous to the host cell. The promoter may be a mutant, a truncated and/or a hybrid promoter. The above-mentioned promoters are known in the art. Examples of suitable promoters useful in fungal cells and particularly filamentous fungal cells such as *Trichoderma* or *Aspergillus* cells include such exemplary promoters as the *T. reesei* promoters *cbh*1, *cbh*2, *egl*1, *egl*2, *eg*5, *xln*1 and *xln*2. Other examples of useful promoters include promoters from *A. awamori* and *A. niger* glucoamylase genes (glaA) *(See,* Nunberg et al., (1984) Mol. Cell Biol. 4:2306-2315 and Boel et al., (1984) EMBO J. 3:1581-1585), *A. oryzae* TAKA amylase promoter, the TPI (triose phosphate isomerase) promoter from *S. cerevisiae,* the promoter from *Aspergillus nidulans* acetamidase genes and *Rhizomucor miehei* lipase genes. Examples of suitable promoters useful in bacterial cells include those obtained from the *E. coli* lac operon; *Bacillus licheniformis* alpha amylase gene (*amy*L), *B. stearothermophilus* amylase gene (*amy*S); *Bacillus subtilis xyl*A and *xyl*B genes, the beta-lactamase gene, and the *tac* promoter. In some embodiments, the promoter is one that is native to the host cell. For example, when *T. reesei* is the host, the promoter is a native *T. reesei* promoter. In other embodiments, the promoter is one that is heterologous to the fungal host cell. In some embodiments, the promoter will be the promoter of a parent glucoamylase (e.g., the TrGA promoter).

In some embodiments, the DNA construct includes nucleic acids coding for a signal sequence, that is, an amino acid sequence linked to the amino terminus of the polypeptide which directs the encoded polypeptide into the cell's secretory pathway. The 5' end of the coding sequence of the nucleic acid sequence may naturally include a signal peptide coding region which is naturally linked in translation reading frame with the segment of the glucoamylase coding sequence which encodes the secreted glucoamylase or the 5' end of the coding sequence of the nucleic acid sequence may include a signal peptide which is foreign to the coding sequence. In some embodiments, the DNA construct includes a signal sequence that is naturally associated with a parent glucoamylase gene from which a variant glucoamylase has been obtained. In some embodiments the signal sequence will be the sequence depicted in SEQ ID NO:1 or a sequence having at least 90%, at least 94% and at least 98% sequence identity thereto. Effective signal sequences may include the signal sequences obtained from other filamentous fungal enzymes, such as from *Trichoderma* (*T. reesei* glucoamylase), *Humicola* (*H. insolens* cellulase *or H. grisea* glucoamylase), *Aspergillus* (*A. niger* glucoamylase and *A. oryzae* TAKA amylase), *and Rhizopus.*

In additional embodiments, a DNA construct or vector comprising a signal sequence and a promoter sequence to be introduced into a host cell are derived from the same source. In some embodiments, the native glucoamylase signal sequence of a *Trichoderma* glucoamylase homologue, such as a signal sequence from a *Hypocrea* strain may be used.

In some embodiments, the expression vector also includes a termination sequence. Any termination sequence functional in the host cell may be used in the present disclosure. In some embodiments, the termination sequence and the promoter sequence are derived from the same source. In another embodiment, the termination sequence is homologous to the host cell. Useful termination sequences include termination sequences obtained from the genes of *Trichoderma reesei cbh*1; *A. niger* or *A. awamori* glucoamylase (Nunberg *et al.* (1984) *supra,* and Boel *et al.,* (1984) *supra),* *Aspergillus nidulans* anthranilate synthase, *Aspergillus oryzae* TAKA amylase, or *A. nidulans trp*C (Punt et al., (1987) Gene 56:117 - 124).

In some embodiments, an expression vector includes a selectable marker. Examples of selectable markers include ones which confer antimicrobial resistance (e.g., hygromycin and phleomycin). Nutritional selective markers also find use in the present disclosure including those markers known in the art as *amd*S (acetamidase), *arg*B (ornithine carbamoyltransferase) and *pyr*G (orotidine-5'phosphate decarboxylase). Markers useful in vector systems for transformation of *Trichoderma* are known in the art (See, *e.g.,* Finkelstein, chapter 6 in BIOTECHNOLOGY OF FILAMENTOUS FUNGI, Finkelstein et al.

(1992) Eds. Butterworth-Heinemann, Boston, MA; Kinghorn et al. (1992) APPLIED MOLECULAR GENETICS OF FILAMENTOUS FUNGI, Blackie Academic and Professional, Chapman and Hall, London; Berges and Barreau (1991) Curr. Genet. 19:359 - 365; and van Hartingsveldt et al., (1987) Mol. Gen. Genet. 206:71 - 75). In some embodiments, the selective marker is the *amd*S gene, which encodes the enzyme acetamidase, allowing transformed cells to grow on acetamide as a nitrogen source. The use of *A. nidulans amd*S gene as a selective marker is described in Kelley et al., (1985) EMBO J. 4:475 - 479 and Penttilä et al., (1987) Gene 61:155-164.

Methods used to ligate the DNA construct comprising a nucleic acid sequence encoding a variant glucoamylase, a promoter, a termination and other sequences and to insert them into a suitable vector are well known in the art. Linking is generally accomplished by ligation at convenient restriction sites. If such sites do not exist, synthetic oligonucleotide linkers are used in accordance with conventional practice. (*See,* Sambrook (1989) *supra,* and Bennett and Lasure, MORE GENE MANIPULATIONS IN FUNGI, Academic Press, San Diego (1991) pp 70 - 76.). Additionally, vectors can be constructed using known recombination techniques (*e.g.,* Invitrogen Life Technologies, Gateway Technology).

**Host cells:**

The present invention also relates to host cells comprising a polynucleotide encoding a variant glucoamylase of the invention as defined in the claims. In some embodiments, the host cells of the present disclosure are chosen from bacterial, fungal, plant and yeast cells. The term host cell includes both the cells, progeny of the cells and protoplasts created from the cells which are used to produce a variant glucoamylase according to the present disclosure.

In some embodiments, the host cells of the present disclosure are fungal cells and preferably filamentous fungal host cells. The term "filamentous fungi" refers to all filamentous forms of the subdivision Eumycotina (*See,* Alexopoulos, C. J. (1962), INTRODUCTORY MYCOLOGY, Wiley, New York). These fungi are characterized by a vegetative mycelium with a cell wall composed of chitin, cellulose, and other complex polysaccharides. The filamentous fungi of the present disclosure are morphologically, physiologically, and genetically distinct from yeasts. Vegetative growth by filamentous fungi is by hyphal elongation and carbon catabolism is obligatory aerobic. In the present disclosure, the filamentous fungal parent cell may be a cell of a species of, but not limited to, *Trichoderma,* (*e.g., Trichoderma reesei,* the asexual morph of *Hypocrea jecorina,* previously classified as *T. longibrachiatum, Trichoderma viride, Trichoderma koningii, Trichoderma harzianum*) (Sheir-Neirs et al., (1984) Appl. Microbiol. Biotechnol 20:46 - 53; ATCC No. 56765 and ATCC No. 26921); *Penicillium sp., Humicola sp.* (*e.g., H. insolens, H. lanuginosa* and *H. grisea*); *Chrysosporium sp.* (*e.g., C. lucknowense*), *Gliocladium sp., Aspergillus sp.* (*e.g., A. oryzae, A. niger, A sojae, A. japonicus, A. nidulans,* and *A. awamori*) (Ward et al., (1993) Appl. Microbiol. Biotechnol. 39:738 - 743 and Goedegebuur et al., (2002) Genet 41:89 -98), *Fusarium sp.,* (e.g. *F. roseum, F. graminum F. cerealis, F. oxysporum* and *F. venenatum*), *Neurospora sp.,* (*N. crassa*), *Hypocrea sp., Mucor sp.,(M. miehei), Rhizopus sp.* and *Emericella sp.* (See also, Innis et al., (1985) Sci. 228:21 -26). The term *"Trichoderma"* or *"Trichoderma sp."* or *"Trichoderma spp."* refer to any fungal genus previously or currently classified as *Trichoderma.*

In some embodiments, the host cells will be gram-positive bacterial cells. Non-limiting examples include strains of *Streptomyces,* (e.g., *S. lividans , S. coelicolor* and S. *griseus)* and *Bacillus.* As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."*

In some embodiments, the host cell is a gram-negative bacterial strain, such as *E. coli* or *Pseudomonas sp.* In other embodiments, the host cells may be yeast cells such as *Saccharomyces sp., Schizosaccharomyces sp., Pichia sp.,* or *Candida sp.*

In other embodiments, the host cell will be a genetically engineered host cell wherein native genes have been inactivated, for example by deletion in bacterial or fungal cells. Where it is desired to obtain a fungal host cell having one or more inactivated genes known methods may be used (*e.g.* methods disclosed in U.S. Patent No. 5,246,853, U.S. Patent No. 5,475,101 and WO 92/06209). Gene inactivation may be accomplished by complete or partial deletion, by insertional inactivation or by any other means which renders a gene nonfunctional for its intended purpose (such that the gene is prevented from expression of a functional protein). In some embodiments, when the host cell is a *Trichoderma* cell and particularly a *T. reesei* host cell, the *cbh*1 , *cbh*2, *egl*1 and *egl*2 genes will be inactivated and/or deleted. Exemplary *Trichoderma reesei* host cells having quad-deleted proteins are set forth and described in USP 5,847,276 and WO 05/001036. In other embodiments, the host cell is a protease deficient or protease minus strain.

**Transformation of host cells:**

Introduction of a DNA construct or vector into a host cell includes techniques such as transformation; electroporation; nuclear microinjection; transduction; transfection, (*e.g.,* lipofection-mediated and DEAE-Dextrin mediated transfection); incubation with calcium phosphate DNA precipitate; high velocity bombardment with DNA-coated microprojectiles; and protoplast fusion. General transformation techniques are known in the art (See, *e.g.,* Ausubel et al., (1987), *supra,* chapter 9; and Sambrook (1989) *supra,* and Campbell et al., (1989) Curr. Genet. 16:53-56).

Transformation methods for *Bacillus* are disclosed in numerous references including Anagnostopoulos C and J. Spizizen (1961) J. Bacteriol. 81:741 - 746 and WO 02/14490.

Transformation methods for *Aspergillus* are described in Yelton et al (1984) Proc. Natl. Acad. Sci. USA 81:1470 - 1474; Berka et al., (1991) in Applications of Enzyme Biotechnology, Eds. Kelly and Baldwin, Plenum Press (NY); Cao et al., (2000) Sci. 9:991 - 1001; Campbell et al., (1989) Curr. Genet. 16:53-56 and EP 238 023. The expression of heterologous protein in *Trichoderma* is described in USP 6,022,725; USP 6,268,328; Harkki et al. (1991); Enzyme Microb. Technol. 13:227-233; Harkki et al., (1989) Bio Technol. 7:596-603; EP 244,234; EP 215,594; and Nevalainen et al., "The Molecular Biology of Trichoderma and its Application to the Expression of Both Homologous and Heterologous Genes", in MOLECULAR INDUSTRIAL MYCOLOGY, Eds. Leong and Berka, Marcel Dekker Inc., NY (1992) pp. 129 - 148). Reference is also made to WO96/00787 and Bajar et al., (1991) Proc. Natl. Acad. Sci. USA 88:8202 - 28212 for transformation of *Fusarium* strains.

In one specific embodiment, the preparation of *Trichoderma sp.* for transformation involves the preparation of protoplasts from fungal mycelia *(See,* Campbell et al., (1989) Curr. Genet. 16:53-56; Pentilla et al., (1987) Gene 61:155 - 164). *Agrobacterium tumefaciens*-mediated transformation of filamentous fungi is known *(See,* de Groot et al., (1998) Nat. Biotechnol. 16:839 - 842). Reference is also made to USP 6,022,725 and USP 6,268,328 for transformation procedures used with filamentous fungal hosts.

In some embodiments, genetically stable transformants are constructed with vector systems whereby the nucleic acid encoding the variant glucoamylase is stably integrated into a host strain chromosome. Transformants are then purified by known techniques.

In some further embodiments, the host cells are plant cells, such as cells from a monocot plant (e.g. corn, wheat and sorghum) or cells from a dicot plant (*e.g.* soybean). Methods for making DNA constructs useful in transformation of plants and methods for plant transformation are known. Some of these methods include *Agrobacterium tumefaciens* mediated gene transfer; microprojectile bombardment, PEG mediated transformation of protoplasts, electroporation and the like. Reference is made to USP 6,803,499, USP 6,777,589; Fromm et al (1990) Biotechnol. 8:833-839; Potrykus et al (1985) Mol. Gen. Genet. 199:169 - 177.

## Production of Proteins:

The present invention further relates to methods of producing the variant glucoamylases as defined in the claims, which comprises transforming a host cell with an expression vector comprising a polynucleotide encoding a variant glucoamylase according to the invention, culturing the host cell under conditions suitable for expression and production of the variant glucoamylase and optionally recovering the variant glucoamylase.

In the expression and production methods of the present invention the host cells are cultured under suitable conditions in shake flask cultivation, small scale or large scale fermentations (including continuous, batch and fed batch fermentations) in laboratory or industrial fermentors, with suitable medium containing physiological salts and nutrients (See, *e.g.,* Pourquie, J. et al., BIOCHEMISTRY AND GENETICS OF CELLULOSE DEGRADATION, eds. Aubert, J. P. et al., Academic Press, pp. 71-86, 1988 and Ilmen, M. et al., (1997) Appl. Environ. Microbiol. 63:1298-1306). Common commercially prepared media (*e.g.,* Yeast Malt Extract (YM) broth, Luria Bertani (LB) broth and Sabouraud Dextrose (SD) broth) find use in the present invention. Culture conditions for bacterial and filamentous fungal cells are known in the art and may be found in the scientific literature and/or from the source of the fungi such as the American Type Culture Collection and Fungal Genetics Stock Center. In cases where a glucoamylase coding sequence is under the control of an inducible promoter, the inducing agent (*e.g.,* a sugar, metal salt or antimicrobial), is added to the medium at a concentration effective to induce glucoamylase expression.

In some embodiments, the present disclosure relates to methods of producing the variant glucoamylase in a plant host comprising transforming a plant cell with a vector comprising a polynucleotide encoding a glucoamylase variant according to the invention and growing the plant cell under conditions suitable for the expression and production of the variant.

In some embodiments, assays are carried out to evaluate the expression of a variant glucoamylase by a cell line that has been transformed with a polynucleotide encoding a variant glucoamylase encompassed by the invention. The assays can be carried out at the protein level, the RNA level and/or by use of functional bioassays particular to glucoamylase activity and/or production. Some of these assays include Northern blotting, dot blotting (DNA or RNA analysis), RT-PCR (reverse transcriptase polymerase chain reaction), *in situ* hybridization using an appropriately labeled probe (based on the nucleic acid coding sequence) and conventional Southern blotting and autoradiography.

In addition, the production and/or expression of a variant glucoamylase may be measured in a sample directly, for example, by assays directly measuring reducing sugars such as glucose in the culture medium and by assays for measuring glucoamylase activity, expression and/or production. In particular, glucoamylase activity may be assayed by the 3,5-dinitrosalicylic acid (DNS) method *(See,* Goto *et al.,*

(1994) Biosci. Biotechnol. Biochem. 58:49 - 54). In additional embodiments, protein expression, is evaluated by immunological methods, such as immunohistochemical staining of cells, tissue sections or immunoassay of tissue culture medium, (*e.g.,* by Western blot or ELISA). Such immunoassays can be used to qualitatively and quantitatively evaluate expression of a glucoamylase. The details of such methods are known to those of skill in the art and many reagents for practicing such methods are commercially available.

The glucoamylases of the present invention may be recovered or purified from culture media by a variety of procedures known in the art including centrifugation, filtration, extraction, precipitation and the like.

## Compositions:

The variant glucoamylases of the invention may be used in enzyme compositions including but not limited to starch hydrolyzing and saccharifying compositions, cleaning and detergent compositions (e.g., laundry detergents, dish washing detergents, and hard surface cleaning compositions), alcohol fermentation compositions, and in animal feed compositions. Further the variant glucoamylases may be used in baking applications, such as bread and cake production, brewing, healthcare, textile, environmental waste conversion processes, biopulp processing, and biomass conversion applications.

In some embodiments, an enzyme composition of the present disclosure comprising a variant glucoamylase encompassed by the invention will be optionally used in combination with any one or combination of the following enzymes - alpha amylases, proteases, pullulanases, isoamylases, cellulases, hemicellulases, xylanases, cyclodextrin glycotransferases, lipases, phytases, laccases, oxidases, esterases, cutinases, xylanases, granular starch hydrolyzing enzymes and other glucoamylases.

In some embodiments, the enzyme composition will include an alpha amylase such as fungal alpha amylases (*e.g. Aspergillus* sp.) or bacterial alpha amylases (*e.g. Bacillus* sp. such as *B. stearothermophilus, B. amyloliquefaciens* and *B. licheniformis*) and variants and hybrids thereof. In some embodiments, the alpha amylase is an acid stable alpha amylase. In some embodiments, the alpha amylase is *Aspergillus kawachi* alpha amylase (AkAA), see US 7,037,704. Commercially available alpha amylases contemplated for use in the compositions of the invention are known and include GZYME G997, SPEZYME FRED, SPEZYME XTRA, (Danisco US, Inc, Genencor Division), TERMAMYL 120-L and SUPRA (Novozymes, Biotech.).

In some embodiments, the enzyme composition will include an acid fungal protease. In a further embodiment, the acid fungal protease is derived from a *Trichoderma sp* and may be any one of the proteases disclosed in US 2006/015342, published 7/13/2006(e.g. SEQ ID NO:10). In a further embodiment, the enzyme composition will include a phytase (e.g., a *Buttiauxiella* spp. or variant thereof (e.g., see PCT patent publication WO 2006/043178).

In other embodiments, the variant glucoamylases of the invention may be combined with other glucoamylases. In some embodiments, the glucoamylases of the invention will be combined with one or more glucoamylases derived from strains of *Aspergillus* or variants thereof, such as *A. oryzae, A. niger, A. kawachi,* and *A. awamori;* glucoamylases derived from strains of *Humicola* or variants thereof, particularly *H. grisea,* such as the glucoamylase having at least 90%, 93%, 95%, 96%, 97%, 98% and 99% sequence identity to SEQ ID NO: 3 disclosed in WO 05/052148; glucoamylases derived from strains of *Talaromyces* or variants thereof, particularly *T. emersonii;* glucoamylases derived from strains of *Athelia* and particularly *A. rolfsii;* glucoamylases derived from strains of *Penicillium,* particularly *P. chrysogenum.*

## Uses:

In particular, the variant glucoamylases disclosed herein may be used for starch conversion processes, and particularly in the production of dextrose for fructose syrups, specialty sugars and in alcohol and other end-product (e.g. organic acid, ascorbic acid, and amino acids) production from fermentation of starch containing substrates (G.M.A van Beynum et al., Eds. (1985) STARCH CONVERSION TECHNOLOGY, Marcel Dekker Inc. NY). Dextrins produced using variant glucoamylase compositions of the present disclosure may result in glucose yields of at least 80%, at least 85%, at least 90% and at least 95%. Production of alcohol from the fermentation of starch substrates using glucoamylases encompassed by the invention may include the production of fuel alcohol or potable alcohol. In some embodiments, the production of alcohol will be greater when the variant glucoamylase is used under the same conditions as the parent glucoamylase. In some embodiments, the production of alcohol will be between about 0.5% and 2.5% better, including but not limited to 0.6%, 0.7%, 0.8%, 0.9%, 1.0%, 1.1%, 1.2%, 1.3%, 1.4%, 1.5%, 1.6%. 1.7%, 1.8%, 1.9%, 2.0%, 2.1%, 2.2%, 2.3%, and 2.4% more alcohol than the parent glucoamylase.

In some embodiments, the variant glucoamylases of the invention will find use in the hydrolysis of starch from various plant-based substrates, which are used for alcohol production. In some embodiments, the plant-based substrates will include corn, wheat, barley, rye, milo, rice, sugar cane, potatoes and combinations thereof. In some embodiments, the plant-based substrate will be fractionated plant material, for example a cereal grain such as corn, which is fractionated into components such as fiber, germ, protein and starch (endosperm) (USP 6,254,914 and USP 6,899,910). Methods of alcohol fermentations are described in THE ALCOHOL TEXTBOOK, A REFERENCE FOR THE BEVERAGE, FUEL AND INDUSTRIAL ALCOHOL INDUSTRIES, 3rd Ed., Eds K.A. Jacques et al., 1999, Nottingham University Press, UK. In certain embodiments, the alcohol will be ethanol. In particular, alcohol fermentation production processes are characterized as wet milling or dry milling processes. In some embodiments, the variant glucoamylase will be used in a wet milling fermentation process and in other embodiments the variant glucoamylase will find use in a dry milling process.

Dry grain milling involves a number of basic steps, which generally include: grinding, cooking, liquefaction, saccharification, fermentation and separation of liquid and solids to produce alcohol and other co-products. Plant material and particularly whole cereal grains, such as corn, wheat or rye are ground. In some cases the grain may be first fractionated into component parts. The ground plant material may be milled to obtain a coarse or fine particle. The ground plant material is mixed with liquid (e.g. water and/or thin stillage) in a slurry tank. The slurry is subjected to high temperatures (e.g. 90°C to 105°C or higher) in a jet cooker along with liquefying enzymes (*e.g.* alpha amylases) to solublize and hydrolyze the starch in the grain to dextrins. The mixture is cooled down and further treated with saccharifying enzymes, such as glucoamylases encompassed by the instant invention, to produce glucose. The mash containing glucose may then be fermented for approximately 24 to 120 hours in the presence of fermentation microorganisms, such as ethanol producing microorganism and particularly yeast (*Saccharomyces* spp). The solids in the mash are separated from the liquid phase and alcohol such as ethanol and useful co-products such as distillers' grains are obtained.

In some embodiments, the saccharification step and fermentation step are combined and the process is referred to as simultaneous saccharification and fermentation or simultaneous saccharification, yeast propagation and fermentation.

In other embodiments, the variant glucoamylase is used in a process for starch hydrolysis wherein the temperature of the process is between 30°C and 75°C, in some embodiments, between 40°C and 65°C. In some embodiments, the variant glucoamylase is used in a process for starch hydrolysis at a pH of between pH 3.0 and pH 6.5. The fermentation processes in some embodiments include milling of a cereal grain or fractionated grain and combining the ground cereal grain with liquid to form a slurry which is then mixed in a single vessel with a variant glucoamylase according to the invention and optionally other enzymes such as, but not limited to, alpha amylases, other glucoamylases, phytases, proteases, pullulanases, isoamylases or other enzymes having granular starch hydrolyzing activity and yeast to produce ethanol and other co-products (See e.g., USP 4,514,496, WO 04/081193 and WO 04/080923).

In some embodiments, the present disclosure pertains to a method of saccharifying a liquid starch solution, which comprises an enzymatic saccharification step using a variant glucoamylase of the invention.

The present disclosure also provides an animal feed composition or formulation comprising at least one variant glucoamylase encompassed by the invention. Methods of using a glucoamylase enzyme in the production of feeds comprising starch are provided in WO 03/049550. Briefly, the glucoamylase variant is admixed with a feed comprising starch. The glucoamylase is capable of degrading resistant starch for use by the animal.

Other objects and advantages of the present invention are apparent from the present Specification.


## EXPERIMENTAL

In the disclosure and experimental section which follows, the following abbreviations apply: GA (glucoamylase); GAU (glucoamylase unit); wt % (weight percent); °C (degrees Centigrade); rpm (revolutions per minute); $H_2O$ (water); $dH_2O$ (deionized water); $dIH_2O$ (deionized water, Milli-Q filtration); aa or AA (amino acid); bp (base pair); kb (kilobase pair); kD (kilodaltons); g or gm (grams); $\mu$g (micrograms); mg (milligrams); $\mu$L (microliters); ml and mL (milliliters); mm (millimeters); $\mu$m (micrometer); M (molar); mM (millimolar); $\mu$M (micromolar); U (units); V (volts); MW (molecular weight); sec(s) or s(s) (second/seconds); min(s) or m(s) (minute/minutes); hr(s) or h(s) (hour/hours); DO (dissolved oxygen); ABS (Absorbance); EtOH (ethanol); PSS (physiological salt solution); m/v (mass/volume); and MTP (microtiter plate); N (Normal); DP1 (monosaccharides); DP2 (disaccharides); DP>3 (oligosaccharides, sugars having a degree of polymerization greater than 3); ppm (parts per million).

Assays and methods used in the examples below are provided. However, it should be noted that different methods may be used to provide variants of a parent molecule and the invention is not limited to the methods used in the examples. It is intended that any suitable means for making variants and selection of variants may be used.


## pNPG glucoamylase activity assay for 96-well microtiter plates:

The reagent solutions were: NaAc buffer (200 mM sodium acetate buffer pH 4.5); Substrate (50 mM p-nitro-phenyl-$\alpha$-D-glucopyranoside (Sigma N-1377) in NaAc buffer (0.3 g/20ml)) and stop solution (800 mM glycine-NaOH buffer pH 10). 30 $\mu$l filtered supernatant was placed in a fresh 96-well flat bottom MTP. To each well 50 $\mu$l NaAc buffer and 120 $\mu$l substrate was added and incubated for 30 minutes at 50°C (Thermolab systems iEMS Incubator/shaker HT). The reaction was terminated by adding 100 $\mu$l stop solution. The absorbance was measured at 405 nm in a MTP-reader (Molecular Devices Spectramax 384 plus) and the activity was calculated using a molar extinction coefficient of 0.011 $\mu$M/cm.

**Thermal stability assay:**

Crude supernatant (8 μl) was added to 280 μl 50 mM NaAc buffer pH 4.5. The diluted sample was equally divided over 2 MTPs. One MTP (initial plate) was incubated for 1 hr at 4°C and the other MTP (residual plate) was incubated at 64°C (Thermolab systems iEMS Incubator/Shaker HT) for 1 hr. The residual plate was chilled for 10 min on ice. Activity was measured of both plates using the ethanol screening assay described below. 60μl of the initial plate or residual plate was added to 120μl 4% soluble corn starch and incubated for 2 hrs at 32°C 900 rpm (Thermolabsystems iEMS Incubator/Shaker HT).

Thermal stability was calculated as % residual activity as follows:

$$\frac{ABS\ (340)\ residual - blank}{ABS\ (340)\ initial - blank}\ \ X\ 100\%.$$

The crude supernatant material was tested for remaining glucose in the culture medium after the growth period. If remaining glucose was found, the thermal stability was not calculated.

Based on the residual activity of WT and mutant the performance index (PI) for the thermal stability was calculated. The PI of a variant is the quotient "Variant-residual activity/WT-residual activity." The PI of WT is 1.0 and a variant with a PI > 1.0 has a specific activity that is greater than WT.

**Caliper protein determination**

Data analysis and calculation of performance index of ethanol application assay.

Protein levels were measured using a microfluidic electrophoresis instrument (Caliper Life Sciences, Hopkinton, MA, USA). The microfluidic chip and protein samples were prepared according to the manufacturer's instructions (LABCHIP® HT Protein Express, P/N 760301). Culture supernatants were prepared and stored in 96-well microtiter plates at -20°C until use, when they were thawed by warming in a 37°C incubator for 30 minutes. After shaking briefly, 2 μl of each culture sample was transferred to a 96-well PCR plate (Bio-Rad, Hercules, CA, USA) containing 7 μl samples buffer (Caliper) followed by heating the plate to 90°C for 5 minutes on a thermostatically controlled plate heater. The plate was allowed to cool before adding 40 μl water to each sample. The plate was then placed in the instrument along with a protein standard supplied and calibrated by the manufacturer. As the proteins move past a focal point in the chip, the fluorescence signal was recorded and the protein concentration was determined by quantitating the signal relative to the signal generated by the calibrated set of protein standards.

After the Caliper protein determination the data was processed in the following way. The calibration ladders are checked for correctness of the peak pattern. If the calibration ladder which was associated with the run did not suffice, it was replaced by a calibration ladder of an adjacent run. For peak detection, the default settings of the global peak find option of the caliper software were used. The peak of interest was selected at 75 kDA +/-10%.

The result was exported to a spreadsheet program and the peak area was related to the corresponding activity (ABS340-blank measurement) in the ethanol application assay.

With the area and activity numbers of 12 Wild Type samples, a calibration line was made using the "Enzyme Kinetics" equation of the program Grafit Version 5 (Erithacus Software, Horley, UK) in combination with a non-linear fit function. The default settings were used to calculate the Km and Vmax parameters. Based on these two parameters, a Michaelis-Menten reference line was made and the specific activity of each variant was calculated based on this reference line.

Based on the specific activity of WT and variants the performance index (PI) for the specific activity was calculated. The PI of a variant is the quotient "Variant-specific activity/WT-specific activity." The PI of WT is 1.0 and a variant with a PI > 1.0 had a specific activity that is greater than WT.

**Hexokinase activity assay:**

Hexokinase cocktail: 10 - 15 minutes prior to use, 90 ml water was added to a BoatIL container glucose HK R1 (IL test glucose (HK) kit, Instrument Laboratory # 182507-40) and gently mixed. 100 μl of Hexokinase cocktail was added to 85 μl of dH$_2$O. 15μl of sample was added to the mixtures and incubated for 10 minutes in the dark at room temperature. Absorbance was read at 340 nm in a MTP-reader. Glucose concentrations were calculated according to a glucose (0 - 2 mg/ml) standard curve.

**Assay conditions ethanol screening-assay:**

8% stock solution: 8 g of soluble corn starch (Sigma #S4180) was suspended in 40 ml dH$_2$O at room temperature. 50 ml of boiling dH$_2$O was added to the slurry in a 250 ml flask and cooked for 5 minutes. The starch solution was cooled to 25°C and the volume adjusted to 100 ml with dH$_2$O.

Stop solution: 800 mM Glycine-NaOH buffer, pH 10.

4% (m/v) soluble starch working solution: stock solution was diluted (1:1) with 100 mM sodium acetate buffer pH 3.7.

5 $\mu$l crude supernatant was diluted with 175 $\mu$l 50mM NaAc buffer pH 4.5 in a flat bottom 96-well MTP. 60 $\mu$l of this dilution was added to 120 $\mu$l 4% soluble corn starch and incubated for 2 hrs at 32°C 900 rpm (Thermolabsystems iEMS Incubator/Shaker HT). The reaction was stopped by adding 90 $\mu$l 4°C-cold Stop Solution. The sample was placed on ice. Starch was spun down at 1118xg at 15°C for 5 minutes (SIGMA 6K15) and 15 $\mu$l supernatant was used in the Hexokinase activity assay described above to determine the glucose content.

The crude supernatant material was tested for remaining glucose in the culture medium after the growth period. If remaining glucose was found, the amount of glucose produced by the Glucoamylase was not calculated.

## EXAMPLES

The following examples are provided in order to demonstrate and further illustrate certain embodiments and aspects of the present invention and are not to be construed as limiting the scope thereof.

### Example 1: Construction of TrGA site evaluation libraries (SELs) in the pTTT vector for expression in *Trichoderma reesei*

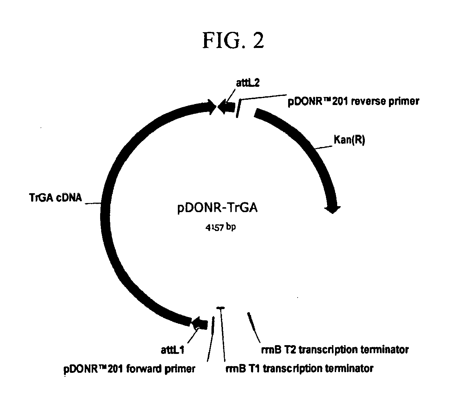

A *Trichoderma reesei* cDNA sequence (SEQ ID NO: 4) was cloned into pDONR™201 via the Gateway® BP recombination reaction (Invitrogen, Carlsbad, CA, USA) resulting in the entry vector pDONR-TrGA (Fig. 2). The cDNA sequence (SEQ ID NO:4) encodes the TrGA signal peptide, the prosequence, and the mature protein, including the catalytic domain, linker region and starch binding domain (SEQ ID NO:1). SEQ ID NO:4 and SEQ ID NO:1 are shown in Figure 1B and 1A. Figure 1C illustrates the precursor and mature protein TrGA domains.

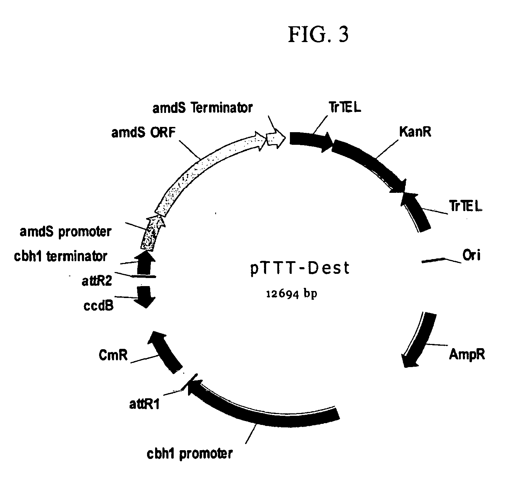

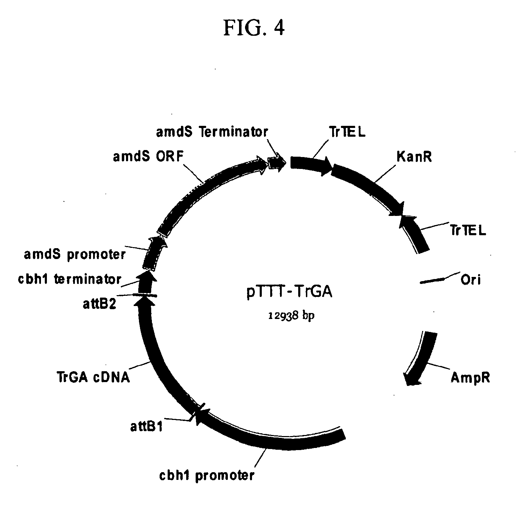

To express the TrGA protein in *Trichoderma reesei*, the TrGA coding sequence (SEQ ID NO:4) was cloned into the Gateway compatible destination vector pTTT-Dest (Fig. 3) via the GATEWAY® LR recombination reaction. The expression vector contained the *T. reesei* cbhI-derived promoter and terminator regions which allowed for strong inducible expression of a gene of interest. The vector also contained the *Aspergillus nidulans* amdS selective marker which allowed for growth of the transformants on acetamide as a sole nitrogen source. The expression vector also contained *T. reesei* telomere regions which allowed for non-chromosomal plasmid maintenance in a fungal cell. On the destination pTTT-Dest plasmid, the cbhI promoter and terminator regions were separated by the chloramphenicol resistance gene, Cm$^R$, and the lethal *E. coli* gene, *ccd*B, flanked by the bacteriophage lambda-based specific recombination sites attR1, attR2. This configuration allowed for direct selection of recombinants containing the TrGA gene under control of the cbhI regulatory elements in the right orientation via the GATEWAY® LR recombination reaction. The final expression vector pTTT-TrGA is shown in Figure 4.

SELs were constructed using the pDONR-TrGA entry vector (Fig. 2) as a template and the primers listed in Table 2. All primers used in the mutagenesis experiments contained the triplet NNS (N= A,C,T,G and S=C or G) at the position that aligns with the codon of the TrGA sequence designed to be mutated (SEQ ID NO:1), allowing for a random incorporation of nucleotides at the preselected position. Construction of each SEL library started with two independent PCR amplifications on the pDONR-TrGA entry vector: one using the Gateway F (pDONR201 - FW) and a specific mutagenesis primer R (Table 2), and the other - the Gateway primer R (pDONR201 - RV) and a specific mutagenesis primer F (Table 2). High fidelity PHUSION DNA polymerase (Finnzymes OY, Espoo, Finland) was used in a PCR amplification reaction including 0.2$\mu$M primers. The reactions were carried out for 25 cycles according to the protocol provided by Finnzymes. 1 $\mu$l aliquots of the PCR fragments obtained were used as templates for a subsequent fusion PCR reaction together with the Gateway FW and Gateway RV primers (Invitrogen). This PCR amplification, after 22 cycles, produced a population of the full-length linear TrGA DNA fragments randomly mutated at the specific codon position. The fragments were flanked by the Gateway-specific attL1, attL2 recombination sites on both ends. The DNA fragments were purified with a CHARGESWITCH® PCR clean-up kit (Invitrogen, Carlsbad USA) and then recombined with 100 ng of the pTTT- destination vector (Fig. 3) using the LR CLONASE™ II enzyme mix according to the protocol supplied by Invitrogen. The recombination products that were generated were transformed into *E.coli* Max Efficiency DH5$\alpha$, as described by the supplier (Invitrogen). The final expression constructs pTTT-TrGA with mutations at the desired position were selected by plating bacteria on 2xYT agar plates (16 g/L Bacto Tryptone (Difco), 10 g/L Bacto Yeast Extract (Difco), 5 g/L NaCl, 16 g/L Bacto Agar (Difco)) with 100$\mu$g/ml ampicillin.

96 single colonies from each library were grown for 24 hrs at 37°C in MTP containing 200 μL 2xYT medium with 100μg/ml ampicillin. Cultures were used directly to amplify PCR fragments encompassing the region where a specific mutation was introduced. The specific PCR products obtained were sequenced using an ABI3100 sequence analyzer (Applied Biosystems). Each library contained from 15 to 19 different TrGA variants in the final expression vector. These variants were individually transformed into *T. reesei*, as described below. Libraries are numbered from 1 to 182 referencing the specific amino acid residue in the TrGA sequence which was randomly mutated.

## Table 2 - Primers used to generate TrGA SELs

| F = Forward; R = Reverse | | |
|---|---|---|
| AA-position | F/R | DNA Sequence 5' to 3' |
| pDONR2 01- | F | TCGCGTTAACGCTAGCATGGATCTC (SEQ ID NO:13) |
| pDONR2 01- | R | TCGCGTTAACGCTAGCATGGATCTC (SEQ ID NO:14) |
| 4 | F | CGTCACCAAGAGGTCTGTTGACNNSTTCATCAGCACCGAGACGCC (SEQ ID NO:15) |
| 4 | R | GTCAACAGACCTCTTGGTGACGTCG (SEQ ID NO:16) |
| 5 | F | CACCAAGAGGTCTGTTGACGACNNSATCAGCACCGAGACGCCTATTGC (SEQ ID NO:17) |
| 5 | R | GTCGTCAACAGACCTCTTGGTGAC (SEQ ID NO:18) |
| 10 | F | TGACGACTTCATCAGCACCGAGNNSCCTATTGCACTG (SEQ ID NO:19) |
| 10 | R | CTCGGTGCTGATGAAGTCGTC (SEQ ID NO:20) |
| 12 | F | TCATCAGCACCGAGACGCCTNNSGCACTGAACAATCTTCTTTGCA (SEQ ID NO:21) |
| 12 | R | AGGCGTCTCGGTGCTGATGAAGTCG (SEQ ID NO:22) |
| 14 | F | CAGCACCGAGACGCCTATTGCANNSAACAATCTTCTT (SEQ ID NO:23) |
| 14 | R | TGCAATAGGCGTCTCGGTGCT (SEQ ID NO:24) |
| 15 | F | CACCGAGACGCCTATTGCACTGNNSAATCTTCTTTGC (SEQ ID NO:25) |
| 1S | R | CAGTGCAATAGGCGTCTCGGT (SEQ ID NO:26) |
| 23 | F | CAATCTTCTTTGCAATGTTGGTNNSATGGATGCCGT (SEQ ID NO:27) |
| 23 | R | ACCAACATTGCAAAGAAGATTG (SEQ ID NO:28) |
| 24 | F | TTCTTTGCAATGTTGGTCCTNNSGGATGCCGTGCATTCGGCACAT (SEQ ID NO:29) |
| 24 | R | AGGACCAACATTGCAAAGAAGATTG (SEQ ID NO:30) |
| 29 | F | GTCCTGATGGATGCCGTGCANNSGGCACATCAGCTGGTGCGGTGA (SEQ ID NO:31) |
| 29 | R | TGCACGGCATCCATCAGGACCAACA (SEQ ID NO:32) |
| 42 | F | TGCGGTGATTGCATCTCCCAGCNNSATTGACCCGGAC (SEQ ID NO:33) |
| 42 | R | GCTGGGAGATGCAATCACCGCA (SEQ ID NO:34) |
| 43 | F | TGATTGCATCTCCCAGCACANNSGACCCGGACTACTATTACATGT (SEQ ID NO:35) |
| 43 | R | TGTGCTGGGAGATGCAATCACCGCA (SEQ ID NO:36) |
| 44 | F | TTGCATCTCCCAGCACAATTNNSCCGGACTACTATTACATGTGGA (SEQ ID NO:37) |
| 44 | R | AATTGTGCTGGGAGATGCAATCACC (SEQ ID NO:38) |
| 45 | F | ATCTCCCAGCACAATTGACNNSGACTACTATTACATGTGGACGC (SEQ ID NO:39) |
| 45 | R | GTCAATTGTGCTGGGAGATGCAATC (SEQ ID NO:40) |
| 46 | F | CTCCCAGCACAATTGACCCGNNSTACTATTACATGTGGACGCGAGA (SEQ ID NO:41) |
| 46 | R | CGGGTCAATTGTGCTGGGAGATGCA (SEQ ID NO:42) |
| 47 | F | CCAGCACAATTGACCCGGACNNSTATTACATGTGGACGCGAGATA (SEQ ID NO:43) |
| 47 | R | GTCCGGGTCAATTGTGCTGGGAGAT (SEQ ID NO:44) |

(continued)

| F = Forward; R = Reverse | | |
|---|---|---|
| AA-position | F/R | DNA Sequence 5' to 3' |
| 49 | F | CAATTGACCCGGACTACTATNNSATGTGGACGCGAGATAGCGCTC (SEQ ID NO:45) |
| 49 | R | ATAGTAGTCCGGGTCAATTGTGCTG (SEQ ID NO:46) |
| 51 | F | ACCCGGACTACTATTACATGNNSACGCGAGATAGCGCTCTTGTCT (SEQ ID NO:47) |
| 51 | R | CATGTAATAGTAGTCCGGGTCAATT (SEQ ID NO:48) |
| 59 | F | GACGCGAGATAGCGCTCTTGTCNNSAAGAACCTCATC (SEQ ID NO:49) |
| 59 | R | GACAAGAGCGCTATCTCGCGT (SEQ ID NO:50) |
| 60 | F | GCGAGATAGCGCTCTTGTCTTCNNSAACCTCATCGAC (SEQ ID NO:51) |
| 60 | R | GAAGACAAGAGCGCTATCTCG (SEQ ID NO:52) |
| 61 | F | AGATAGCGCTCTTGTCTTCAAGNNSCTCATCGACCGC (SEQ ID NO:53) |
| 61 | R | CTTGAAGACAAGAGCGCTATC (SEQ ID NO:54) |
| 65 | F | TGTCTTCAAGAACCTCATCGACNNSTTCACCGAAACG (SEQ ID NO:55) |
| 65 | R | GTCGATGAGGTTCTTGAAGAC (SEQ ID NO:56) |
| 67 | F | CAAGAACCTCATCGACCGCTTCNNSGAAACGTACGAT (SEQ ID NO:57) |
| 67 | R | GAAGCGGTCGATGAGGITCTT (SEQ ID NO:58) |
| 68 | F | GAACCTCATCGACCGCTTCACCNNSACGTACGATGCG (SEQ ID NO:59) |
| 68 | R | GGTGAAGCGGTCGATGAGGTT (SEQ ID NO:60) |
| 70 | F | TCGACCGCTTCACCGAAACGNNSGATGCGGGCCTGCAGCGCCGCA (SEQ ID NO:61) |
| 70 | R | CGTTTCGGTGAAGCGGTCGATGAGG (SEQ ID NO:62) |
| 72 | F | CGCTTCACCGAAACGTACGATNNSGGCCTGCAGCGC (SEQ ID NO:63) |
| 72 | R | ATCGTACGTTTCGGTGAAGCGG (SEQ ID NO:64) |
| 73 | F | CTTCACCGAAACGTACGATGCGNNSCTGCAGCGCCGC (SEQ ID NO:65) |
| 73 | R | CGCATCGTACGTTTCGGTGAA (SEQ ID NO:66) |
| 75 | F | AAACGTACGATGCGGGCCTGNNSCGCCGCATCGAGCAGTACATTA (SEQ ID NO:67) |
| 75 | R | CAGGCCCGCATCGTACGTTTCGGTG (SEQ ID NO:68) |
| 76 | F | CGTACGATGCGGGCCTGCAGNNSCGCATCGAGCAGTACATTACTG (SEQ ID NO:69) |
| 76 | R | CTGCAGGCCCGCATCGTACGTTTCG (SEQ ID NO:70) |
| 94 | F | CTCTCCAGGGCCTCTCTAACNNSTCGGGCTCCCTCGCGGACGGCT (SEQ ID NO:71) |
| 94 | R | GTTAGAGAGGCCCTGGAGAGTGACC (SEQ ID NO:72) |
| 97 | F | GGGCCTCTCTAACCCCTCGGGCNNSTCGCGGACGGC (SEQ ID NO:73) |
| 97 | R | GCCCGAGGGGTTAGAGAGGCC (SEQ ID NO:74) |
| 98 | F | CCTCTCTAACCCCTCGGGCTCCNNSGCGGACGGCTCT (SEQ ID NO:75) |
| 98 | R | GGAGCCCGAGGGGTTAGAGAG (SEQ ID NO:76) |
| 99 | F | CTCTAACCCCTCGGGCTCCCTCNNSGACGGCTCTGGT (SEQ ID NO:77) |
| 99 | R | GAGGGAGCCCGAGGGGTTAGA (SEQ ID NO:78) |
| 100 | F | ACCCCTCGGGCTCCCTCGCGNNSGGCTCTGGTCTCGGCGAGCCCA (SEQ ID NO:79) |
| 100 | R | CGCGAGGGAGCCCGAGGGGTTAGAG (SEQ ID NO:80) |
| 102 | F | CTCGGGCTCCCTCGCGGACGGCNNSGGTCTCGGCGAG (SEQ ID NO:81) |

(continued)

| AA-position | F/R | DNA Sequence 5' to 3' |
|---|---|---|
| | | F = Forward; R = Reverse |
| 102 | R | GCCGTCCGCGAGGGAGCCCGA (SEQ ID NO:82) |
| 110 | F | TGGTCTCGGCGAGCCCAAGTTTNNSTTGACCCTGAAG (SEQ ID NO:83) |
| 110 | R | AAACTTGGGCTCGCCGAGACCA (SEQ ID NO:84) |
| 111 | F | TCTCGGCGAGCCCAAGTTTGAGNNSACCCTGAAGCCT (SEQ ID NO:85) |
| 111 | R | CTCAAACTTGGGCTCGCCGAG (SEQ ID NO:86) |
| 113 | F | CGAGCCCAAGTTTGAGTTGACCNNSAAGCCTTTCACC (SEQ ID NO:87) |
| 113 | R | GGTCAACTCAAACTTGGGCTC (SEQ ID NO:88) |
| 114 | F | CCAAGTTTGAGTTGACCCTGNNSCCTTTCACCGGCAACTGGGGTC (SEQ ID NO:89) |
| 114 | R | CAGGGTCAACTCAAACTTGGGCTCG (SEQ ID NO:90) |
| 116 | F | TTGAGTTGACCCTGAAGCCTNNSACCGGCAACTGGGGTCGACCGCA (SEQ ID NO:91) |
| 116 | R | AGGCTTCAGGGTCAACTCAAACTTG (SEQ ID NO:92) |
| 119 | F | CCTGAAGCCTTTCACCGGCNNSTGGGGTCGACCGCAGCGGGATG (SEQ ID NO:93) |
| 119 | R | GCCGGTGAAAGGCTTCAGGGTCAAC (SEQ ID NO:94) |
| 122 | F | CTTTCACCGGCAACTGGGGTNNSCCGCAGCGGGATGGCCCAGCTC(SEQID NO:95) |
| 122 | R | ACCCCAGTTGCCGGTGAAAGGCTTC (SEQ ID NO:96) |
| 125 | F | GCAACTGGGGTCGACCGCAGNNSGATGGCCCAGCTCTGCGAGCCA (SEQ ID NO:97) |
| 125 | R | CTGCGGTCGACCCCAGTTGCCGGTG (SEQ ID NO:98) |
| 133 | F | GGATGGCCCAGCTCTGCGAGCCNNSGCCTTGATTGGA (SEQ ID NO:99) |
| 133 | R | GGCTCGCAGAGCTGGGCCATCC (SEQ ID NO:100) |
| 137 | F | TGCGAGCCATTGCCTTGATTNNSTACTCAAAGTGGCTCATCAACA (SEQ ID NO:101) |
| 137 | R | AATCAAGGCAATGGCTCGCAGAGCT (SEQ ID NO:102) |
| 140 | F | CATTGCCTTGATTGGATACTCANNSTGGCTCATCAAC (SEQ ID NO:103) |
| 140 | R | TGAGTATCCAATCAAGGCAATG (SEQ ID NO:104) |
| 144 | F | TGGATACTCAAAGTGGCTCATCNNSAACAACTATCAG (SEQ ID NO:105) |
| 144 | R | GATGAGCCACTTTGAGTATCC (SEQ ID NO:106) |
| 145 | F | ATACTCAAAGTGGCTCATCAACNNSAACTATCAGTCG (SEQ ID NO:107) |
| 145 | R | GTTGATGAGCCACTTTGAGTA (SEQ ID NO:108) |
| 146 | F | CAAAGTGGCTCATCAACAACNNSTATCAGTCGACTGTGTCCAACG (SEQ ID NO:109) |
| 146 | R | GTTGTTGATGAGCCACTTTGAGTAT (SEQ ID NO:110) |
| 147 | F | AAAGTGGCTCATCAACAACAACNNSCAGTCGACTGTG (SEQ ID NO:111) |
| 147 | R | GTTGTTGTTGATGAGCCACTT (SEQ ID NO:112) |
| 148 | F | GGCTCATCAACAACAACTATNNSTCGACTGTGTCCAACGTCATCT (SEQ ID NO:113) |
| 148 | R | ATAGTTGTTGTTGATGAGCCACTTT (SEQ ID NO:114) |
| 152 | F | CAACAACTATCAGTCGACTGTGNNSAACGTCATCTGG (SEQ ID NO:115) |
| 152 | R | CACAGTCGACTGATAGTTGTT (SEQ ID NO:116) |
| 153 | F | CAACTATCAGTCGACTGTGTCCNNSGTCATCTGGCCT (SEQ ID NO:117) |
| 153 | R | GGACACAGTCGACTGATAGTT (SEQ ID NO:118) |

(continued)

| AA-position | F/R | DNA Sequence 5' to 3' |
|---|---|---|
| F = Forward; R = Reverse | | |
| 164 | F | CCTATTGTGCGCAACGACCTCNNSTATGTTGCCCAGT (SEQ ID NO:119) |
| 164 | R | GAGGTCGTTGCGCACAATAGG (SEQ ID NO:120) |
| 169 | F | ACCTCAACTATGTTGCCCAGNNSTGGAACCAAACCGGCTTTGACC (SEQ ID NO:121) |
| 169 | R | CTGGGCAACATAGTTGAGGTCGTTG (SEQ ID NO:122) |
| 172 | F | ATGTTGCCCAGTACTGGAACNNSACCGGCTTTGACCTCTGGGAAG (SEQ ID NO:123) |
| 172 | R | GTTCCAGTACTGGGCAACATAGTTG (SEQ ID NO:124) |
| 175 | F | AGTACTGGAACCAAACCGGCNNSGACCTCTGGGAAGAAGTCAATG (SEQ ID NO:125) |
| 175 | R | GCCGGTTTGGTTCCAGTACTGGGCA (SEQ ID NO:126) |
| 178 | F | ACCAAACCGGCTTTGACCTCNNSGAAGAAGTCAATGGGAGCTCAT (SEQ ID NO:127) |
| 178 | R | GAGGTCAAAGCCGGTTTGGTTCCAG (SEQ ID NO:128) |
| 180 | F | CGGCTTTGACCTCTGGGAANNSGTCAATGGGAGCTCATTCTTTA (SEQ ID NO:129) |
| 180 | R | TTCCCAGAGGTCAAAGCCGGTTTGG (SEQ ID NO:130) |
| 181 | F | GCTTTGACCTCTGGGAAGAANNSAATGGGAGCTCATTCTTTACTG (SEQ ID NO:131) |
| 181 | R | TTCTTCCCAGAGGTCAAAGCCGGTT (SEQ ID NO:132) |
| 182 | F | CTTTGACCTCTGGGAAGAAGTCNNSGGGAGCTCATTC (SEQ ID NO:133) |
| 182 | R | GACTTCTTCCCAGAGGTCAAAG (SEQ ID NO:134) |
| 204 | F | TGTCGAGGGCGCCACTCTTGCTNNSACTCTTGGCCAG (SEQ ID NO:135) |
| 204 | R | AGCAAGAGTGGCGCCCTCGAC (SEQ ID NO:136) |
| 205 | F | CGAGGGCGCCACTCTTGCTGCCNNSCTTGGCCAGTCG (SEQ ID NO:17) |
| 205 | R | GGCAGCAAGAGTGGCGCCCTC (SEQ ID NO:138) |
| 208 | F | CTCTTGCTGCCACTCTTGGCNNSTCGGGAAGCGCTTATTCATCTG (SEQ ID NO:139) |
| 208 | R | GCCAAGAGTGGCAGCAAGAGTGGCG (SEQ ID NO:140) |
| 211 | F | CCACTCTTGGCCAGTCGGGANNSGCTTATTCATCTGTTGCTCCCC (SEQ ID NO:141) |
| 211 | R | TCCCGACTGGCCAAGAGTGGCAGCA (SEQ ID NO:142) |
| 214 | F | TGGCCAGTCGGGAAGCGCTTATNNSTCTGTTGCTCCC (SEQ ID NO:143) |
| 214 | R | ATAAGCGCTTCCCGACTGGCC (SEQ ID NO:144) |
| 216 | F | GTCGGGAAGCGCTTATTCATCTNNSGCTCCCCAGGTT (SEQ ID NO:145) |
| 216 | R | AGATGAATAAGCGCTTCCCGA (SEQ ID NO:146) |
| 219 | F | CGCTTATTCATCTGTTGCTCCCNNSGTTTTGTGCTTT (SEQ ID NO:147) |
| 219 | R | GGGAGCAACAGATGAATAAGC (SEQ ID NO:148) |
| 228 | F | TGTGCTTTCTCCAACGATTCNNSGTGTCGTCTGGTGGATACGTCG (SEQ ID NO:149) |
| 228 | R | GAATCGTTGGAGAAAGCACAAAACCT (SEQ ID NO:150) |
| 229 | F | GTGCTTTCTCCAACGATTCTGGNNSTCGTCTGGTGGA (SEQ ID NO:151) |
| 229 | R | CCAGAATCGTTGGAGAAAGCA (SEQ ID NO:152) |
| 230 | F | CTTTCTCCAACGATTCTGGGTGNNSTCTGGTGGATACG (SEQ ID NO:153) |
| 230 | R | CACCCAGAATCGTTGGAGAAA (SEQ ID NO:154) |
| 231 | F | TCTCCAACGATTCTGGGTGTCGNNSGGTGGATACGTC (SEQ ID NO:155) |

(continued)

| AA-position | F/R | DNA Sequence 5' to 3' |
|---|---|---|
| | | F = Forward; R = Reverse |
| 231 | R | CGACACCCAGAATCGTTGGAGA (SEQ ID NO:156) |
| 236 | F | GGTGTCGTCTGGTGGATACGTCNNSTCCAACATCAACAC (SEQ ID NO:157) |
| 236 | R | GACGTATCCACCAGACGACAC (SEQ ID NO:158) |
| 239 | F | TGGTGGATACGTCGACTCCAACNNSAACACCAACGAG (SEQ ID NO:159) |
| 239 | R | GTTGGAGTCGACGTATCCACC (SEQ ID NO:160) |
| 240 | F | TGGATACGTCGACTCCAACATCNNSACCAACGAGGGCA (SEQ ID NO:161) |
| 240 | R | GATGTTGGAGTCGACGTATCCA (SEQ ID NO:162) |
| 241 | F | ATACGTCGACTCCAACATCAACNNSAACGAGGGCAGGAC (SEQ ID NO:163) |
| 241 | R | GTTGATGTTGGAGTCGACGTA (SEQ ID NO:164) |
| 242 | F | TCGACTCCAACATCAACACCNNSGAGGGCAGGACTGGCAAGGATG (SEQ ID NO:165) |
| 242 | R | GGTGTTGATGTTGGAGTCGACGTAT (SEQ ID NO:166) |
| 243 | F | ACTCCAACATCAACACCAACNNSGGCAGGACTGGCAAGGATGTCA (SEQ ID NO:167) |
| 243 | R | GTTGGTGTTGATGTTGGAGTCGACG (SEQ ID NO:168) |
| 244 | F | CTCCAACATCAACACCAACGAGNNSAGGACTGGCAAG (SEQ ID NO:169) |
| 244 | R | CTCGTTGGTGTTGATGTTGGAGT (SEQ ID NO:170) |
| 245 | F | ACATCAACACCAACGAGGGCNNSACTGGCAAGGATGTCAACTCCG (SEQ ID NO:171) |
| 245 | R | GCCCTCGTTGGTGTTGATGTTGGAGT (SEQ ID NO:172) |
| 263 | F | TTCCATCCACACCTTCGATCCCNNSCTTGGCTGTGAC (SEQ ID NO:173) |
| 263 | R | GGGATCGAAGGTGTGGATGGA (SEQ ID NO:174) |
| 264 | F | CATCCACACCTTCGATCCCAACNNSGGCTGTGACGCA (SEQ ID NO:175) |
| 264 | R | GTTGGGATCGAAGGTGTGGAT (SEQ ID NO:176) |
| 265 | F | CCACACCTTCGATCCCAACCTTNNSTGTGACGCAGGC (SEQ ID NO:177) |
| 265 | R | AAGGTTGGGATCGAAGGTGTG (SEQ ID NO:178) |
| 268 | F | CGATCCCAACCTTGGCTGTGACNNSGGCACCTTCCAGC (SEQ ID NO:179) |
| 268 | R | GTCACAGCCAAGGTTGGGATC (SEQ ID NO:180) |
| 269 | F | TCCCAACCTTGGCTGTGACGCANNSACCTTCCAGCCA (SEQ ID NO:181) |
| 269 | R | TGCGTCACAGCCAAGGTTGGG (SEQ ID NO:182) |
| 276 | F | AGGCACCTTCCAGCCATGCAGTNNSAAAGCGCTCTCC (SEQ ID NO:183) |
| 276 | R | ACTGCATGGCTGGAAGGTGCC (SEQ ID NO:184) |
| 284 | F | CAAAGCGCTCTCCAACCTCAAGNNSGTTGTCGACTCCT (SEQ ID NO:185) |
| 284 | R | CTTGAGGTTGGAGAGCGCTTT (SEQ ID NO:186) |
| 291 | F | GGTTGTTGTCGACTCCTTCCGCNNSATCTACGGCGTG (SEQ ID NO:187) |
| 291 | R | GCGCAAGGAGTCGACAACAAC (SEQ ID NO:188) |
| 292 | F | TTGTCGACTCCTTCCGCTCCNNSTACGGCGTGAACAAGGGCATTC (SEQ ID NO:189) |
| 292 | R | GGAGCGGAAGGAGTCGACAACAACC (SEQ ID NO:190) |
| 294 | F | ACTCCTTCCGCTCCATCTACNNSGTGAACAAGGGCATTCCTGCCG (SEQ ID NO:191) |
| 294 | R | GTAGATGGAGCGGAAGGAGTCGACA (SEQ ID NO:192) |

(continued)

| AA-position | F/R | DNA Sequence 5' to 3' |
|---|---|---|
| | | F = Forward; R = Reverse |
| 297 | F | GCTCCATCTACGGCGTGAACNNSGGCATTCCTGCCGGTGCTGCCG (SEQ ID NO:193) |
| 297 | R | GTTCACGCCGTAGATGGAGCGGAAG (SEQ ID NO:194) |
| 300 | F | CTACGGCGTGAACAAGGGCATTNNSGCCGGTGCTGCCG (SEQ ID NO:195) |
| 300 | R | AATGCCCTTGTTCACGCCGTA (SEQ ID NO:196) |
| 301 | F | CGGCGTGAACAAGGGCATTCCTNNSGGTGCTGCCGTC (SEQ ID NO:197) |
| 301 | R | AGGAATGCCCTTGTTCACGCC (SEQ ID NO:198) |
| 303 | F | GAACAAGGGCATTCCTGCCGGTNNSGCCGTCGCCATT (SEQ ID NO:199) |
| 303 | R | ACCGGCAGGAATGCCCTTGTT (SEQ ID NO:200) |
| 309 | F | GTGCTGCCGTCGCCATTGGCNNSTATGCAGAGGATGTGTACTACA (SEQ ID NO:201) |
| 309 | R | GCCAATGGCGACGGCAGCACCGGCA (SEQ ID NO:202) |
| 310 | F | CTGCCGTCGCCATTGGCCGGNNSGCAGAGGATGTGTACTACAACG (SEQ ID NO:203) |
| 310 | R | CCGGCCAATGGCGACGGCAGCACCG (SEQ ID NO:204) |
| 311 | F | TGCCGTCGCCATTGGCCGGTATNNSGAGGATGTGTAC (SEQ ID NO:205) |
| 311 | R | ATACCGGCCAATGGCGACGGC (SEQ ID NO:206) |
| 313 | F | CCATTGGCCGGTATGCAGAGNNSGTGTACTACAACGGCAACCCTT (SEQ ID NO:207) |
| 313 | F | CCATTGGCCGGTATGCAGAGNNSGTGTACTACAACGGCAACCCTT (SEQ ID NO:208) |
| 313 | R | CTCTGCATACCGGCCAATGGCGACG (SEQ ID NO:209) |
| 313 | R | CTCTGCATACCGGCCAATGGCGACG (SEQ ID NO:210) |
| 314 | F | TTGGCCGGTATGCAGAGGATNNSTACTACAACGGCAACCCTTGGT (SEQ ID NO:211) |
| 314 | R | ATCCTCTGCATACCGGCCAATGGCG (SEQ ID NO:212) |
| 315 | F | GCCGGTATGCAGAGGATGTGNNSTACAACGGCAACCCTTGGTATC (SEQ ID NO:213) |
| 315 | R | CACATCCTCTGCATACCGGCCAATG (SEQ ID NO:214) |
| 316 | F | CGTATGCAGAGGATGTGTACNNSAACGGCAACCCTTGGTATCTTG (SEQ ID NO:215) |
| 316 | R | GTACACATCCTCTGCATACCGGCCAAT (SEQ ID NO:216) |
| 317 | F | ATGCAGAGGATGTGTACTACNNSGGCAACCCTTGGTATCTTGCTA (SEQ ID NO:217) |
| 317 | F | ATGCAGAGGATGTGTACTACNNSGGCAACCCTTGGTATCTTGCTA (SEQ ID NO:218) |
| 317 | R | GTAGTACACATCCTCTGCATACCGGC (SEQ ID NO:219) |
| 317 | R | GTAGTACACATCCTCTGCATACCGGC (SEQ ID NO:220) |
| 321 | F | TGTACTACAACGGCAACCCTNNSTATCTTGCTACATTTGCTGCTG (SEQ ID NO:221) |
| 321 | F | TGTACTACAACGGCAACCCTNNSTATCTTGCTACATTTGCTGCTG (SEQ ID NO:222) |
| 321 | R | AGGGTTGCCGTRGTAGTACACATCC (SEQ ID NO:223) |
| 321 | R | AGGGTTGCCGTTGTAGTACACATCC (SEQ ID NO:224) |
| 338 | F | GCAGCTGTACGATGCCATCTACNNSTGGAAGAAGACG (SEQ ID NO:225) |
| 338 | R | GTAGATGGCATCGTACAGCTG (SEQ ID NO:226) |
| 340 | F | ACGATGCCATCTACGTCTGGNNSAAGACGGGCTCCATCACGGTGA (SEQ ID NO:227) |
| 340 | R | CCAGACGTAGATGGCATCGTACAGC (SEQ ID NO:228) |
| 341 | F | ATGCCATCTACGTCTGGAAGNNSACGGGCTCCATCACGGTGACCG (SEQ ID NO:229) |

(continued)

| AA-position | F/R | DNA Sequence 5' to 3' |
|---|---|---|
| | | F = Forward; R = Reverse |
| 341 | R | CTTCCAGACGTAGATGGCATCGTACAGC (SEQ ID NO:230) |
| 342 | F | ATGCCATCTACGTCTGGAAGAAGNNSGGCTCCATCACG (SEQ ID NO:231) |
| 342 | R | CTTCTTCCAGACGTAGATGGC (SEQ ID NO:232) |
| 344 | F | CTACGTCTGGAAGAAGACGGGCNNSATCACGGTGACC (SEQ ID NO:233) |
| 344 | R | GCCCGTCTTCTTCCAGACGTAG (SEQ ID NO:234) |
| 346 | F | CTGGAAGAAGACGGGCTCCATCNNSGTGACCGCCACCTC (SEQ ID NO:235) |
| 346 | R | GATGGAGCCCGTCTTCTTCCA (SEQ ID NO:236) |
| 349 | F | GACGGGCTCCATCACGGTGACCNNSACCTCCCTGGCC (SEQ ID NO:237) |
| 349 | R | GGTCACCGTGATGGAGCCCGT (SEQ ID NO:238) |
| 350 | F | GCTCCATCACGGTGACCGCCNNSTCCCTGGCCTTCTTCCAGGAGC (SEQ ID NO:239) |
| 350 | R | GGCGGTCACCGTGATGGAGCCCGTC (SEQ ID NO:240) |
| 356 | F | CCACCTCCCTGGCCTTCTTCNNSGAGCTTGTTCCTGGCGTGACGG (SEQ ID NO:241) |
| 356 | R | GAAGAAGGCCAGGGAGGTGGCGGTC (SEQ ID NO:242) |
| 359 | F | CCTGGCCTTCTTCCAGGAGCTTNNSCCTGGCGTGACG (SEQ ID NO:243) |
| 359 | R | AAGCTCCTGGAAGAAGGCCAG (SEQ ID NO:244) |
| 361 | F | CTTCTTCCAGGAGCTTGTTCCTNNSGTGACCGCCGGG (SEQ ID NO:245) |
| 361 | R | AGGAACAAGCTCCTGGAAGAA (SEQ ID NO:246) |
| 363 | F | AGGAGCTTGTTCCTGGCGTGNNSGCCGGGACCTACTCCAGCAGCT (SEQ ID NO:247) |
| 363 | R | CACGCCAGGAACAAGCTCCTGGAAG (SEQ ID NO:248) |
| 364 | F | GGAGCTTGTTCCTGGCGTGACGNNSGGGACCTACTCC (SEQ ID NO:249) |
| 364 | R | CGTCACGCCAGGAACAAGCTC (SEQ ID NO:250) |
| 368 | F | GCGTGACGGCCGGGACCTACNNSAGCAGCTCTTCGACCTTTACCA (SEQ ID NO:251) |
| 368 | R | GTAGGTCCCGGCCGTCACGCCAGGA (SEQ ID NO:252) |
| 369 | F | TGACGGCCGGGACCTACTCCNNSAGCTCTTCGACCTTTACCAACA (SEQ ID NO:253) |
| 369 | R | GGAGTAGGTCCCGGCCGTCACGCCA (SEQ ID NO:254) |
| 375 | F | GTCCAGCAGCTCTTCGACCTTTNNSAACATCATCAACG (SEQ ID NO:255) |
| 375 | R | AAAGGTCGAAGAGCTGCTGGA (SEQ ID NO:256) |
| 376 | F | GCAGCTCTTCGACCTTTACCNNSATCATCAACGCCGTCTCGACAT (SEQ ID NO:257) |
| 376 | R | GGTAAAGGTCGAAGAGCTGCTGGAG (SEQ ID NO:258) |
| 379 | F | TTCGACCTTTACCAACATCATCNNSGCCGTCTCGACA (SEQ ID NO:259) |
| 379 | R | GATGATGTTGGTAAAGGTCGA (SEQ ID NO:260) |
| 382 | F | TACCAACATCATCAACGCCGTCNNSACATACGCCGAT (SEQ ID NO:261) |
| 382 | R | GACGGCGTTGATGATGTTGGT (SEQ ID NO:262) |
| 390 | F | GACATACGCCGATGGCTTCCTCNNSGAGGCTGCCAAG (SEQ ID NO:263) |
| 390 | R | GAGGAAGCCATCGGCGTATGT (SEQ ID NO:264) |
| 391 | F | ATACGCCGATGGCTTCCTCAGCNNSGCTGCCAAGTAC (SEQ ID NO:265) |
| 391 | R | GCTGAGGAAGCCATCGGCGTA (SEQ ID NO:266) |

(continued)

| | AA-position | F/R | DNA Sequence 5' to 3' |
|---|---|---|---|
| F = Forward; R = Reverse | | | |
| | 393 | F | CGATGGCTTCCTCAGCGAGGCTNNSAAGTACGTCCCC (SEQ ID NO:267) |
| | 393 | R | AGCCTCGCTGAGGAAGCCATC (SEQ ID NO:268) |
| | 394 | F | TGGCTTCCTCAGCGAGGCTGCCNNSTACGTCCCCGCC (SEQ ID NO:269) |
| | 394 | R | GGCAGCCTCGCTGAGGAAGCC (SEQ ID NO:270) |
| | 395 | F | TCCTCAGCGAGGCTGCCAAGNNSGTCCCCGCCGACGGTTCGCTGG (SEQ ID NO:271) |
| | 395 | R | CTTGGCAGCCTCGCTGAGGAAGCCA (SEQ ID NO:272) |
| | 398 | F | AGGCTGCCAAGTACGTCCCCNNSGACGGTTCGCTGGCCGAGCAGTT (SEQ ID NO:273) |
| | 398 | R | GGGACGTACTTGGCAGCCTCGCTG (SEQ ID NO:274) |
| | 401 | F | AGTACGTCCCCGCCGACGGTNNSCTGGCCGAGCAGTTTGACCGCA (SEQ ID NO:275) |
| | 401 | R | ACCGTCGGCGGGGACGTACTTGGCAG (SEQ ID NO:276) |
| | 408 | F | CGCTGGCCGAGCAGTTTGACNNSAACAGCGGCACTCCGCTGTCTG (SEQ ID NO:277) |
| | 408 | R | GTCAAACTGCTCGGCCAGCGAACCG (SEQ ID NO:278) |
| | 409 | F | TGGCCGAGCAGTTTGACCGCNNSAGCGGCACTCCGCTGTCTGCGC (SEQ ID NO:279) |
| | 409 | R | GCGGTCAAACTGCTCGGCCAGCGAA (SEQ ID NO:280) |
| | 410 | F | GGCCGAGCAGTTTGACCGCAACNNSGGCACTCCGCTG (SEQ ID NO:281) |
| | 410 | R | GTTGCGGTCAAACTGCTCGGC (SEQ ID NO:282) |
| | 412 | F | AGTTTGACCGCAACAGCGGCNNSCCGCTGTCTGCGCTTCACCTGA (SEQ ID NO:283) |
| | 412 | R | GCCGCTGTTGCGGTCAAACTGCTCG (SEQ ID NO:284) |
| | 415 | F | GCAACAGCGGCACTCCGCTGNNSGCGCTTCACCTGACGTGGTCGT (SEQ ID NO:285) |
| | 415 | R | CAGCGGAGTGCCGCTGTTGCGGTCA (SEQ ID NO:286) |
| | 417 | F | CAGCGGCACTCCGCTGTCTGCGNNSCACCTGACGTGGT (SEQ ID NO:287) |
| | 417 | R | CGCAGACAGCGGAGTGCCGCT (SEQ ID NO:288) |
| | 418 | F | GCACTCCGCTGTCTGCGCTTNNSCTGACGTGGTCGTACGCCTCGT (SEQ ID NO:289) |
| | 418 | R | AAGCGCAGACAGCGGAGTGCCGCTG (SEQ ID NO:290) |
| | 421 | F | TGTCTGCGCTTCACCTGACGNNSTCGTACGCCTCGTTCTTGACAG (SEQ ID NO:291) |
| | 421 | R | CGTCAGGTGAAGCGCAGACAGCGGA (SEQ ID NO:292) |
| | 430 | F | GTACGCCTCGTTCTTGACAGCCNNSGCCCGTCGGGCT (SEQ ID NO:293) |
| | 430 | R | GGCTGTCAAGAACGAGGCGTA (SEQ ID NO:294) |
| | 431 | F | CGCCTCGTTCTTGACAGCCACGNNSCGTCGGGCTGGC (SEQ ID NO:295) |
| | 431 | R | CGTGGCTGTCAAGAACGAGGC (SEQ ID NO:296) |
| | 433 | F | TCTTGACAGCCACGGCCCGTNNSGCTGGCATCGTGCCCCCCTCGT (SEQ ID NO:297) |
| | 433 | R | ACGGGCCGTGGCTGTCAAGAACGAG (SEQ ID NO:298) |
| | 436 | F | CCACGGCCCGTCGGGCTGGCNNSGTGCCCCCCTCGTGGGCCAACA (SEQ ID NO:299) |
| | 436 | R | GCCAGCCCGACGGGCCGTGGCTGTC (SEQ ID NO:300) |
| | 442 | F | TGGCATCGTGCCCCCCTCGTGGNNSAACAGCAGCGCT (SEQ ID NO:301) |
| | 442 | R | CCACGAGGGGGGCACGATGCC (SEQ ID NO:302) |
| | 443 | F | CATCGTGCCCCCCTCGTGGGCCNNSAGCAGCGCTAGC (SEQ ID NO:303) |

(continued)

| F = Forward; R = Reverse | | |
|---|---|---|
| AA-position | F/R | DNA Sequence 5' to 3' |
| 443 | R | CGCCCACGAGGGGGGCACGAT (SEQ ID NO:304) |
| 444 | F | CGTGCCCCCCTCGTGGGCCAACNNSAGCGCTAGCACG (SEQ ID NO:305) |
| 444 | R | GTTGGCCCACGAGGGGGGCAC (SEQ ID NO:306) |
| 448 | F | GTGGGCCAACAGCAGCGCTAGCNNSATCCCCTCGACG (SEQ ID NO:307) |

## Example 2: Transformation of TrGA SELs into *Trichoderma reesei*

The SELs were transformed into T. *reesei* using the PEG protoplast method. The *E.coli* clones of the SELs confirmed by sequence analysis were grown overnight at 37°C in deep well microtiter plates (Greiner Art. No. 780271) containing 1.200 $\mu$l of 2xYT medium with ampicillin (100 $\mu$g/ml) and kanamycin (50 $\mu$g/ml). Plasmid DNAs were isolated from the cultures using CHEMAGIC® Plasmid Mini Kit (Chemagen - Biopolymer Technologie AG, Baesweiler, Germany) and were transformed individually into a *T. reesei* host strain derived from RL-P37 bearing four gene deletions ($\Delta$cbh1, $\Delta$cbh2, $\Delta$egl1, $\Delta$egl2, i.e., "quad-deleted"; see USP 5,847,276, WO 92/06184 and WO 05/001036) using the PEG-Protoplast method (Penttilä et al. (1987) *Gene* 61:155-164) with the following modifications. 1) For protoplast preparation, spores were grown for 16-24 hours at 24°C in *Trichoderma* Minimal Medium (MM) (20g/L glucose, 15g/L $KH_2PO_4$, pH 4.5, 5g/L $(NH_4)_2SO_4$, 0.6g/L $MgSO_4x7H_2O$, 0.6 g/L $CaCl_2x2H_2O$, 1 ml of 1000X T. *reesei* Trace elements solution {5 g/L $FeSO_4x7H_2O$, 1.4 g/L $ZnSO_4x7H_2O$, 1.6 g/L $MnSO_4xH_2O$, 3.7 g/L $CoCl_2x6H_2O$}) with shaking at 150 rpm. Germinating spores were harvested by centrifugation and treated with 15mg/ml of $\beta$-D-glucanase-G (Interspex- Art.No. 0439-1) solution to lyse the fungal cell walls. Further preparation of protoplasts was performed by a standard method, as described by Penttilä et al. (1987 *supra*). 2) The transformation method was scaled down 10 fold. In general, transformation mixtures containing up to 600 ng of DNA and 1-5x $10^5$ protoplasts in a total volume of 25 $\mu$l were treated with 200 ml of 25% PEG solution, diluted with 2 volumes of 1.2M sorbitol solution, mixed with 3% selective top agarose MM with acetamide (the same Minimal Medium as mentioned above but $(NH_4)_2SO_4$ was substituted with 20 mM acetamide) and poured onto 2% selective agarose with acetamide either in 24 well microtiter plates or in a 20x20 cm Q-tray divided in 48 wells. The plates were incubated at 28°C for 5 to 8 days. Spores from the total population of transformants regenerated on each individual well were harvested from the plates using a solution of 0.85% NaCl, 0.015% Tween 80. Spore suspensions were used to inoculate fermentations in 96 wells MTPs. In the case of 24 well MTPs, an additional plating step on a fresh 24 well MTP with selective acetamide MM was introduced in order to enrich the spore numbers.

## Example 3: Fermentation of *T. reesei* transformants expressing TrGA variants in a MTP format

The tranformants were fermented and the supernatants containing the expressed variant TrGA proteins were tested for various properties. In brief, 96 well filter plates (Corning Art.No. 3505) containing 200$\mu$l of LD-GSM medium (5.0 g/L $(NH_4)_2SO_4$, 33 g/L 1,4-Piperazinebis(propanesulfonic acid), pH 5.5, 9.0 g/L Casamino acids, 1.0 g/L $KH_2PO_4$, 1.0 g/L $CaCl_2x2H_2O$, 1.0 g/L $MgSO_4x7H_2O$, 2.5 ml/L of 1000X *T.reesei* trace elements, 20 g/L Glucose, 10 g/L Sophorose) were inoculated in quadruplicate with spore suspensions of *T. reesei* transformants expressing TrGA variants (more than $10^4$ spores per well). The plates were incubated at 28°C with 230 rpm shaking and 80% humidity for 6 days. Culture supernatants were harvested by vacuum filtration. The supernatants were used in different assays for screening of variants with improved properties.

## Example 4: Preparation of the whole broth samples from GA-producing transformants

TrGA producing transformants were initially pregrown in 250 ml shake flasks containing 30 ml of Proflo medium. Proflo medium contained: 30 g/L $\alpha$-lactose, 6.5 g/L $(NH_4)_2SO_4$, 2 g/L $KH_2PO_4$, 0.3 g/L $MgSO_4x7H_2O$, 0.2 g/L $CaCl_2x2H_2O$, 1 ml/L 1000X trace element salt solution as mentioned above, 2 ml/L 10% Tween 80, 22.5 g/L ProFlo cottonseed flour (Traders protein, Memphis, TN), 0.72 g/L $CaCO_3$. After two days of growth at 28°C and 140 rpm, 10% of the Proflo culture was transferred into a 250 ml shake flask containing 30 ml of Lactose Defined Medium. The composition of the Lactose Defined Medium was as follows: 5 g/L $(NH_4)_2SO_4$, 33 g/L 1,4-Piperazinebis (propanesulfonic acid) buffer, pH 5.5, 9 g/L casamino acids, 4.5 g/L $KH_2PO_4$, 1.0 g/L $MgSO_4$ $x7H_2O$, 5 ml/L Mazu DF60-P antifoam (Mazur Chemicals, IL), 1ml/l of 1000X trace element solution. 40 ml/L of 40% (w/v) lactose solution was added to the medium after sterilization. Shake flasks with the Lactose Defined Medium were incubated at 28°C, 140 rpm for 4 - 5 days.

Mycelium was removed from the culture samples by centrifugation and the supernatant was analyzed for total protein content (BCA Protein Assay Kit, Pierce Cat. No.23225) and GA activity, as described above in the Experimental section.

The protein profile of the whole broth samples was determined by SDS-PAGE electrophoresis. Samples of the culture supernatant were mixed with an equal volume of 2X sample loading buffer with reducing agent and separated on NUPAGE® Novex 10% Bis-Tris Gel with MES SDS Running Buffer (Invitrogen, Carlsbad, CA, USA). Polypeptide bands were visualized in the SDS gel with SIMPLYBLUE SafeStain (Invitrogen, Carlsbad, CA, USA).

**Example 5: Variants with improved thermal stability**

The parent TrGA molecule had a residual activity between 15 and 44 % (day-to-day variation) under the conditions described. The performance index was calculated based on the TrGA thermostability of the same batch. The performance indices are the quotients PI = (Variant residual activity)/(TrGA residual activity). A performance index >1 indicated an improved stability. Variants which had a thermal stability performance index (PI) of more than 1.0 are shown in the following Table 3.

Table 3 - Thermal stability screening

| Variant | Performance Index of Thermal Stability | | |
|---------|-----------------------------------------|---------|------|
| T010F | 1.11 | K114C | 1.19 |
| T010G | 1.13 | K114D | 1.17 |
| T010M | 1.12 | K114E | 1.16 |
| T010Q | 1.06 | K114L | 1.10 |
| T010R | 1.06 | K114M | 1.21 |
| T010S | 1.24 | K114Q | 1.25 |
| T042V | 1.31 | I133V | 1.21 |
| F059A | 1.03 | K140D | 1.09 |
| F059G | 1.07 | K140Q | 1.06 |
| F059L | 1.06 | K140S | 1.05 |
| F059M | 1.12 | K140W | 1.04 |
| F059Q | 1.10 | N144A | 1.11 |
| F059V | 1.03 | N144F | 1.06 |
| N061V | 1.06 | S152C | 1.04 |
| E068C | 1.23 | S152G | 1.09 |
| E068F | 1.14 | S152I | 1.09 |
| E068G | 1.15 | S152N | 1.12 |
| E068I | 1.19 | N153A | 1.28 |
| E068K | 1.01 | N153D | 1.06 |
| E068M | 1.29 | N153E | 1.29 |
| E068N | 1.18 | N153F | 1.16 |
| E068Q | 1.15 | N153H | 1.01 |
| E068W | 1.09 | N153L | 1.06 |
| A072E | 1.07 | N153M | 1.27 |
| A072Q | 1.02 | N153S | 1.31 |
| G073F | 1.44 | N153V | 1.34 |
| G073M | 1.01 | N153W | 1.19 |
| G073N | 1.10 | N182R | 1.02 |
| G073W | 1.36 | A204D | 1.02 |
| S097E | 1.08 | A204M | 1.08 |
| S097G | 1.03 | T205C | 1.02 |
| S097T | 1.17 | T205D | 1.06 |
| S097V | 1.03 | T205N | 1.09 |
| L098C | 1.07 | T205P | 1.17 |
| A099C | 1.07 | T205S | 1.04 |
| A099F | 1.02 | T205V | 1.06 |
| A099I | 1.14 | T205Y | 1.07 |
| A099L | 1.09 | S214C | 1.02 |
| A099Q | 1.07 | S214E | 1.03 |
| A099R | 1.02 | S214N | 1.07 |
| A099S | 1.11 | S214P | 1.04 |
| A099T | 1.02 | S214Q | 1.13 |
| S102A | 1.02 | S214T | 1.06 |
| | | S214V | 1.13 |
| | | S214W | 1.03 |

| | | | | |
|---|---|---|---|---|
| S214Y | 1.04 | | S231M | 1.09 |
| V216I | 1.13 | | S231N | 1.13 |
| W228A | 1.01 | | S231Q | 1.18 |
| W228F | 1.12 | | S231R | 1.13 |
| W228G | 1.06 | | S231T | 1.16 |
| W228H | 1.05 | | S231V | 1.21 |
| W228I | 1.06 | | S231W | 1.06 |
| W228L | 1.14 | | S231Y | 1.10 |
| W228M | 1.04 | | D236A | 1.10 |
| W228Q | 1.15 | | D236C | 1.16 |
| W228S | 1.15 | | D236E | 1.06 |
| W228T | 1.06 | | D236F | 1.11 |
| W228V | 1.21 | | D236G | 1.07 |
| W228Y | 1.10 | | D236H | 1.16 |
| V229A | 1.16 | | D236I | 1.14 |
| V229D | 1.18 | | D236K | 1.13 |
| V229E | 1.16 | | D236L | 1.08 |
| V229F | 1.17 | | D236M | 1.15 |
| V229G | 1.18 | | D236N | 1.15 |
| V229H | 1.13 | | D236P | 1.06 |
| V229I | 1.21 | | D236R | 1.29 |
| V229L | 1.20 | | D236T | 1.16 |
| V229M | 1.11 | | D236V | 1.18 |
| V229N | 1.07 | | D236Y | 1.07 |
| V229P | 1.13 | | T241V | 1.05 |
| V229Q | 1.10 | | N242F | 1.03 |
| V229R | 1.07 | | N242H | 1.01 |
| V229S | 1.12 | | N263A | 1.18 |
| V229T | 1.11 | | N263C | 1.14 |
| V229W | 1.07 | | N263L | 1.02 |
| V229Y | 1.08 | | N263M | 1.10 |
| S230C | 1.09 | | N263R | 1.04 |
| S230D | 1.08 | | N263T | 1.07 |
| S230E | 1.11 | | L264A | 1.03 |
| S230F | 1.08 | | L264D | 1.20 |
| S230G | 1.09 | | L264I | 1.14 |
| S230H | 1.03 | | L264K | 1.24 |
| S230K | 1.02 | | L264M | 1.15 |
| S230L | 1.02 | | L264P | 1.02 |
| S230M | 1.08 | | L264R | 1.02 |
| S230N | 1.16 | | L264V | 1.04 |
| S230P | 1.12 | | L264Y | 1.15 |
| S230Q | 1.20 | | G265D | 1.08 |
| S230R | 1.11 | | G265E | 1.03 |
| S230T | 1.07 | | G265F | 1.08 |
| S230V | 1.11 | | G265H | 1.09 |
| S230Y | 1.05 | | G265I | 1.09 |
| S231A | 1.06 | | G265K | 1.05 |
| S231C | 1.07 | | G265L | 1.07 |
| S231D | 1.18 | | G265N | 1.15 |
| S231E | 1.10 | | G265P | 1.07 |
| S231F | 1.14 | | G265Q | 1.15 |
| S231K | 1.09 | | G265R | 1.12 |
| S231L | 1.15 | | G265S | 1.13 |

| | | | | |
|---|---|---|---|---|
| G265T | 1.17 | | P300A | 1.10 |
| G265V | 1.15 | | P300N | 1.06 |
| G265W | 1.11 | | P300Q | 1.05 |
| G265Y | 1.10 | | P300R | 1.02 |
| A268C | 1.16 | | P300T | 1.07 |
| A268D | 1.20 | | P300V | 1.02 |
| A268E | 1.10 | | P300W | 1.11 |
| A268F | 1.07 | | P300Y | 1.04 |
| A268G | 1.11 | | A301A | 1.13 |
| A268I | 1.12 | | A301K | 1.11 |
| A268K | 1.11 | | A301L | 1.07 |
| A268L | 1.11 | | A301P | 1.22 |
| A268M | 1.18 | | A301R | 1.21 |
| A268N | 1.10 | | A301S | 1.12 |
| A268P | 1.13 | | A301T | 1.14 |
| A268R | 1.15 | | A301V | 1.07 |
| A268S | 1.09 | | A301W | 1.14 |
| A268T | 1.14 | | A301Y | 1.14 |
| A268W | 1.05 | | A303E | 1.02 |
| G269K | 1.01 | | A303I | 1.02 |
| G269N | 1.08 | | A303L | 1.03 |
| G269P | 1.08 | | A303Q | 1.01 |
| G269Q | 1.02 | | A311C | 1.05 |
| G269R | 1.06 | | A311E | 1.06 |
| D276E | 1.03 | | A311G | 1.11 |
| D276Q | 1.03 | | A311H | 1.09 |
| D276S | 1.02 | | A311I | 1.04 |
| D276V | 1.17 | | A311K | 1.13 |
| V284C | 1.09 | | A311L | 1.07 |
| V284E | 1.14 | | A311R | 1.07 |
| V284G | 1.13 | | A311S | 1.01 |
| V284I | 1.10 | | A311Y | 1.02 |
| V284Q | 1.08 | | V338H | 1.12 |
| V284R | 1.09 | | V338I | 1.21 |
| V284S | 1.06 | | V338L | 1.17 |
| V284T | 1.08 | | V338M | 1.13 |
| V284W | 1.08 | | V338N | 1.23 |
| V284Y | 1.12 | | V338P | 1.18 |
| S291A | 1.21 | | V338Q | 1.20 |
| S291D | 1.02 | | V338S | 1.19 |
| S291E | 1.02 | | V338Y | 1.10 |
| S291F | 1.24 | | T342C | 1.06 |
| S291G | 1.05 | | T342I | 1.04 |
| S291H | 1.25 | | T342L | 1.17 |
| S291I | 1.10 | | T342P | 1.08 |
| S291K | 1.13 | | S344A | 1.05 |
| S291M | 1.21 | | S344C | 1.16 |
| S291N | 1.08 | | S344D | 1.02 |
| S291P | 1.11 | | S344F | 1.10 |
| S291Q | 1.08 | | S344K | 1.12 |
| S291R | 1.07 | | S344M | 1.26 |
| S291T | 1.21 | | S344N | 1.16 |
| S291V | 1.06 | | S344P | 1.20 |
| S291W | 1.15 | | S344Q | 1.22 |

36

| | | | | |
|---|---|---|---|---|
| S344R | 1.22 | | A364G | 1.25 |
| S344T | 1.19 | | A364K | 1.27 |
| S344V | 1.20 | | A364L | 1.31 |
| S344W | 1.03 | | A364M | 1.21 |
| T346D | 1.03 | | A364Q | 1.19 |
| T346L | 1.02 | | A364R | 1.28 |
| T346M | 1.07 | | A364S | 1.23 |
| T346N | 1.15 | | A364T | 1.23 |
| T346P | 1.13 | | A364V | 1.23 |
| T346Q | 1.12 | | A364W | 1.23 |
| T346S | 1.13 | | T375A | 1.17 |
| T346V | 1.09 | | T375E | 1.03 |
| T346W | 1.06 | | T375F | 1.06 |
| T346Y | 1.07 | | T375G | 1.05 |
| A349D | 1.15 | | T375H | 1.13 |
| A349E | 1.13 | | T375K | 1.10 |
| A349F | 1.09 | | T375L | 1.03 |
| A349G | 1.12 | | T375M | 1.17 |
| A349H | 1.11 | | T375N | 1.20 |
| A349K | 1.14 | | T375P | 1.15 |
| A349L | 1.16 | | T375V | 1.16 |
| A349M | 1.07 | | T375W | 1.11 |
| A349N | 1.09 | | T375Y | 1.17 |
| A349P | 1.03 | | N379A | 1.11 |
| A349Q | 1.09 | | N379C | 1.03 |
| A349R | 1.04 | | N379D | 1.06 |
| A349T | 1.09 | | N379F | 1.07 |
| A349V | 1.08 | | N379G | 1.09 |
| A349W | 1.04 | | N379H | 1.07 |
| A349Y | 1.04 | | N379I | 1.04 |
| V359Q | 1.02 | | N379K | 1.04 |
| V359R | 1.13 | | N379L | 1.04 |
| V359Y | 1.01 | | N379M | 1.08 |
| G361A | 1.13 | | N379P | 1.17 |
| G361C | 1.16 | | N379Q | 1.11 |
| G361D | 1.35 | | N379R | 1.07 |
| G361E | 1.24 | | N379T | 1.09 |
| G361F | 1.20 | | N379V | 1.13 |
| G361H | 1.11 | | N379W | 1.09 |
| G361I | 1.20 | | N379Y | 1.06 |
| G361K | 1.16 | | S382A | 1.06 |
| G361L | 1.22 | | S382D | 1.04 |
| G361M | 1.28 | | S382E | 1.05 |
| G361P | 1.27 | | S382G | 1.06 |
| G361R | 1.19 | | S382I | 1.07 |
| G361S | 1.22 | | S382K | 1.03 |
| G361T | 1.16 | | S382N | 1.07 |
| G361V | 1.15 | | S382P | 1.10 |
| G361W | 1.22 | | S382R | 1.05 |
| G361Y | 1.26 | | S382T | 1.02 |
| A364C | 1.18 | | S382Y | 1.04 |
| A364D | 1.25 | | S390M | 1.06 |
| A364E | 1.27 | | S390Q | 1.02 |
| A364F | 1.33 | | S390R | 1.02 |

| | | | | |
|---|---|---|---|---|
| E391L | 1.04 | | T430F | 1.06 |
| E391R | 1.02 | | T430H | 1.10 |
| E391S | 1.02 | | T430I | 1.04 |
| E391W | 1.04 | | T430K | 1.08 |
| E391Y | 1.02 | | T430M | 1.17 |
| A393D | 1.11 | | T430N | 1.13 |
| A393E | 1.03 | | T430Q | 1.05 |
| A393F | 1.09 | | T430R | 1.13 |
| A393G | 1.12 | | T430S | 1.17 |
| A393H | 1.05 | | T430V | 1.05 |
| A393I | 1.06 | | A431I | 1.03 |
| A393K | 1.05 | | A431N | 1.03 |
| A393L | 1.17 | | A431P | 1.08 |
| A393M | 1.07 | | A431R | 1.08 |
| A393N | 1.11 | | A431V | 1.03 |
| A393Q | 1.02 | | R433A | 1.13 |
| A393R | 1.03 | | R433C | 1.24 |
| A393S | 1.13 | | R433E | 1.22 |
| A393T | 1.08 | | R433F | 1.17 |
| A393V | 1.09 | | R433G | 1.23 |
| A393W | 1.10 | | R433K | 1.12 |
| A393Y | 1.12 | | R433L | 1.23 |
| K394A | 1.08 | | R433M | 1.10 |
| K394C | 1.06 | | R433N | 1.28 |
| K394E | 1.07 | | R433S | 1.23 |
| K394F | 1.09 | | R433V | 1.28 |
| K394G | 1.05 | | R433W | 1.16 |
| K394H | 1.08 | | R433Y | 1.18 |
| K394L | 1.08 | | I436E | 1.07 |
| K394M | 1.06 | | I436F | 1.02 |
| K394Q | 1.07 | | I436G | 1.09 |
| K394R | 1.06 | | I436H | 1.20 |
| K394T | 1.03 | | I436K | 1.14 |
| K394V | 1.02 | | I436P | 1.15 |
| S410E | 1.05 | | I436R | 1.16 |
| S410H | 1.11 | | I436S | 1.17 |
| S410I | 1.06 | | I436T | 1.18 |
| S410K | 1.11 | | I436V | 1.12 |
| S410L | 1.05 | | I436Y | 1.05 |
| S410M | 1.02 | | A442N | 1.06 |
| S410N | 1.10 | | A442R | 1.04 |
| S410Q | 1.15 | | A442T | 1.09 |
| S410R | 1.18 | | S444E | 1.04 |
| S410T | 1.04 | | S444K | 1.07 |
| S410V | 1.13 | | S444M | 1.05 |
| L417I | 1.04 | | S444Q | 1.04 |
| L417K | 1.20 | | T448A | 1.02 |
| L417M | 1.05 | | T448E | 1.09 |
| L417Q | 1.04 | | T448F | 1.12 |
| L417R | 1.20 | | T448I | 1.12 |
| L417V | 1.07 | | T448L | 1.09 |
| L417Y | 1.01 | | T448M | 1.11 |
| T430A | 1.05 | | T448Q | 1.17 |
| T430E | 1.02 | | T448R | 1.09 |

| | |
|---|---|
| T448S | 1.07 |
| T448V | 1.13 |
| T448W | 1.01 |
| T448Y | 1.08 |
| S451E | 1.04 |

| | |
|---|---|
| S451H | 1.18 |
| S451K | 1.08 |
| S451L | 1.01 |
| S451Q | 1.06 |
| S451T | 1.02 |

Table 3 includes those variants that, when tested, showed an increased performance index over the parent glucoamylase. These included the following sites: 10, 42, S9, 60, 61, 68, 72, 73, 97, 98, 99, 102, 114, 133, 140,144,147,152,153, 164, 182, 204, 205, 214, 216, 228, 229, 230, 231, 236, 241, 242, 263, 264, 265, 268, 269, 276, 284, 291, 300, 301, 303, 311, 338, 342, 344, 346, 349, 359, 361, 364, 375, 379, 382, 390, 391, 393, 394, 410, 417, 430, 431, 433, 436, 442, 443, 444, 448, and 451. The sites with substitutions showing the highest increase included: T10S, T42V, E68C, E68M, G73F, G73W, K114M, K114T, 1133V, N153A, N153S, N153V, W228V, V2291, V229L, S231V, D236R, L264D, S291M, S291T, A301 P, A301R, S344M, S344P, S344Q, S344V, G361D, G361E, G361F, G361I, G361 L, G361M, G361P, G361S, G361W, G361Y, A364D, A364E, A364F, A364G, A364K, A364L, A354R, A364S, A364V, A364W, T375N, R433C, R433G, R433N, R433S, R433V, and I436H.

### Example 6: Variants with improved specific activity (SA) in an ethanol screening assay

Variants were tested in an ethanol screening assay using the assays described above. Table 4 shows the results of the screening assay for variants with a Performance Index (PI) > 1.0 compared to the parent TrGA PI. The PI is calculated from the specific activities (activity/mg enzyme) of the WT and the variant enzymes. It is the quotient "Variant-specific activity/WT-specific activity." The PI of the specific activity for the wildtype TrGA was 1.0 and a variant with a PI > 1.0 had a specific activity that was greater than the parent TrGA. The specific activity was the activity measured by the ethanol screening assay divided by the results obtained in the Caliper assay described above.

### Table 4 – Ethanol Screening

| Variant | PI for Specific Activity |
|---|---|
| T010D | 1.09 |
| T010F | 1.06 |
| T010G | 1.12 |
| T010K | 1.05 |
| T010L | 1.07 |
| T010M | 1.05 |
| T010P | 1.05 |
| T010R | 1.08 |
| T010S | 1.09 |
| L014E | 1.03 |
| L014H | 1.05 |
| N015D | 1.02 |
| P023A | 1.16 |

| | |
|---|---|
| P023F | 1.13 |
| P023N | 1.05 |
| F059A | 1.17 |
| F059F | 1.05 |
| F059G | 1.05 |
| K060F | 1.06 |
| K060H | 1.03 |
| N061D | 1.05 |
| N0611 | 1.21 |
| N061L | 1.18 |
| N061Q | 1.08 |
| N061V | 1.11 |
| N061W | 1.02 |
| R065A | 1.17 |
| R065C | 1.08 |
| R065G | 1.08 |
| R0651 | 1.11 |
| R065K | 1.09 |
| R065M | 1.07 |

| | | | | |
|---|---|---|---|---|
| R065S | 1.12 | | I133T | 1.29 |
| R065V | 1.14 | | K140A | 1.04 |
| R065Y | 1.01 | | K140E | 1.04 |
| T067C | 1.14 | | K140F | 1.03 |
| T067I | 1.13 | | K140H | 1.14 |
| T067K | 1.05 | | K140L | 1.10 |
| T067M | 1.22 | | K140M | 1.11 |
| E068I | 1.06 | | K140N | 1.15 |
| E068M | 1.10 | | K140Q | 1.08 |
| E068W | 1.03 | | K140R | 1.12 |
| A072E | 1.11 | | K140S | 1.13 |
| A072G | 1.02 | | K140V | 1.15 |
| A072L | 1.03 | | K140W | 1.07 |
| A072M | 1.11 | | K140Y | 1.06 |
| A072Q | 1.10 | | N144C | 1.05 |
| A072R | 1.10 | | N144D | 1.15 |
| A072W | 1.06 | | N144E | 1.16 |
| A072Y | 1.30 | | N144I | 1.13 |
| G073C | 1.02 | | N144K | 1.05 |
| G073L | 1.07 | | N145A | 1.07 |
| G073W | 1.03 | | N145C | 1.09 |
| S097F | 1.11 | | N145E | 1.03 |
| S097M | 1.11 | | N145I | 1.20 |
| S097N | 1.23 | | N145K | 1.05 |
| S097P | 1.18 | | N145L | 1.03 |
| S097R | 1.07 | | N145M | 1.07 |
| S097V | 1.12 | | N145Q | 1.14 |
| S097W | 1.09 | | N145R | 1.11 |
| S097Y | 1.18 | | N145V | 1.12 |
| L098H | 1.04 | | N145W | 1.14 |
| L098M | 1.09 | | N145Y | 1.05 |
| A099C | 1.07 | | Y147A | 1.02 |
| A099L | 1.01 | | Y147M | 1.02 |
| A099M | 1.03 | | Y147R | 1.12 |
| A099N | 1.11 | | S152H | 1.08 |
| A099P | 1.08 | | S152M | 1.10 |
| S102A | 1.20 | | N153C | 1.09 |
| S102C | 1.04 | | N153D | 1.20 |
| S102I | 1.04 | | N153K | 1.13 |
| S102L | 1.05 | | N153L | 1.12 |
| S102M | 1.25 | | N153W | 1.07 |
| S102N | 1.19 | | N153Y | 1.13 |
| S102R | 1.21 | | N164A | 1.02 |
| S102V | 1.07 | | N164G | 1.03 |
| S102W | 1.06 | | N182C | 1.12 |
| S102Y | 1.10 | | N182E | 1.13 |
| E110Q | 1.02 | | N182K | 1.07 |
| E110S | 1.07 | | N182P | 1.01 |
| E110W | 1.11 | | N182R | 1.03 |
| L113E | 1.15 | | A204C | 1.04 |
| L113N | 1.08 | | A204D | 1.09 |
| I133K | 1.04 | | A204G | 1.02 |
| I133R | 1.16 | | A204I | 1.06 |
| I133S | 1.08 | | A204M | 1.09 |

| | | | | |
|---|---|---|---|---|
| A204Q | 1.09 | | S230R | 1.84 |
| A204T | 1.05 | | S230T | 1.11 |
| T205A | 1.03 | | S230V | 1.12 |
| T205D | 1.03 | | S230Y | 1.08 |
| T205H | 1.03 | | S231C | 1.13 |
| T205I | 1.05 | | S231D | 1.08 |
| T205K | 1.09 | | S231F | 1.17 |
| T205M | 1.05 | | S231L | 1.29 |
| T205N | 1.09 | | S231M | 1.08 |
| T205P | 1.17 | | S231N | 1.04 |
| T205Q | 1.25 | | S231Q | 1.05 |
| T205S | 1.10 | | S231R | 1.02 |
| T205V | 1.06 | | S231V | 1.07 |
| T205W | 1.05 | | S231Y | 1.07 |
| T205Y | 1.18 | | D236F | 1.14 |
| S214P | 1.08 | | D236G | 1.05 |
| S214T | 1.07 | | D236L | 1.11 |
| V216C | 1.08 | | D236M | 1.07 |
| V216G | 1.05 | | D236N | 1.03 |
| V216H | 1.03 | | D236P | 1.06 |
| V216K | 1.02 | | D236S | 1.03 |
| V216N | 1.13 | | D236T | 1.14 |
| V216Y | 1.09 | | D236V | 1.04 |
| Q219D | 1.05 | | I239M | 1.04 |
| Q219G | 1.06 | | I239Q | 1.08 |
| Q219H | 1.03 | | I239S | 1.11 |
| Q219N | 1.08 | | I239V | 1.52 |
| Q219P | 1.16 | | I239W | 1.02 |
| Q219S | 1.29 | | I239Y | 1.25 |
| W228A | 1.20 | | T241C | 1.07 |
| W228F | 1.22 | | T241E | 1.03 |
| W228G | 1.17 | | T241H | 1.10 |
| W228H | 1.33 | | T241L | 1.04 |
| W228I | 1.18 | | T241M | 1.05 |
| W228L | 1.12 | | T241P | 1.02 |
| W228M | 1.35 | | T241S | 1.08 |
| W228T | 1.19 | | T241V | 1.05 |
| V229E | 1.01 | | N242C | 1.08 |
| V229I | 1.02 | | N242F | 1.06 |
| V229M | 1.03 | | N242M | 1.04 |
| V229N | 1.01 | | N242T | 1.08 |
| V229Q | 1.02 | | N242V | 1.03 |
| S230C | 1.23 | | N242W | 1.05 |
| S230D | 1.13 | | N263H | 1.05 |
| S230E | 1.10 | | N263K | 1.02 |
| S230F | 1.63 | | N263P | 1.40 |
| S230G | 1.77 | | L264A | 1.04 |
| S230H | 1.05 | | L264C | 1.08 |
| S230I | 1.18 | | L264E | 1.16 |
| S230K | 1.04 | | L264F | 1.03 |
| S230L | 1.20 | | L264S | 1.05 |
| S230N | 1.23 | | G265E | 1.10 |
| S230P | 1.13 | | G265H | 1.12 |
| S230Q | 1.20 | | G265I | 1.06 |

| | | | | |
|---|---|---|---|---|
| G265K | 1.03 | | A311S | 1.01 |
| G265R | 1.06 | | A311Y | 1.06 |
| G265T | 1.10 | | V338P | 1.04 |
| A268C | 1.50 | | V338Q | 1.12 |
| A268D | 1.14 | | V338S | 1.14 |
| A268E | 1.18 | | V338Y | 1.05 |
| A268F | 1.15 | | T342N | 1.06 |
| A268G | 1.35 | | T342V | 1.23 |
| A268I | 1.15 | | S344A | 1.05 |
| A268K | 1.23 | | S344T | 1.01 |
| A268L | 1.06 | | T346G | 1.14 |
| A268P | 1.08 | | T346H | 1.07 |
| A268R | 1.14 | | T346M | 1.06 |
| A268T | 1.18 | | T346N | 1.09 |
| A268W | 1.05 | | T346P | 1.07 |
| G269E | 1.01 | | T346Q | 1.04 |
| D276S | 1.01 | | T346Y | 1.06 |
| V284R | 1.06 | | A349L | 1.02 |
| V284T | 1.05 | | V359I | 1.14 |
| V284Y | 1.07 | | V359K | 1.08 |
| S291A | 1.26 | | V359M | 1.09 |
| S291E | 1.09 | | V359N | 1.02 |
| S291F | 1.13 | | V359Q | 1.14 |
| S291H | 1.13 | | V359R | 1.15 |
| S291K | 1.07 | | V359W | 1.04 |
| S291N | 1.04 | | G361H | 1.11 |
| S291P | 1.12 | | G361L | 1.04 |
| S291W | 1.04 | | G361R | 1.04 |
| P300K | 1.10 | | A364M | 1.05 |
| P300P | 1.12 | | A364W | 1.07 |
| P300R | 1.11 | | T375C | 1.12 |
| A301A | 1.01 | | T375D | 1.01 |
| A301E | 1.09 | | T375E | 1.02 |
| A301K | 1.09 | | T375H | 1.05 |
| A301L | 1.05 | | T375V | 1.04 |
| A301P | 1.02 | | T375W | 1.02 |
| A301S | 1.03 | | T375Y | 1.05 |
| A301W | 1.04 | | N379A | 1.05 |
| A303C | 1.06 | | N379C | 1.10 |
| A303D | 1.04 | | N379D | 1.05 |
| A303F | 1.09 | | N379G | 1.07 |
| A303H | 1.05 | | N379I | 1.01 |
| A303I | 1.09 | | N379M | 1.04 |
| A303K | 1.02 | | N379P | 1.06 |
| A303L | 1.05 | | N379S | 1.01 |
| A303N | 1.04 | | S382A | 1.02 |
| A303R | 1.10 | | S382N | 1.02 |
| A303T | 1.11 | | S382P | 1.10 |
| A303V | 1.04 | | S382S | 1.09 |
| A303W | 1.15 | | S382V | 1.10 |
| A303Y | 1.07 | | S382W | 1.10 |
| A311N | 1.04 | | S390A | 1.05 |
| A311P | 1.09 | | S390Y | 1.03 |
| A311Q | 1.19 | | E391A | 1.17 |

42

| | | | | | |
|---|---|---|---|---|---|
| E391E | 1.10 | | L417V | 1.21 |
| E391I | 1.13 | | L417W | 1.05 |
| E391K | 1.18 | | L417Y | 1.10 |
| E391L | 1.18 | | H418E | 1.01 |
| E391M | 1.05 | | H418M | 1.12 |
| E391Q | 1.04 | | T430A | 1.19 |
| E391R | 1.08 | | T430E | 1.15 |
| E391V | 1.05 | | T430F | 1.09 |
| E391W | 1.12 | | T430G | 1.16 |
| E391Y | 1.08 | | T430H | 1.15 |
| A393E | 1.05 | | T430I | 1.06 |
| A393G | 1.14 | | T430K | 1.24 |
| A393H | 1.10 | | T430M | 1.16 |
| A393I | 1.07 | | T430N | 1.07 |
| A393K | 1.09 | | T430Q | 1.15 |
| A393L | 1.12 | | T430R | 1.04 |
| A393M | 1.07 | | T430V | 1.09 |
| A393N | 1.18 | | A431C | 1.04 |
| A393Q | 1.02 | | A431E | 1.08 |
| A393R | 1.09 | | A431H | 1.11 |
| A393S | 1.10 | | A431I | 1.20 |
| A393T | 1.13 | | A431L | 1.21 |
| A393V | 1.16 | | A431M | 1.12 |
| A393W | 1.04 | | A431Q | 1.22 |
| A393Y | 1.03 | | A431R | 1.11 |
| K394A | 1.11 | | A431S | 1.09 |
| K394H | 1.11 | | A431W | 1.04 |
| K394K | 1.08 | | A431Y | 1.13 |
| K394L | 1.01 | | R433A | 1.09 |
| K394M | 1.14 | | R433M | 1.17 |
| K394Q | 1.09 | | R433W | 1.06 |
| K394R | 1.19 | | R433Y | 1.22 |
| K394S | 1.22 | | A442A | 1.13 |
| K394T | 1.08 | | S444K | 1.03 |
| K394V | 1.05 | | S444M | 1.13 |
| K394W | 1.13 | | S444N | 1.04 |
| S410E | 1.01 | | S444P | 1.08 |
| S410H | 1.06 | | S444Q | 1.08 |
| S410N | 1.04 | | S444R | 1.03 |
| L417A | 1.12 | | S444T | 1.15 |
| L417D | 1.19 | | S444V | 1.14 |
| L417E | 1.10 | | S444W | 1.16 |
| L417F | 1.08 | | T448F | 1.02 |
| L417G | 1.19 | | T448G | 1.08 |
| L417I | 1.10 | | T448I | 1.10 |
| L417K | 1.02 | | T448P | 1.08 |
| L417Q | 1.04 | | T448Q | 1.04 |
| L417R | 1.30 | | T448V | 1.04 |
| L417S | 1.05 | | T451K | 1.29 |
| L417T | 1.10 | | | |

Table 4 provides the variants having a performance index of at least 1.0. These included the following sites: 10, 14, 15, 23, 59, 60, 61, 65, 67, 68, 72, 73, 97, 98, 99, 102, 110, 113, 133, 140, 144, 145, 147, 152, 153, 164, 182, 204, 205, 214, 216, 219, 228, 229, 230, 231, 236, 239, 241, 242, 263, 264, 265, 268, 269, 276, 284, 291, 300, 301, 303, 1 I, 338, 342, 344, 346, 349, 359, 361, 364, 375, 379, 382, 390, 391, 393, 394, 410, 417, 418, 430, 431, 433, 442, 444, 448, and 451. The sites showing the highest specific activity (having a performance index above 1.2), included: 61, 67, 72, 97, 102, 133, 145, 153, 205, 219, 228, 230, 231, 239, 263, 268, 291, 311, 342, 394, 430, 431 and 451.

**Example 7: Combined specific activity and Thermostability variants**

Table 5 shows the variants that had a performance index of 1.0 or better as compared to the parent for both properties: specific activity and thermostability. These included the following sites: 10, 15, 59, 61, 68, 72, 73, 97, 99, 102, 133, 140, 153, 182, 204, 205, 214, 228, 229, 230, 231, 236, 241, 242, 264, 265, 268, 275, 284, 291, 300, 301, 303, 311, 338, 344, 346, 359, 361, 364, 375, 370, 382, 391, 393, 394, 410, 417, 430, 431, 433, 444, 448, and 451. The sites showing the highest specific activity and thermostability combined included: 228, 230, 231, 268, 291, 417, 433, and 451.

## Table 5: Combined variants

| Variant | PI of Specific Activity | PI of Thermal Stability |
|---|---|---|
| T010F | 1.06 | 1.11 |
| T010G | 1.12 | 1.13 |
| T010M | 1.05 | 1.12 |
| T010R | 1.08 | 1.06 |
| T010S | 1.09 | 1.24 |
| N015N | 1.06 | 1.06 |
| F059A | 1.17 | 1.03 |
| F059G | 1.05 | 1.07 |
| N061V | 1.11 | 1.06 |
| E068I | 1.06 | 1.19 |
| E068M | 1.10 | 1.29 |
| E068W | 1.03 | 1.09 |
| A072E | 1.11 | 1.07 |
| A072Q | 1.10 | 1.02 |
| G073W | 1.03 | 1.36 |
| S097V | 1.12 | 1.03 |
| A099C | 1.07 | 1.07 |
| A099L | 1.01 | 1.09 |
| S102A | 1.20 | 1.02 |
| K140Q | 1.08 | 1.06 |
| K140S | 1.13 | 1.05 |
| K140W | 1.07 | 1.04 |
| N153D | 1.20 | 1.06 |
| N153L | 1.12 | 1.06 |
| N153W | 1.07 | 1.19 |
| N182R | 1.03 | 1.02 |
| A204D | 1.09 | 1.02 |
| A204M | 1.09 | 1.08 |
| T205D | 1.03 | 1.06 |
| T205N | 1.09 | 1.09 |
| T205P | 1.17 | 1.17 |
| T205S | 1.10 | 1.04 |
| T205V | 1.06 | 1.06 |
| T205Y | 1.18 | 1.07 |
| S214P | 1.08 | 1.04 |
| S214T | 1.07 | 1.06 |
| W228A | 1.20 | 1.01 |
| W228F | 1.22 | 1.12 |
| W228G | 1.17 | 1.06 |
| W228H | 1.33 | 1.05 |
| W228I | 1.18 | 1.06 |
| W228L | 1.12 | 1.14 |
| W228M | 1.35 | 1.04 |
| W228T | 1.19 | 1.06 |
| V229E | 1.01 | 1.16 |
| V229I | 1.02 | 1.21 |
| V229M | 1.03 | 1.11 |
| V229N | 1.01 | 1.07 |
| V229Q | 1.02 | 1.10 |
| S230C | 1.23 | 1.09 |
| S230D | 1.13 | 1.08 |
| S230E | 1.10 | 1.11 |
| S230F | 1.63 | 1.08 |
| S230G | 1.77 | 1.09 |
| S230H | 1.05 | 1.03 |

| | | | | | | |
|---|---|---|---|---|---|---|
| S230K | 1.04 | 1.02 | | S291F | 1.13 | 1.24 |
| S230L | 1.20 | 1.02 | | S291H | 1.13 | 1.25 |
| S230N | 1.23 | 1.16 | | S291K | 1.07 | 1.13 |
| S230P | 1.13 | 1.12 | | S291N | 1.04 | 1.08 |
| S230Q | 1.20 | 1.20 | | S291P | 1.12 | 1.11 |
| S230R | 1.84 | 1.11 | | S291W | 1.04 | 1.15 |
| S230T | 1.11 | 1.07 | | P300R | 1.11 | 1.02 |
| S230V | 1.12 | 1.11 | | A301K | 1.09 | 1.11 |
| S230Y | 1.08 | 1.05 | | A301L | 1.05 | 1.07 |
| S231C | 1.13 | 1.07 | | A301P | 1.02 | 1.22 |
| S231D | 1.08 | 1.18 | | A301S | 1.03 | 1.12 |
| S231F | 1.17 | 1.14 | | A301W | 1.04 | 1.14 |
| S231L | 1.29 | 1.15 | | A303I | 1.09 | 1.02 |
| S231M | 1.08 | 1.09 | | A303L | 1.05 | 1.03 |
| S231N | 1.04 | 1.13 | | A311S | 1.01 | 1.01 |
| S231Q | 1.05 | 1.18 | | A311Y | 1.06 | 1.02 |
| S231R | 1.02 | 1.13 | | V338P | 1.04 | 1.18 |
| S231V | 1.07 | 1.21 | | V338Q | 1.12 | 1.20 |
| S231Y | 1.07 | 1.10 | | V338S | 1.14 | 1.19 |
| D236F | 1.14 | 1.11 | | V338Y | 1.05 | 1.10 |
| D236G | 1.05 | 1.07 | | S344A | 1.05 | 1.05 |
| D236L | 1.11 | 1.08 | | S344T | 1.01 | 1.19 |
| D236M | 1.07 | 1.15 | | T346M | 1.06 | 1.07 |
| D236N | 1.03 | 1.15 | | T346N | 1.09 | 1.15 |
| D236P | 1.06 | 1.06 | | T346P | 1.07 | 1.13 |
| D236T | 1.14 | 1.16 | | T346Q | 1.04 | 1.12 |
| D236V | 1.04 | 1.18 | | T346Y | 1.06 | 1.07 |
| T241V | 1.05 | 1.05 | | A349L | 1.02 | 1.16 |
| N242F | 1.06 | 1.03 | | V359Q | 1.14 | 1.02 |
| L264A | 1.04 | 1.03 | | V359R | 1.15 | 1.13 |
| G265E | 1.10 | 1.03 | | G361H | 1.11 | 1.11 |
| G265H | 1.12 | 1.09 | | G361L | 1.04 | 1.22 |
| G265I | 1.06 | 1.09 | | G361R | 1.04 | 1.19 |
| G265K | 1.03 | 1.05 | | A364M | 1.05 | 1.21 |
| G265R | 1.06 | 1.12 | | A364W | 1.07 | 1.23 |
| G265T | 1.10 | 1.17 | | T375E | 1.02 | 1.03 |
| A268C | 1.50 | 1.16 | | T375H | 1.05 | 1.13 |
| A268D | 1.14 | 1.20 | | T375V | 1.04 | 1.16 |
| A268E | 1.18 | 1.10 | | T375W | 1.02 | 1.11 |
| A268F | 1.15 | 1.07 | | T375Y | 1.05 | 1.17 |
| A268G | 1.35 | 1.11 | | N379A | 1.05 | 1.11 |
| A268I | 1.15 | 1.12 | | N379C | 1.10 | 1.03 |
| A268K | 1.23 | 1.11 | | N379D | 1.05 | 1.06 |
| A268L | 1.06 | 1.11 | | N379G | 1.07 | 1.09 |
| A268P | 1.08 | 1.13 | | N379I | 1.01 | 1.04 |
| A268R | 1.14 | 1.15 | | N379M | 1.04 | 1.08 |
| A268T | 1.18 | 1.14 | | N379P | 1.06 | 1.17 |
| A268W | 1.05 | 1.05 | | S382A | 1.02 | 1.06 |
| D276S | 1.01 | 1.02 | | S382N | 1.02 | 1.07 |
| V284R | 1.06 | 1.09 | | S382P | 1.10 | 1.10 |
| V284T | 1.05 | 1.08 | | E391L | 1.18 | 1.04 |
| V284Y | 1.07 | 1.12 | | E391R | 1.08 | 1.02 |
| S291A | 1.26 | 1.21 | | E391W | 1.12 | 1.04 |
| S291E | 1.09 | 1.02 | | E391Y | 1.08 | 1.02 |

| | | | | | | |
|---|---|---|---|---|---|---|
| A393E | 1.05 | 1.03 | | L417R | 1.30 | 1.20 |
| A393G | 1.14 | 1.12 | | L417V | 1.21 | 1.07 |
| A393H | 1.10 | 1.05 | | L417Y | 1.10 | 1.01 |
| A393I | 1.07 | 1.06 | | T430A | 1.19 | 1.05 |
| A393K | 1.09 | 1.05 | | T430E | 1.15 | 1.02 |
| A393L | 1.12 | 1.17 | | T430F | 1.09 | 1.06 |
| A393M | 1.07 | 1.07 | | T430H | 1.15 | 1.10 |
| A393N | 1.18 | 1.11 | | T430I | 1.06 | 1.04 |
| A393Q | 1.02 | 1.02 | | T430K | 1.24 | 1.08 |
| A393R | 1.09 | 1.03 | | T430M | 1.16 | 1.17 |
| A393S | 1.10 | 1.13 | | T430N | 1.07 | 1.13 |
| A393T | 1.13 | 1.08 | | T430Q | 1.15 | 1.05 |
| A393V | 1.16 | 1.09 | | T430R | 1.04 | 1.13 |
| A393W | 1.04 | 1.10 | | T430V | 1.09 | 1.05 |
| A393Y | 1.03 | 1.12 | | A431I | 1.20 | 1.03 |
| K394A | 1.11 | 1.08 | | A431R | 1.11 | 1.08 |
| K394H | 1.11 | 1.08 | | R433A | 1.09 | 1.13 |
| K394L | 1.01 | 1.08 | | R433M | 1.17 | 1.10 |
| K394M | 1.14 | 1.06 | | R433W | 1.06 | 1.16 |
| K394Q | 1.09 | 1.07 | | R433Y | 1.22 | 1.18 |
| K394R | 1.19 | 1.06 | | S444K | 1.03 | 1.07 |
| K394T | 1.08 | 1.03 | | S444M | 1.13 | 1.05 |
| K394V | 1.05 | 1.02 | | S444Q | 1.08 | 1.04 |
| S410E | 1.01 | 1.05 | | T448F | 1.02 | 1.12 |
| S410H | 1.06 | 1.11 | | T448I | 1.10 | 1.12 |
| S410N | 1.04 | 1.10 | | T448Q | 1.04 | 1.17 |
| L417I | 1.10 | 1.04 | | T448V | 1.04 | 1.13 |
| L417K | 1.02 | 1.20 | | S451K | 1.29 | 1.08 |
| L417Q | 1.04 | 1.04 | | | | |

### Reference **Example 8: Crystal structure of TrGA**

The complete three dimensional structure of *Trichoderma reesei* (*Hypocrea jecorina*) glucoamylase (TrGA) was determined at 1.9 Å resolution. Table 6 shows the coordinates for the *Trichoderma* glucoamylase crystal structure. TrGA was crystallized in an intact form containing 599 residues and all post-translational modifications that would normally occur in the natural host. The crystal structure was produced and analyzed as follows:

Protein expression and purification - The gene encoding *H. jecorina* GA was cloned and expressed according to the protocols described in patent application publication no. US 2006/0094080 A1, by Dunn-Coleman et al. (May 4, 2006).

The TrGA protein material used for all crystallization experiments was initially purified in one step by anion exchange chromatography as follows: concentrated culture supernatants of expressed TrGA, consisting of 180 mg/ml total protein, were prepared by diluting sample 1:10 in a 25 mM Tris-HCl, pH 8.0 buffer. A HIPREP 16/10 Q Sepharose FF column (GE Helthcare) was employed for the anion exchange purification. The HIPREP column was equilibrated with 4 column volumes (CV) starting buffer (25 mM Tris-HCl, pH 8.0) followed by application of 10 ml of the diluted protein sample. An 8 CV linear gradient of 0 to 140 mM NaCl in the running buffer (25 mM Tris-HCl, pH 8.0) was applied to elute bound protein. Bound TrGA eluted from the HIPREP Q sepharose column at a salt concentration of approximately 80 mM NaCl. Fractions containing pure TrGA protein were pooled and concentrated to 50 mg/ml using a 25 ml VIVASPIN centrifugal concentration tube (Viva Science) with a molecular weight cutoff (MWCO) of 10 kD. Purified and concentrated TrGA material was buffer exchanged using a DG-10 desalting column (Bio-Rad) equilibrated with 50 mM sodium acetate buffer, pH 4.3. Protein concentrations were determined by measuring the absorbance at 280 nm. The initially purified and concentrated TrGA protein stock was thereafter stored at -20°C.

Two additional purification steps, one additional anion exchange purification, and a size exclusion purification, were introduced to enhance the TrGA protein material's propensity to form crystals. These two additional purification steps were performed as follows: In the first anion exchange purification step a 10 ml MONOQ column (GE Healthcare) was employed. A sample of 1 ml of the initially purified and frozen TrGA material (50 mg protein) was thawed and the buffer was changed to 20 mM Tris-HCl, pH 8.0, by repeated dilution of the sample to 6 ml in the new buffer, followed by a concentration of the sample again to 0.5 ml using a 6 ml 5 kD MWCO concentration tube. The TrGA sample was diluted after the last concentration step in distilled water until a conductivity of the protein sample was reached that corresponded to the conductivity of the starting buffer of the anion purification, i.e. 25 mM Tris-HCl, pH 8.0. The MONOQ column was first equilibrated with 4 column volumes (CV) starting buffer, followed by application of the diluted protein sample to the column. Bound protein was eluted from the MONOQ column by two different gradients. In the first a 4 CV linear pH gradient was applied where the pH of the starting buffer was decreased from 8.0 to 6.0. In the second gradient an 8 CV long salt gradient was applied in which the salt concentration was increased from 0 to 350 mM NaCl in the running buffer (25 mM Tris-HCl, pH 6.0). Bound TrGA was found to elute from the column during the second salt gradient at an approximate NaCl concentration of 150 mM. Fractions containing TrGA were pooled and concentrated to 2 ml using a 6 ml 5 kD MWCO VIVASPIN concentration tube. The concentrated TrGA sample was thereafter applied to a SUPERDEX 200 16/60 size exclusion column (GE Healthcare) equilibrated with 4 CV of 20 mM Tris-Cl, pH 8.0, and 50 mM NaCl, which also was used as running buffer. Fractions from the main elution peak after the size exclusion purification were pooled and concentrated to an approximate protein concentration of 7.5 mg/ml using a 6 ml 5 kD MWCO VIVASPIN concentration tube.

Protein crystallization - The protein sample that was used to find the initial TrGA crystallization conditions was a sample of the TrGA material that was purified once by anion exchange purification and thereafter stored at -20°C. The TrGA protein sample was thawed and diluted with 50 mM sodium acetate buffer, pH 4.3, to approximately 12 mg/ml, prior to the initial crystallization experiments. TrGA crystals were found to grow in solution consisting of 25% PEG 3350, 0.20M ammonium acetate, 0.10M Bis-Tris pH 5.5 (reservoir solution), using the vapor-diffusion method with hanging drops (McPherson 1982), at 20° C. Crystallization drops were prepared by mixing equal amounts of protein solution (12 mg/ml) and reservoir solution to a final volume of 10 $\mu$l. The TrGA crystals were found to belong to the orthorhombic space group P212121 with approximate cell dimensions: $a$ = 52.2 Å, $b$ = 99.2 Å, $c$ = 121.2 Å, and have a calculated $V_m$ of 2.3 (Matthews 1968) with one molecule in the asymmetric unit.

X-ray data collection - The two orthorhombic TrGA datasets were collected from single crystals mounted in sealed capillary tubes, at room temperature. The initial lo-resolution orthorhombic TrGA X-ray dataset used to solve the structure by molecular replacement methods (MR), was collected on a home X-ray source, an MSC/Rigaku (Molecular Structures Corp., The Woodlands, Texas) Raxis IV++ image plate detector with focusing mirrors using Cu K$\alpha$ radiation from a Rigaku RU200 rotating anode generator. This dataset was processed, scaled, and averaged using the d*trek software provided by MSC/Rigaku. The orthorhombic X-ray dataset was used to solve the TrGA structure by molecular replacement (MR) and the high resolution orthorhombic dataset was used for the final orthorhombic space group TrGA structure model. The C centered monoclinic dataset was collected from a single frozen TrGA crystal at 100K, equilibrated in a cryo-protective agent comprised of 25% PEG 3350, 15% Glycerol 50 mM CaCl$_2$ and 0.1 M Bis-Tris pH 5.5 as cryoprotectant, mounted in rayon-fiber loops, and plunge frozen in liquid nitrogen prior to transportation to the synchrotron. The high-resolution orthorhombic (1.9 Å) data set and the C centric monoclinic dataset (1.8 Å) were both collected at a synchrotron source, beam line 911:5 at MAX LAB in Lund, Sweden. Both datasets that were collected at a synchrotron source were processed with MOSFLM, and scaled with program SCALA included in the CCP4 program package (Collaborative Computational Project Number 4 1994). All subsequent data processing was performed using the CCP4 program package (Collaborative Computational Project Number 4 1994), unless otherwise stated. A set of 5% of the reflections from each data set was set aside and used for monitoring the R-free (Brunger, A (1992) Nature, 355: 472-475).

Structure solution - The TrGA structure was initially solved by MR with the automatic replacement program MOLREP (Collaborative Computational Project Number 4 1994), included in the CCP4 program package, using the initial lo-resolution orthorhombic dataset, and using the coordinates of *Aspergillus awamori* GA (AaGA) variant X 100 (pdb entry 1GLM (Aleshin, et al (1994) J. Mol. Biol. 238:575-591) as search model. The *A. awamori* GA search model was edited to remove all glycosylation moieties attached to the protein molecule as N- and O- glycosylations, and all solvent molecules before carrying out the MR experiments. All reflections between 36.8 and 2.8 Å resolution, from the initial low resolution TrGA dataset, was used for the MR solution. The MR program found a single rotation function solution, with a maxima of 11.1 $\sigma$ above background, the next highest maxima was 3.8$\sigma$ above the background. The translation function solution gave an R-factor of 48.7% and had a contrast factor of 17.4. The MR solution was refined for 10 cycles of restrained least squares refinement using the program Refmac 5.0 (Murshudov, et al. (1997) Acta Crystallogr., D53:240-255). This lowered the crystallographic R-factor to 31.1% while the R-free value dropped from 42.2% to 41.1%.

Model fitting and refinement - The refined MR solution model was used to calculate an initial density map from the lo-resolution orthorhombic TrGA dataset. Electron density for a disulfide bridge between residues 19 and 26 of TrGA, a disulfide bridge not present in the *A. awamori* variant X100 structure model, could readily be identified in this electron density map. This was taken as an indication that the electron density map was of sufficient quality to be used to build a structure model of TrGA from its amino acid sequence. The initial TrGA structure model, based on the lo-resolution dataset, was refined with alternating cycles of model building using Coot (Emsley et al, (2004) Acta Crystallogr D Biol Crystallogr 60:2126-32), and maximum likelihood refinement using Refmac 5.0.

The resolution of the initial TrGA structure model was extended to the resolution of the high-resolution orthorhombic dataset (1.9Å) by refining the initial TrGA structure model against the high-resolution dataset for 10 cycles of restrained refinement using the program Refmac 5.0. Most water molecules in the structure models were located automatically by using the water picking protocols in the refinement programs, and then manually selected or discarded by inspection by eye. All structural comparisons were made with either Coot (Emsley et al. (2004) *supra*) or O (Jones et al. (1991) Acta Crystallogr., A47: 110-119), and figures were prepared with PyMOL (DeLano W.L. (2002) The PyMOL Molecular Graphics System. Palo Alto, CA, USA; DeLano Scientific).

From these results, it can be seen that the TrGA catalytic core segment followed the same $(\alpha/\alpha)_6$-barrel topology described by Aleshin et al. (1992, *supra*) for the AaGA, consisting of a double barrel of alpha helices with the C-terminal of the outer helix leading into the N-terminus of an inner helix. It was possible to identify key differences in the electron density such as the disulfide bridge between residues 19 and 26 and ar insertion (residues 257-260) relative to AaGA. The segment comprising 80-100 also underwent extensive model rebuilding. One major glycosylation site was identified at Asn 171, which had up to four glycoside moieties attached. A similar glycosylation site was identified in AaGA. Additionally, the catalytic core containing three cis-peptides between residues 22-23, 44-45 and 122-123 were conserved between TrGA and AaGA. Overall there was an rms variation of 0.535 Å between 409 out of 453 C$\alpha$ atoms when comparing the coordinates of the catalytic cores of TrGA and AaGA.

Reference **Example 9: Homology between TrGA and *Aspergillus awamori* GA**

The crystal structure of the TrGA identified in Example 8, was superposed on the previously identified crystal structure of the *Aspergillus awamori* GA (AaGA). The AaGA crystal structure was obtained from the protein database (PDB) and the form of AaGA that was crystallized was the form containing only a catalytic domain. The structure of the *Trichoderma reesei* glucoamylase with all three regions intact was determined to 1.8 Angstrom resolution herein (see Table 6 and Example 8). Using the coordinates (see Table 6) the structure was aligned with the coordinates of the catalytic domain from *Aspergillus awamori* strain X100 that was determined previously (Aleshin, A.E., Hoffman, C., Firsov, L.M., and Honzatko, R.B. (1994) J Mol Biol 238: 575-591 and the PDB). As seen in Figures 6 and 7 the structure of the catalytic domain overlapped very closely and allowed the identification of equivalent residues based on this structural superposition.

Based on this analysis, sites were identified that could be mutated in TrGA and result in increased stability and/or specific activity. These sites include 108, 124, 175 and 316 at the active site. Also identified were specific pairwise variants Y47W/Y315F and Y47F/Y315W. Other sites identified were I43, D44, P45, D46, R122, R125, V181, E242, Y310, D313, V314, N317, R408, and N409. Because of the high structural homology it is expected that beneficial variants found at sites in *Trichoderma reesei* GA would have similar consequences in *Aspergillus awamori* and other homologous glucoamylases.

**Claims**

1. A glucoamylase variant, which is a variant of a parent glucoamylase, said parent glucoamylase having an amino acid sequence having at least 97% sequence identity with the amino acid sequence:

SVDDFISTETPIALNNLLCNVGPDGCRAFGTSAGAVIASPSTIDPDYYYMWTRDSAL
VFKNLIDRFTETYDAGLQRRIEQYITAQVTLQGLSNPSGSLADGSGLGEPKFELTLKP
FTGNWGRPQRDGPALRAIALIGYSKWLINNNYQSTVSNVIWPIVRNDLNYVAQYWN
QTGFDLWEEVNGSSFFTVANQHRALVEGATLAATLGQSGSAYSSVAPQVLCFLQR
FWVSSGGYVDSNINTNEGRTGKDVNSVLTSIHTFDPNLGCDAGTFQPCSDKALSNL
KVVVDSFRSIYGVNKGIPAGAAVAIGRYAEDVYYNGNPWYLATFAAAEQLYDAIYVW
KKTGSITVTATSLAFFQELVPGVTAGTYSSSSSTFTNIINAVSTYADGFLSEAAKYVP
ADGSLAEQFDRNSGTPLSALHLTWSYASFLTATARRAGIVPPSWANSSASTIPSTCS
GASVVGSYSRPTATSFPPSQTPKPGVPSGTPYTPLPCATPTSVAVTFHELVSTQFG
QTVKVAGNAAALGNWSTSAAVALDAVNYADNHPLWIGTVNLEAGDVVEYKYINVG
QDGSVTWESDPNHTYTVPAVACVTQVVKEDTWQS (SEQ ID NO: 2),

said variant only having one, two, three or four substitutions as compared to said parent, wherein said substitutions comprise the substitution L417V, L417A, L417D, L417E, L417F, L417G, L417I, L417K, L417Q, L417R, L417S, L417T, L417W or L417Y in SEQ ID NO: 2 or an equivalent position in said parent glucoamylase as determined by sequence alignment.

2. The glucoamylase variant of claim 1, wherein said parent glucoamylase has at least 98% sequence identity with the sequence of SEQ ID NO: 2.

3. The glucoamylase variant of claim 1, wherein said variant has only two, three or four substitutions as compared to said parent, the further substitutions being selected from substitutions at a position corresponding to position 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182,204, 205, 214, 216, 219, 228, 229, 230, 231, 236, 239, 240, 241, 242, 244, 263. 264, 265, 268, 269, 276, 284, 291, 300, 301, 303, 310, 311, 313, 316, 338, 342, 344, 346, 349, 359, 361, 364, 379, 382, 390, 391, 393, 394, 408, 410, 415, 418, 430, 431, 433, 436, 442, 443, 444, 448 and 451 of SEQ ID NO: 2 or an equivalent position in said parent glucoamylase as determined by sequence alignment.

4. The glucoamylase variant of claim 1, wherein the variant has increased specific activity compared to the glucoamylase comprising the sequence of SEQ ID NO: 2.

5. The glucoamylase variant of claim 1, wherein the variant has increased thermostability compared to the glucoamylase comprising the sequence of SEQ ID NO: 2.

6. The glucoamylase variant of claim 1, wherein said variant has increased specific activity and increased thermostability compared to the glucoamylase comprising SEQ ID NO: 2.

7. The glucoamylase variant of claim 1, wherein the parent glucoamylase is obtained from a Trichoderma spp.

8. A polynucleotide encoding the variant of claim 1.

9. A host cell comprising a polynucleotide of claim 8.

10. An enzyme composition comprising the glucoamylase variant of claim 1.

11. The enzyme composition of claim 10, wherein the composition is suitable for use in a starch conversion process, an animal feed composition, or a starch containing substrate fermentation process.

12. The enzyme composition of claim 10, further comprising an alpha amylase.

13. A method of producing a variant glucoamylase in a host cell comprising:

transforming a host cell with a DNA construct comprising a polynucleotide of claim 8; and
culturing the host cell under conditions suitable for the expression and production of said glucoamylase variant and producing said variant.

14. The method according to claim 13, further comprising recovering the glucoamylase variant from said culture.

**Patentansprüche**

1. Glucoamylasevariante, die eine Variante einer Stamm-Glucoamylase ist, wobei die Stamm-Glucoamylase eine Aminosäuresequenz aufweist, die mindestens 97 % Sequenzidentität mit der folgenden Aminosäuresequenz aufweist:

SVDDFISTETPIALNNLLCNVGPDGCRAFGTSAGAVIASPSTIDPDYYYMWTRDSAL
VFKNLIDRFTETYDAGLQRRIEQYITAQVTLQGLSNPSGSLADGSGLGEPKFELTLKP
FTGNWGRPQRDGPALRAIALIGYSKWLINNNYQSTVSNVIWPIVRNDLNYVAQYWN
QTGFDLWEEVNGSSFFTVANQHRALVEGATLAATLGQSGSAYSSVAPQVLCFLQR
FWVSSGGYVDSNINTNEGRTGKDVNSVLTSIHTFDPNLGCDAGTFQPCSDKALSNL
KVVVDSFRSIYGVNKGIPAGAAVAIGRYAEDVYYNGNPWYLATFAAAEQLYDAIYVW
KKTGSITVTATSLAFFQELVPGVTAGTYSSSSSTFTNIINAVSTYADGFLSEAAKYVP
ADGSLAEQFDRNSGTPLSALHLTWSYASFLTATARRAGIVPPSWANSSASTIPSTCS
GASVVGSYSRPTATSFPPSQTPKPGVPSGTPYTPLPCATPTSVAVTFHELVSTQFG
QTVKVAGNAAALGNWSTSAAVALDAVNYADNHPLWIGTVNLEAGDVVEYKYINVG
QDGSVTWESDPNHTYTVPAVACVTQVVKEDTWQS (SEQ ID NO: 2),

wobei die Variante im Vergleich mit dem Stamm nur eine, zwei, drei oder vier Substitutionen aufweist, wobei die Substitutionen die Substitution L417V, L417A, L417D, L417E, L417F, L417G, L417I, L417K, L417Q, L417R, L417S, L417T, L417W oder L417Y in SEQ ID NO: 2 oder an einer äquivalenten Position in der Stamm-Glucoamylase, wie durch Sequenz-Alignment bestimmt, aufweist.

2. Glucoamylasevariante nach Anspruch 1, wobei die Stamm-Glucoamylase mindestens 98 % Sequenzidentität mit der Sequenz von SEQ ID NO: 2 aufweist.

3. Glucoamylasevariante nach Anspruch 1, wobei die Variante nur zwei, drei oder vier Substitutionen im Vergleich mit dem Stamm aufweist, wobei die weiteren Substitutionen aus Substitutionen an einer Position korrespondierend mit Position 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182, 204, 205, 214, 216, 219, 228, 229, 230, 231, 236, 239, 240, 241, 242, 244, 263, 264, 265, 268, 269, 276, 284, 291, 300, 301, 303, 310, 311, 313, 316, 338, 342, 344, 346, 349, 359, 361, 364, 379, 382, 390, 391, 393, 394, 408, 410, 415, 418, 430, 431, 433, 436, 442, 443, 444, 448 und 451 von SEQ ID NO: 2 oder an einer äquivalenten Position in der Stamm-Glucoamylase, wie durch Sequenz-Alignment bestimmt, ausgewählt sind.

4. Glucoamylasevariante nach Anspruch 1, wobei die Variante im Vergleich mit der Glucoamylase, umfassend die Sequenz von SEQ ID NO: 2, erhöhte spezifische Aktivität aufweist.

5. Glucoamylasevariante nach Anspruch 1, wobei die Variante im Vergleich mit der Glucoamylase, umfassend die Sequenz von SEQ ID NO: 2, erhöhte Thermostabiltität aufweist.

6. Glucoamylasevariante nach Anspruch 1, wobei die Variante im Vergleich mit der Glucoamylase, umfassend die Sequenz von SEQ ID NO: 2, erhöhte spezifische Aktivität und erhöhte Thermostabiltität aufweist.

7. Glucoamylasevariante nach Anspruch 1, wobei die Stamm-Glucoamylase aus einem Trichoderma spp. erhalten wird.

8. Polynucleotid, das die Variante nach Anspruch 1 codiert.

9. Wirtszelle, umfassend ein Polynucleotid nach Anspruch 8.

10. Enzym-Zusammensetzung, umfassend die Glucoamylasevariante nach Anspruch 1.

11. Enzym-Zusammensetzung nach Anspruch 10, wobei die Zusammensetzung zur Verwendung in einem Stärkeumwandlungsprozess, einer Tierfutterzusammensetzung oder einem Stärke enthaltenden Substratfermentierungsprozess geeignet ist.

12. Enzym-Zusammensetzung nach Anspruch 10, ferner umfassend eine Alpha-Amylase.

13. Verfahren zum Produzieren einer varianten Glucoamylase in einer Wirtszelle, umfassend:

Transformieren einer Wirtszelle mit einem DNA-Konstrukt, umfassend ein Polynucleotid nach Anspruch 8; und Kultivieren der Wirtszelle unter Bedingungen, die für die Expression und Produktion der Glucoamylasevariante geeignet sind, und Produzieren der Variante.

14. Verfahren nach Anspruch 13, ferner umfassend die Glucoamylasevariante aus der Kultur zurückzugewinnen.


**Revendications**

1. Variant de glucoamylase, qui est un variant d'une glucoamylase parente, ladite glucoamylase parente possédant une séquence d'acides aminés possédant au moins 97% d'identité de séquence avec la séquence d'acides aminés:

SVDDFISTETPIALNNLLCNVGPDGCRAFGTSAGAVIASPSTIDPDYYYMWTRDSAL
VFKNLIDRFTETYDAGLQRRIEQYITAQVTLQGLSNPSGSLADGSGLGEPKFELTLKP
FTGNWGRPQRDGPALRAIALIGYSKWLINNNYQSTVSNVIWPIVRNDLNYVAQYWN
QTGFDLWEEVNGSSFFTVANQHRALVEGATLAATLGQSGSAYSSVAPQVLCFLQR
FWVSSGGYVDSNINTNEGRTGKDVNSVLTSIHTFDPNLGCDAGTFQPCSDKALSNL
KVVVDSFRSIYGVNKGIPAGAAVAIGRYAEDVYYNGNPWYLATFAAAEQLYDAIYVW
KKTGSITVTATSLAFFQELVPGVTAGTYSSSSSTFTNIINAVSTYADGFLSEAAKYVP
ADGSLAEQFDRNSGTPLSALHLTWSYASFLTATARRAGIVPPSWANSSASTIPSTCS
GASVVGSYSRPTATSFPPSQTPKPGVPSGTPYTPLPCATPTSVAVTFHELVSTQFG
QTVKVAGNAAALGNWSTSAAVALDAVNYADNHPLWIGTVNLEAGDVVEYKYINVG
QDGSVTWESDPNHTYTVPAVACVTQVVKEDTWQS (SEQ ID NO: 2),

ledit variant possédant seulement une, deux, trois ou quatre substitutions lorsqu'il est comparé audit parent, dans lequel lesdites substitutions comprennent la substitution L417V, L417A, L417D, L417E, L417F, L417G, L417I, L417K, L417Q, L417R, L417S, L417T, L417W ou L417Y dans la SEQ ID NO: 2 ou à une position équivalente dans ladite glucoamylase parente tel que déterminé par un alignement de séquences.

2. Variant de glucoamylase selon la revendication 1, dans lequel ladite glucoamylase parente possède au moins 98% d'identité de séquence avec la séquence de la SEQ ID NO: 2.

3. Variant de glucoamylase selon la revendication 1, où ledit variant possède seulement deux, trois ou quatre substitutions lorsqu'il est comparé audit parent, les substitutions supplémentaires étant choisies parmi des substitutions à une position correspondant à la position 10, 14, 15, 23, 42, 45, 46, 59, 60, 61, 67, 68, 72, 73, 97, 98, 99, 102, 108, 110, 113, 114, 122, 124, 125, 133, 140, 144, 145, 147, 152, 153, 164, 175, 182, 204, 205, 214, 216, 219, 228, 229, 230, 231, 236, 239, 240, 241, 242, 244, 263, 264, 265, 268, 269, 276, 284, 291, 300, 301, 303, 310, 311, 313, 316, 338, 342, 344, 346, 349, 359, 361, 364, 379, 382, 390, 391, 393, 394, 408, 410, 415, 418, 430, 431, 433, 436, 442, 443, 444, 448 et 451 de la SEQ ID NO: 2 ou à une position équivalente dans ladite glucoamylase parente tel que déterminé par un alignement de séquences.

4. Variant de glucoamylase selon la revendication 1, où le variant possède une activité spécifique accrue comparé à la glucoamylase comprenant la séquence de la SEQ ID NO: 2.

5. Variant de glucoamylase selon la revendication 1, où le variant possède une thermostabilité accrue comparé à la glucoamylase comprenant la séquence de la SEQ ID NO: 2.

6. Variant de glucoamylase selon la revendication 1, où ledit variant possède une activité spécifique accrue et une thermostabilité accrue comparé à la glucoamylase comprenant la SEQ ID NO: 2.

7. Variant de glucoamylase selon la revendication 1, dans lequel la glucoamylase parente est obtenue à partir d'une espèce Trichoderma.

8. Polynucléotide codant pour le variant selon la revendication 1.

9. Cellule hôte comprenant un polynucléotide selon la revendication 8.

10. Composition d'enzyme comprenant le variant de glucoamylase selon la revendication 1.

11. Composition d'enzyme selon la revendication 10, où la composition est appropriée pour une utilisation dans un procédé de conversion d'amidon, une formulation d'alimentation animale ou un procédé de fermentation d'un substrat contenant de l'amidon.

12. Composition d'enzyme selon la revendication 10, comprenant en outre une alpha-amylase.

13. Méthode pour la production d'un variant de glucoamylase dans une cellule hôte comprenant:

la transformation d'une cellule hôte avec une construction d'ADN comprenant un polynucléotide selon la revendication 8; et
la culture de la cellule hôte dans des conditions appropriées pour l'expression et la production dudit variant de glucoamylase et la production dudit variant.

14. Méthode selon la revendication 13, comprenant en outre la récupération du variant de glucoamylase à partir de ladite culture.

FIG. 1A

TrGA parent protein (632 amino acids) (SEQ ID NO: 1)

```
  1 MHVLSTAVLL GSVAVQKVLG RPGSSGLSDV TKRSVDDFIS TETPIALNNL
 51 LCNVGPDGCR AFGTSAGAVI ASPSTIDPDY YYMWTRDSAL VFKNLIDRFT
101 ETYDAGLQRR IEQYITAQVT LQGLSNPSGS LADGSGLGEP KFELTLKPFT
151 GNWGRPQRDG PALRAIALIG YSKWLINNNY QSTVSNVIWP IVRNDLNYVA
201 QYWNQTGFDL WEEVNGSSFF TVANQHRALV EGATLAATLG QSGSAYSSVA
251 PQVLCFLQRF WVSSGGYVDS NINTNEGRTG KDVNSVLTSI HTFDPNLGCD
301 AGTFQPCSDK ALSNLKVVVD SFRSIYGVNK GIPAGAAVAI GRYAEDVYYN
351 GNPWYLATFA AAEQLYDAIY VWKKTGSITV TATSLAFFQE LVPGVTAGTY
401 SSSSSTFTNI INAVSTYADG FLSEAAKYVP ADGSLAEQFD RNSGTPLSAL
451 HLTWSYASFL TATARRAGIV PPSWANSSAS TIPSTCSGAS VVGSYSRPTA
501 TSFPPSQTPK PGVPSGTPYT PLPCATPTSV AVTFHELVST QFGQTVKVAG
551 NAAALGNWST SAAVALDAVN YADNHPLWIG TVNLEAGDVV EYKYINVGQD
601 GSVTWESDPN HTYTVPAVAC VTQVVKEDTW QS
```

53

# FIG. 1B

DNA coding sequence of TrGA (1899 bp) (SEQ ID NO: 4)

```
   1 ATGCACGTCC TGTCGACTGC GGTGCTGCTC GGCTCCGTTG CCGTTCAAAA
  51 GGTCCTGGGA AGACCAGGAT CAAGCGGTCT GTCCGACGTC ACCAAGAGGT
 101 CTGTTGACGA CTTCATCAGC ACCGAGACGC CTATTGCACT GAACAATCTT
 151 CTTTGCAATG TTGGTCCTGA TGGATGCCGT GCATTCGGCA CATCAGCTGG
 201 TGCGGTGATT GCATCTCCCA GCACAATTGA CCCGGACTAC TATTACATGT
 251 GGACGCGAGA TAGCGCTCTT GTCTTCAAGA ACCTCATCGA CCGCTTCACC
 301 GAAACGTACG ATGCGGGCCT GCAGCGCCGC ATCGAGCAGT ACATTACTGC
 351 CCAGGTCACT CTCCAGGGCC TCTCTAACCC CTCGGGCTCC CTCGCGGACG
 401 GCTCTGGTCT CGGCGAGCCC AAGTTTGAGT TGACCCTGAA GCCTTTCACC
 451 GGCAACTGGG GTCGACCGCA GCGGGATGGC CCAGCTCTGC GAGCCATTGC
 501 CTTGATTGGA TACTCAAAGT GGCTCATCAA CAACAACTAT CAGTCGACTG
 551 TGTCCAACGT CATCTGGCCT ATTGTGCGCA ACGACCTCAA CTATGTTGCC
 601 CAGTACTGGA ACCAAACCGG CTTTGACCTC TGGGAAGAAG TCAATGGGAG
 651 CTCATTCTTT ACTGTTGCCA ACCAGCACCG AGCACTTGTC GAGGGCGCCA
 701 CTCTTGCTGC CACTCTTGGC CAGTCGGGAA GCGCTTATTC ATCTGTTGCT
 751 CCCCAGGTTT TGTGCTTTCT CCAACGATTC TGGGTGTCGT CTGGTGGATA
 801 CGTCGACTCC AACATCAACA CCAACGAGGG CAGGACTGGC AAGGATGTCA
 851 ACTCCGTCCT GACTTCCATC CACACCTTCG ATCCCAACCT TGGCTGTGAC
 901 GCAGGCACCT TCCAGCCATG CAGTGACAAA GCGCTCTCCA ACCTCAAGGT
 951 TGTTGTCGAC TCCTTCCGCT CCATCTACGG CGTGAACAAG GGCATTCCTG
1001 CCGGTGCTGC CGTCGCCATT GGCCGGTATG CAGAGGATGT GTACTACAAC
1051 GGCAACCCTT GGTATCTTGC TACATTTGCT GCTGCCGAGC AGCTGTACGA
1101 TGCCATCTAC GTCTGGAAGA AGACGGGCTC CATCACGGTG ACCGCCACCT
1151 CCCTGGCCTT CTTCCAGGAG CTTGTTCCTG GCGTGACGGC CGGGACCTAC
1201 TCCAGCAGCT CTTCGACCTT TACCAACATC ATCAACGCCG TCTCGACATA
1251 CGCCGATGGC TTCCTCAGCG AGGCTGCCAA GTACGTCCCC GCCGACGGTT
1301 CGCTGGCCGA GCAGTTTGAC CGCAACAGCG GCACTCCGCT GTCTGCGCTT
1351 CACCTGACGT GGTCGTACGC CTCGTTCTTG ACAGCCACGG CCCGTCGGGC
1401 TGGCATCGTG CCCCCCTCGT GGGCCAACAG CAGCGCTAGC ACGATCCCCT
1451 CGACGTGCTC CGGCGCGTCC GTGGTCGGAT CCTACTCGCG TCCCACCGCC
1501 ACGTCATTCC CTCCGTCGCA GACGCCCAAG CCTGGCGTGC CTTCCGGTAC
1551 TCCCTACACG CCCCTGCCCT GCGCGACCCC AACCTCCGTG GCCGTCACCT
1601 TCCACGAGCT CGTGTCGACA CAGTTTGGCC AGACGGTCAA GGTGGCGGGC
1651 AACGCCGCGG CCCTGGGCAA CTGGAGCACG AGCGCCGCCG TGGCTCTGGA
1701 CGCCGTCAAC TATGCCGATA ACCACCCCCT GTGGATTGGG ACGGTCAACC
1751 TCGAGGCTGG AGACGTCGTG GAGTACAAGT ACATCAATGT GGGCCAAGAT
1801 GGCTCCGTGA CCTGGGAGAG TGATCCCAAC CACACTTACA CGGTTCCTGC
1851 GGTGGCTTGT GTGACGCAGG TTGTCAAGGA GGACACCTGG CAGTCGTAA
```

# FIG. 1C

signal peptide | pro | catalytic region | mature chain | linker | starch binding domain

**TrGA precursor**
632 aa

# FIG. 2

# FIG. 3

# FIG. 4

amdS Terminator TrTEL

amdS ORF KanR

pTTT-TrGA
12938 bp

amdS promoter

cbh1 terminator

attB2

TrTEL

Ori

AmpR

TrGA cDNA

attB1

cbh1 promoter

# FIG. 5A

```
AaGA    (1)   -ATLDSWLSNEATVARTAILNNIGADGAWVSGADSGIVVASPSTDNPDYF
AnGA    (1)   -ATLDSWLSNEATVARTAILNNIGADGAWVSGADSGIVVASPSTDNPDYF
AoGA    (1)   QSDLNAFIEAQTPIAKQGYLNNIGADGKLVEGAAAGIVYASPSKSNPDYF
HgGA    (1)   -AAVDTFINTEKPIAWNKLLANIGPNGKAAPGAAAGVVIASPSRTDPPYF
HvGA    (1)   --SVDDFINTQTPIALNNLLCNVGPDGCRAFGTSAGAVIASPSTTDPDYY
TrGA    (1)   --SVDDFISTETPIALNNLLCNVGPDGCRAFGTSAGAVIASPSTIDPDYY
                    *    *  *   *      *    * *  ****   *  *


AaGA    (50)  YTWTRDSGLVIKTLVDLFRNGDTD-LLSTIENYISSQAIVQGISNPSGDL
AnGA    (50)  YTWTRDSGLVLKTLVDLFRNGDTS-LLSTIENYISAQAIVQGISNPSGDL
AoGA    (51)  YTWTRDAGLTMEEYIEQFIGGDAT-LESTIQNYVDSQANEQAVSNPSGGL
HgGA    (50)  FTWTPDAALVLTGIIESLGHNYNT--------------TLQQVSNPSGTF
HvGA    (49)  YMWTRDSALVFKNIVDRFTQQYDAGLQRRIEQYISAQVTLQGISNPSGSL
TrGA    (49)  YMWTRDSALVFKNLIDRFTETYDAGLQRRIEQYITAQVTLQGLSNPSGSL
                  ** *  *                               *   *****


AaGA    (99)  SSGG-LGEPKFNVDETAYTGSWGRPQRDGPALRATAMIGFRQWLLDNGYT
AnGA    (99)  SSGAGLGEPKFNVDETAYTGSWGRPQRDGPALRATAMIGFGQWLLDNGYT
AoGA    (100) SDGSGLAEPKFYYNISQFTDSWGRPQRDGPALRASALIAYGNSLISSDKQ
HgGA    (86)  ADGSGLGEAKFNVDLTAFTGEWGRPQRDGPPLRAIALIQYAKWLIANGYK
HvGA    (99)  SDGSGLGEPKFELTLSQFTGNWGRPQRDGPALRAIALIGYSKWLINNNYQ
TrGA    (99)  ADGSGLGEPKFELTLKPFTGNWGRPQRDGPALRAIALIGYSKWLINNNYQ
                *   *  *  **       *  ********* *** * *        *


AaGA    (148) SAATEIVWPLVRNDLSYVAQYWNQTGYDLWEEVNGSSFFTIAVQHRALVE
AnGA    (149) STATDIVWPLVRNDLSYVAQYWNQTGYDLWEEVNGSSFFTIAVQHRALVE
AoGA    (150) SVVKANIWPIYQNDLSYVGQYWNQTGFDLWEEVQGSSFFTVAVQHKALVE
HgGA    (136) STAKSVVWPVVKNDLAYTAQYWNETGFDLWEEVPGSSFFTIASSHRALTE
HvGA    (149) STVSNIIWPIVRNDLNYVAQYWNQTGFDLWEEVNGSSFFTVANQHRALVE
TrGA    (149) STVSNVIWPIVRNDLNYVAQYWNQTGFDLWEEVNGSSFFTVANQHRALVE
                *         **   *** *   **** **  ****** ****** *   *  ** *


AaGA    (198) GSAFATAVGSSCSWCDSQAPQILCYLQSFWTG--EYILANFDSS--RSGK
AnGA    (199) GSAFATAVGSSCSWCDSQAPEILCYLQSFWTG--SFILANFDSS--RSGK
AoGA    (200) GDAFAKALGEECQACS-VAPQILCHLQDFWNG--SAVLSNLPTNG-RSGL
HgGA    (186) GAYLAAQLDTECPPCTTVAPQVLCFQQAFWNSKGNYVVSTSTAGEYRSGK
HvGA    (199) GATLAATLGQSGSTYSSVAPQILCFLQRFWVS-GGYIDSNINTNEGRTGK
TrGA    (199) GATLAATLGQSGSAYSSVAPQVLCFLQRFWVSSGGYVDSNINTNEGRTGK
                *   *          **  **  * **               * *


AaGA    (244) DTNTLLGSIHTFDPEAGCDDSTFQPCSPRALANHKEVVDSFRSIYTLNDG
AnGA    (245) DANTLLGSIHTFDPEAACDDSTFQPCSPRALANHKEVVDSFRSIYTLNDG
AoGA    (246) DTNSLLGSIHTFDPAAACDDTTFQPCSSRALSNHKLVVDSFRSVYGINNG
HgGA    (236) DANSILASIHNFDPEAGCDNLTFQPCSERALANHKAYVDSFRNLYAINKG
HvGA    (248) DANSLLASIHTFDPSLGCDASTFQPCSDKALSNLKVVVDSFRSIYGVNKG
TrGA    (249) DVNSVLTSIHTFDPNLGCDAGTFQPCSDKALSNLKVVVDSFRSIYGVNKG
                * *  *  *** ***   **  *****  ** *  *  ***** *   *  *


AaGA    (294) LSDSEAVAVGRYPKDSYYNGNPWFLCTLAAAEQLYDALYQWDKQGSLEIT
AnGA    (295) LSDSEAVAVGRYPEDTYYNGNPWFLCTLAAAEQLYDALYQWDKQGSLEVT
AoGA    (296) RGAGKAAAVGPYAEDTYQGGNPWYLTTLVAAELLYDALYQWDKQGQVNVT
HgGA    (286) IAQGKAVAVGRYSEDVYYNGNPWYLANFAAAEQLYDAIYVWNKQGSITVT
HvGA    (298) IPAGSAVAIGRYPEDVYFNGNPWYLATFAAAEQLYDSVYVWKKTGSITVT
TrGA    (299) IPAGAAVAIGRYAEDVYYNGNPWYLATFAAAEQLYDAIYVWKKTGSITVT
                *  *  *   *  *  *   ****  *       *** ***   *  *  *  *        *
```

# FIG. 5B

```
AaGA  (344)  DVSLDFFQALYSDAATGTYSSSSSTYSSIVDAVKTFADGFVSIVETHAAS
AnGA  (345)  DVSLDFFKALYSDAATGTYSSSSSTYSSIVDAVKTFADGFVSIVETHAAS
AoGA  (346)  ETSLPFFKDLSSNVTTGSYAKSSSAYESLTSAVKTYADGFISVVQEYTPD
HgGA  (336)  SVSLPFFRDLVSSVSTGTYSKSSSTFTNIVNAVKAYADGFIEVAAKYTPS
HvGA  (348)  STSSAFFQELVPGVAAGTYSSSQSTFTSIINAISTYADGFLSEAAKYVPA
TrGA  (349)  ATSLAFFQELVPGVTAGTYSSSSSTFTNIINAVSTYADGFLSEAAKYVPA
             *   **   *     *   *  *  *        *      * * * *


AaGA  (394)  NGSLSEQYDKSDGDELSARDLTWSYAALLTANNRRNSVMPPSWGETSAS-
AnGA  (395)  NGSMSEQYDKSDGEQLSARDLTWSYAALLTANNRRNSVVPASWGETSAS-
AoGA  (396)  GGALAEQYSRDQGTPVSASDLTWSYAAFLSAVGRRNGTVPASWGSSTAN-
HgGA  (386)  NGALAEQYDRNTGKPDSAADLTWSYSAFLSAIDRRAGLVPPSWRASVAKS
HvGA  (398)  DGSLAEQFDRNTGTPLSAVHLTWSYASFLTAAARRAGVVPPSWASSGAN-
TrGA  (399)  DGSLAEQFDRNSGTPLSALHLTWSYASFLTATARRAGIVPPSWANSSAS-
             *    **     *    **  * * * * *   * *  * *    * * *     *


AaGA  (443)  SVPGTC
AnGA  (444)  SVPGTC
AoGA  (445)  AVPSQC
HgGA  (436)  QLPSTC
HvGA  (447)  TVPSSC
TrGA  (448)  TIPSTC
               •   *
```

60

# FIG. 5C

gsldsflatetpialqgvlnnigpngadvagasagivvaspsrsdpdyfyswtrdaaltakylvdafia
gnkdleqtiqeyisaqaqvqtisnpsgdlstgglgepkfnvnetaftgpwgrpqrdgpalrataliaya
nylidngqastadeiiwpivqndlsyvtqywnsstfdlweevegssffttavqhralvegnalatrlnh
tcpncvsqapqvlcflqsywtgsyvlanfggsgrsgkdvnsilgsihtfdpaggcddstfqpcsarala
nhkvvtdsfrsvyavnsgiaegsavavgrypedvyqggnpwylataaaaeqlydaiyqwnkigsisitd
vslaffqdiypsaavgtynsgsstfndiisavqtyadgylsiiekytpsdgslteqfsrsdgtplsasg
ltwsyaslltaaarrqsivpaswgessassvpavcsatsatgpystatntawpssgsgpstttsvpctt
ptsvavtfdeivsttygetiylagsipelgnwspssaiplradaytssnplwyvtlnlpagtsfeykff
kketdgtivweddpnrsytvpaycgqttailddswq

FIG. 6

Starch Binding Domain

Linker

Catalytic Domain

FIG. 7

Starch Binding Domain
SBD

Catalytic Domain

EP 2 479 264 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US P5024941 A
- US P4794175 A
- WO 8809795 A
- US P4247637 A
- US P6255084 B
- US P6620924 B
- US P4863864 A
- WO 05052148 A
- US P4618579 A
- WO 2006060062 A
- US 20060094080 A
- US P6582914 A
- US 5874276 A
- US 5246853 A
- US 5475101 A
- WO 9206209 A
- US P5847276 A
- WO 05001036 A
- WO 0214490 A

- EP 238023 A
- US P6022725 A
- US P6268328 B
- EP 244234 A
- EP 215594 A
- WO 9600787 A
- US P6803499 B
- US P6777589 B
- US 7037704 B
- US 2006015342 A
- WO 2006043178 A
- US P6254914 B
- US P6899910 B
- US 4514496 A
- WO 04081193 A
- WO 04080923 A
- WO 03049550 A
- US 20060094080 A1, Dunn-Coleman

### Non-patent literature cited in the description

- **SVENSSON et al.** *Carlsberg Res. Commun.,* 1983, vol. 48, 529-544
- **BOEL et al.** *EMBO J.,* 1984, vol. 3, 1097-1102
- **HAYASHIDA et al.** *Agric. Biol. Chem.,* 1989, vol. 53, 923-929
- **ASHIKARI et al.** *Agric. Biol. Chem.,* 1986, vol. 50, 957-964
- **ASHIKARI et al.** *App. Microbiol. and Biotech.,* 1989, vol. 32, 129-133
- **HOUGHTON-LARSEN et al.** *Appl. Microbiol. Biotechnol.,* 2003, vol. 62, 210-217
- **SINGLETON et al.** DICTIONARY OF MICROBIOLOGY AND MOLECULAR BIOLOGY. John Wiley and Sons, 1994
- **HALE ; MARKHAM.** THE HARPER COLLINS DICTIONARY OF BIOLOGY. Harper Perennial, 1991
- **SMITH ; WATERMAN.** *Adv. Appl. Math.,* 1981, vol. 2, 482
- **NEEDLEMAN ; WUNSCH.** *J. Mol. Biol.,* 1988, vol. 48, 443
- **PEARSON ; LIPMAN.** *Proc. Natl. Acad. Sci. USA,* 1988, vol. 85, 2444
- **DEVEREUX et al.** *Nucl. Acid Res.,* 1984, vol. 12, 387-395

- **ALTSCHUL et al.** *J. Mol. Biol.,* 1990, vol. 215, 403-410
- **KARLIN et al.** *Proc. Natl. Acad. Sci. USA,* 1993, vol. 90, 5873-5787
- **ALTSCHUL et al.** *Meth. Enzymol.,* 1996, vol. 266, 460-480
- **FENG ; DOOLITTLE.** *J. Mol. Evol.,* 1987, vol. 35, 351-360
- **HIGGINS ; SHARP.** *CABIOS,* 1989, vol. 5, 151-153
- **COUTINHO et al.** *Protein Eng.,* 1994, vol. 7, 393-400
- **COUTINHO et al.** *Protein Eng.,* 1994, vol. 7, 749-760
- **ALESHIN et al.** *J Mol Biol,* 1994, vol. 238, 575-591
- **MINSHULL J. et al.** Engineered protein function by selective amino acid diversification. *Methods,* 2004, vol. 32 (4), 416-427
- MORE GENE MANIPULATIONS IN FUNGI. Academic Press, 1991, 396-428
- **NUNBERG et al.** *Mol. Cell Biol.,* 1984, vol. 4, 2306-2315
- **BOEL et al.** *EMBO J.,* 1984, vol. 3, 1581-1585
- **PUNT et al.** *Gene,* 1987, vol. 56, 117-124

- **FINKELSTEIN.** BIOTECHNOLOGY OF FILAMENTOUS FUNGI. 1992
- **KINGHORN et al.** APPLIED MOLECULAR GENETICS OF FILAMENTOUS FUNGI. Blackie Academic and Professional, Chapman and Hall, 1992
- **BERGES ; BARREAU.** *Curr. Genet.,* 1991, vol. 19, 359-365
- **VAN HARTINGSVELDT et al.** *Mol. Gen. Genet.,* 1987, vol. 206, 71-75
- **KELLEY et al.** *EMBO J.,* 1985, vol. 4, 475-479
- **PENTTILÄ et al.** *Gene,* 1987, vol. 61, 155-164
- **BENNETT ; LASURE.** MORE GENE MANIPULATIONS IN FUNGI. Academic Press, 1991, 70-76
- **ALEXOPOULOS, C. J.** INTRODUCTORY MYCOLOGY. Wiley, 1962
- **SHEIR-NEIRS et al.** *Appl. Microbiol. Biotechnol,* 1984, vol. 20, 46-53
- **WARD et al.** *Appl. Microbiol. Biotechnol.,* 1993, vol. 39, 738-743
- **GOEDEGEBUUR et al.** *Genet,* 2002, vol. 41, 89-98
- **INNIS et al.** *Sci.,* 1985, vol. 228, 21-26
- **CAMPBELL et al.** *Curr. Genet.,* 1989, vol. 16, 53-56
- **ANAGNOSTOPOULOS C ; J. SPIZIZEN.** *J. Bacteriol.,* 1961, vol. 81, 741-746
- **YELTON et al.** *Proc. Natl. Acad. Sci. USA,* 1984, vol. 81, 1470-1474
- **BERKA et al.** Applications of Enzyme Biotechnology. Plenum Press, 1991
- **CAO et al.** *Sci,* 2000, vol. 9, 991-1001
- **HARKKI et al.** *Enzyme Microb. Technol.,* 1991, vol. 13, 227-233
- **HARKKI et al.** *Bio Technol.,* 1989, vol. 7, 596-603
- The Molecular Biology of Trichoderma and its Application to the Expression of Both Homologous and Heterologous Genes. **NEVALAINEN et al.** MOLECULAR INDUSTRIAL MYCOLOGY. Marcel Dekker Inc, 1992, 129-148
- **BAJAR et al.** *Proc. Natl. Acad. Sci. USA,* 1991, vol. 88, 8202-28212
- **PENTILLA et al.** *Gene,* 1987, vol. 61, 155-164
- **DE GROOT et al.** *Nat. Biotechnol.,* 1998, vol. 16, 839-842
- **FROMM et al.** *Biotechnol.,* 1990, vol. 8, 833-839
- **POTRYKUS et al.** *Mol. Gen. Genet.,* 1985, vol. 199, 169-177
- **POURQUIE, J. et al.** BIOCHEMISTRY AND GENETICS OF CELLULOSE DEGRADATION. Academic Press, 1988, 71-86
- **ILMEN, M. et al.** *Appl. Environ. Microbiol.,* 1997, vol. 63, 1298-1306
- *Biosci. Biotechnol. Biochem.,* 1994, vol. 58, 49-54
- STARCH CONVERSION TECHNOLOGY. Marcel Dekker Inc, 1985
- THE ALCOHOL TEXTBOOK, A REFERENCE FOR THE BEVERAGE, FUEL AND INDUSTRIAL ALCOHOL INDUSTRIES. Nottingham University Press, 1999
- **BRUNGER, A.** *Nature,* 1992, vol. 355, 472-475
- **ALESHIN et al.** *J. Mol. Biol.,* 1994, vol. 238, 575-591
- **MURSHUDOV et al.** *Acta Crystallogr.,* 1997, vol. D53, 240-255
- **EMSLEY et al.** *Acta Crystallogr D Biol Crystallogr,* 2004, vol. 60, 2126-32
- **JONES et al.** *Acta Crystallogr.,* 1991, vol. A47, 110-119
- The PyMOL Molecular Graphics System. Palo Alto, CA , USA. **DELANO W.L.** The PyMOL Molecular Graphics System. Palo Alto, CA, USA. DeLano Scientific, 2002
- **ALESHIN, A.E. ; HOFFMAN, C. ; FIRSOV, L.M. ; HONZATKO, R.B.** *J Mol Biol,* 1994, vol. 238, 575-591